# EUROPÄISCHE PATENTSCHRIFT

(11) **EP 2 632 922 B1**
(45) Veröffentlichungstag und Bekanntmachung des Hinweises auf die Patenterteilung: **27.02.2019**
(21) Anmeldenummer: 11778827.3
(22) Anmeldetag: 25.10.2011
(51) Int. Cl.: C07D 417/14, A01N 43/78

(54) **HETEROARYLPIPERIDIN UND -PIPERAZINDERIVATE ALS FUNGIZIDE**
HETEROARYL PIPERIDINE AND HETEROARYL PIPERAZINE DERIVATIVES AS FUNGICIDES
HÉTÉROARYLPIPÉRIDINE ET DÉRIVÉS D'HÉTÉROARYLPIPÉRIDINE COMME FONGICIDE

(30) Priorität: 27.10.2010 US 407200 P; 27.10.2010 EP 10189067
(43) Veröffentlichungstag der Anmeldung: 04.09.2013
(73) Patentinhaber: Bayer CropScience Aktiengesellschaft, 40789 Monheim (DE)
(72) Erfinder: HILLEBRAND, Stefan, 41462 Neuss (DE); CRISTAU, Pierre, F-69009 Lyon (FR); HOFFMANN, Sebastian, 41470 Neuss (DE); KLUTH, Joachim, 40764 Langenfeld (DE); SEITZ, Thomas, 40764 Langenfeld (DE); WASNAIRE, Pierre, 40225 Düsseldorf (DE); TSUCHIYA, Tomoki, 69009 Lyon (FR); BENTING, Jürgen, 42799 Leichlingen (DE); WACHENDORFF-NEUMANN, Ulrike, 56566 Neuwied (DE)
(74) Vertreter: BIP Patents
(86) Internationale Anmeldenummer: PCT/EP2011/068592
(87) Internationale Veröffentlichungsnummer: WO 2012/055837

(56) Entgegenhaltungen:
- WO-A1-2011/076699
- WO-A2-2008/013622
- WO-A2-2008/013925
- WO-A2-2009/055514
- WO-A2-2009/094407
- WO-A2-2009/094445
- WO-A2-2010/065579

## Beschreibung

Die Erfindung betrifft Heteroarylpiperidin und -piperazinderivate, deren agrochemisch wirksame Salze, deren Verwendung sowie Verfahren und Mittel zur Bekämpfung von pflanzenpathogenen Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von pflanzenpathogenen Schadpilzen in der Land-, Garten- und Forstwirtschaft, in der Tiergesundheit, im Materialschutz sowie im Bereich Haushalt und Hygiene. Die vorliegende Erfindung betrifft weiterhin ein Verfahren zur Herstellung von Heteroarylpiperidin und -piperazinderivaten.

Es ist bereits bekannt, dass bestimmte heterocyclisch substituierte Thiazole als fungizide Pflanzenschutzmittel benutzt werden können (siehe Patentanmeldungen mit der Veröffentlichungsnummer: WO 07/014290, WO 08/013925, WO 08/013622, WO 08/091594, WO 08/091580, WO 09/055514, WO 09/094407, WO 09/094445, WO 09/132785, WO 10/037479, WO10/065579, WO2010/066353, WO2010/149275, WO2011/051243, WO2011/051244, WO 2011/076510, WO 2011/018415, WO 2011/018401, WO 2011/076699, siehe auch Patentanmeldungen mit der Anmeldenummer: DE102010000662.9, PCT/EP2011/056594, PCT/EP2011/057912, PCT/EP2011/058330, PCT/EP2011/063783, PCT/EP2011/064527). Die fungizide Wirksamkeit dieser Verbindungen ist jedoch gerade bei niedrigeren Aufwandmengen nicht immer ausreichend.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden Heteroarylpiperidin und -piperazinderivate die genannten Aufgaben zumindest in Teilaspekten lösen und sich als Pflanzenschutzmittel, insbesondere als Fungizide eignen.

Gegenstand der Erfindung sind Verbindungen der Formel **(I)**, 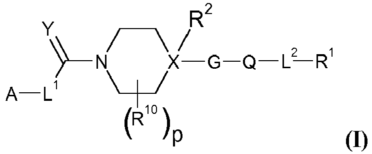 in welcher die Restedefinitionen folgende Bedeutungen haben:
- A: steht für Phenyl, das bis zu zwei Substituenten enthalten kann, wobei die Substituenten unabhängig voneinander ausgewählt sind aus folgender Liste: Fluor, Brom, Iod, Chlor, Cyano, Nitro, Methyl, Ethyl, *n*-Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, *n*-Propoxy, Methoxy, Trifluormethoxy, Difluormethoxy, 1-Methylethylthio, Methylthio, Ethylthio, *n*-Propylthio, Difluormethylthio oder Trifluormethylthio, oder
- A: steht für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten gleich oder verschieden und unabhängig voneinander ausgewählt sind aus folgender Liste:
Substituenten am Kohlenstoff:
Fluor, Chlor, Brom, Iod, Cyano, Nitro, Methyl, Ethyl, *n*-Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, *n*-Propoxy, Methoxy, Trifluormethoxy, Difluormethoxy, 1-Methylethylthio, Methylthio, Ethylthio, *n*-Propylthio, Difluormethylthio, Trifluormethylthio oder Phenyl,
Substituenten am Stickstoff:
Methyl, Ethyl, *n*-Propyl, 1-Methylethyl, Methylsulfonyl, Trifluormethylsulfonyl, Methylcarbonyl, Trifluormethylcarbonyl, Chlormethylcarbonyl, 2,2-Trifluorethyl, 2,2-Difluorethyl, 2,2-Dichlor-2-fluorethyl, 2-Chlor-2-difluorethyl oder 2-Chlor-2-fluorethyl
- L¹: steht für CHR^{L11} oder NR^{L12},
- R^{L11}: steht für Wasserstoff oder Methyl,
R^{L12} steht für Wasserstoff oder Methyl,
- Y: steht für Sauerstoff,
- X: steht für Kohlenstoff,
- R²: steht für Wasserstoff, Fluor, Chlor, Brom oder Hydroxy,
- R¹⁰: steht für Oxo, Alkyl, Alkenyl, Halogenalkyl, Alkoxy, Halogen, Cyano oder Hydroxy,
- p: steht für 0,
- G: steht für 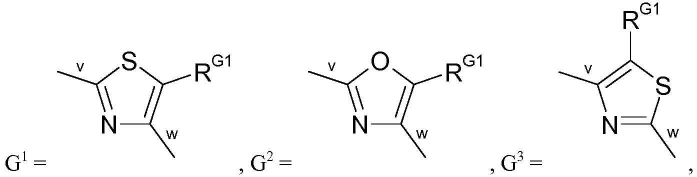 wobei die Bindung, die mit "v" identifiziert ist, direkt an X gebunden ist, und wobei die Bindung, die mit "w" identifiziert ist, direkt an Q gebunden ist,
Q steht für 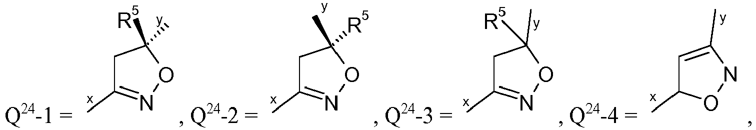 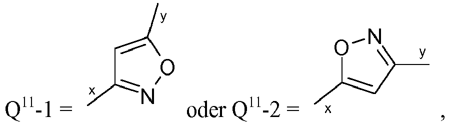 wobei die Bindung, die mit "x" identifiziert ist, direkt an G gebunden ist und die Bindung, die mit "y" identifiziert ist, direkt an L² gebunden ist,
R⁵ steht für Wasserstoff, Cyano, Methyl, Trifluomethyl, Difluormethyl oder Methoxymethyl,
R^{G1} steht für Wasserstoff,
L² steht für eine direkte Bindung, -C(=O)-, -CHR²⁰- oder -NR²¹-,
R²⁰ steht für Wasserstoff, Methyl, Ethyl, Trifluormethyl,
R²¹ steht für Wasserstoff oder Methyl,
R¹ steht für Phenyl, das 1, 2 oder 3 Substituenten enthalten kann, wobei ein Substituent ausgewählt ist aus Z⁴ und gegebenenfalls weitere Substituenten ausgewählt sind aus folgender Liste: Fluor, Chlor, Brom, Iod, Cyano, Nitro, Hydroxy, Amino, -SH, -C(=O)H, Methyl, Ethyl, n-Propyl, 1-Methylethyl, n-Butyl, 1,1-Dimethylethyl, 1,2-Dimethylethyl, Ethenyl, Ethinyl, Trifluormethyl, Difluormethyl, Trichlormethyl, Dichlormethyl, Cyclopropyl, Methoxy, Ethoxy, *n*-Propoxy, 1-Methylethoxy, 1,1-Dimethylethoxy, Methylcarbonyl, Ethylcarbonyl, Trifluormethylcarbonyl, Methoxycarbonyl, Ethoxycarbonyl, *n-*Propoxycarbonyl, 1-Methylethoxycarbonyl, 1,1-Dimethylethoxycarbonyl, 1-Ethenyloxy, 2-Propenyloxy, 2-Propinyloxy, Methylcarbonyloxy, Trifluoralkylcarbonyloxy, Chlormethylcarbonyloxy, Methylthio, Ethylthio, Methylsulfonyl, Methylsulfonylamino, Trifluormethylsulfonylamino oder -L³R³,
L³ steht für eine direkte Bindung,
R³ steht für Wasserstoff, Methyl, Ethyl, *n*-Propyl, 1-Methylethyl, *n*-Butyl oder 1,1-Dimethylethyl,
Z⁴ steht für C(=O)H, Alkylsulfonylamino oder Halogenalkylsulfonylamino,
sowie Salze, Metallkomplexe und N-Oxide der Verbindungen der Formel **(I)**, mit der Maßgabe, dass folgende Verbindungen ausgenommen sind: 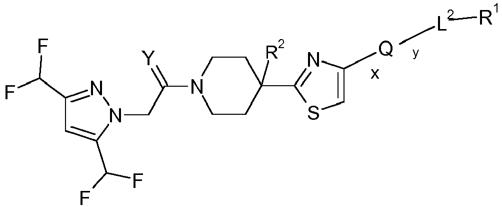 in welcher die Symbole folgende Bedeutungen haben:
- Y: steht für Sauerstoff oder Schwefel,
- R²: steht für Wassertoff oder Halogen

- Q: steht für

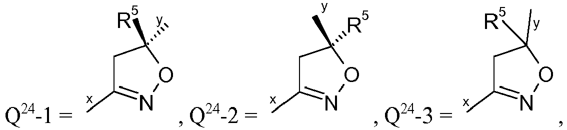

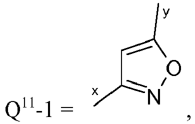

- R⁵: steht für Wasserstoff, Cyano, C₁-C₃-Alkyl oder C₁-C₃-Halogenalkyl,
- L²: steht für eine direkte Bindung und
- R¹: hat die oben genannte Bedeutung, wobei wenn R¹ für jeweils substituiertes Phenyl, Naphthyl, Dihydronaphthalenyl-, Tetrahydronaphthalenyl-, Hexahydronaphthalenyl-, Octahydronaphthalenyl-, Indenyl- oder für eine gegebenenfalls benzokondensiertes, 5- oder 6-gliedriges Heteroaryl steht, höchstens zwei Substituetenten an den genannten Carbocyclen bzw. Heterocyclen vorliegen.

Ein weiterer Gegenstand ist die Verwendung der Verbindungen der Formel **(I)** als Fungizide.

Erfindungsgemäße Heteroarylpiperidin und -piperazinderivate der Formel **(I)** sowie deren Salze, Metallkomplexe und N-Oxide eignen sich sehr gut als zur Bekämpfung pflanzenpathogener Schadpilze. Die vorgenannten erfindungsgemäßen Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel **(I)** können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die Restedefinitionen der erfindungsgemäßen Verbindungen der Formel **(I)** haben bevorzugt, besonders bevorzugt und ganz besonders bevorzugt folgende Bedeutungen:
- A: steht ganz besonders bevorzugt für Phenyl, der bis zu zwei Substituenten enthalten kann, wobei die Substituenten unabhängig voneinander ausgewählt sind aus folgender Liste: Methyl, Ethyl, Iod, Chlor, Brom, Fluor, Methoxy, Ethoxy, Difluormethyl oder Trifluormethyl oder
- A: steht ganz besonders bevorzugt für Pyrazol-1-yl, der bis zu zwei Substituenten enthalten kann, wobei die Substituenten unabhängig voneinander ausgewählt sind aus folgender Liste: Methyl, Ethyl, Chlor, Brom, Fluor oder Trifluormethyl,
- L¹: steht ganz besonders bevorzugt für CH₂,
- R^{L12}: steht ganz besonders bevorzugt für Wasserstoff,
- R²: steht ganz besonders bevorzugt für Wasserstoff oder Fluor (und insbesondere nur für Wasserstoff),
- R¹⁰: steht ganz besonders bevorzugt für Fluor,
- G: steht ganz besonders bevorzugt für G¹, 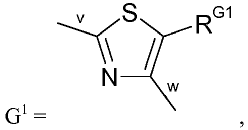
wobei die Bindung, die mit "v" identifiziert ist, direkt an X gebunden ist, und wobei die Bindung, die mit "w" identifiziert ist, direkt an Q gebunden ist,
- Q: steht ganz besonders bevorzugt für Q²⁴-3 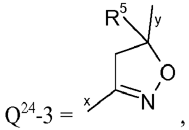
wobei die Bindung, die mit "x" identifiziert ist, direkt an G gebunden ist und die Bindung, die mit "y" identifiziert ist, direkt an L² gebunden ist,
- R⁵: steht ganz besonders bevorzugt für Wasserstoff,
- L²: steht am bevorzugtesten steht L² für eine direkte Bindung,

Die erfindungsgemäß verwendbaren Heteroarylpiperidin und -piperazinderivate sind durch die Formel **(I)** allgemein definiert. Die Restedefinitionen der vorstehenden und nachfolgend genannten Restedefinitionen der Formel **(I)** gelten für die Endprodukte der Formel **(I)** wie für alle Zwischenprodukte (siehe auch unten unter "Erläuterungen der Verfahren und Zwischenprodukte") gleichermaßen.

Die oben aufgeführten bzw. nachfolgenden allgemeinen oder in Vorzugsbereichen aufgeführten Restedefinitionen bzw. Erläuterungen können auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

Bevorzugt sind solche Verbindungen der Formel **(I)**, in welcher alle Reste jeweils die oben genannten bevorzugten Bedeutungen haben.

Besonders bevorzugt sind solche Verbindungen der Formel **(I)**, in welcher alle Reste jeweils die oben genannten besonders bevorzugten Bedeutungen haben.

Ganz besonders bevorzugt sind solche Verbindungen der Formel **(I)**, in welcher alle Reste jeweils die oben genannten ganz besonders bevorzugten Bedeutungen haben.

Weiterhin bevorzugt sind Verbindungen der Formel **(I)** sowie agrochemisch wirksame Salze, Metallkomplexe und N-Oxide davon in welcher:
- A: für 5-Methyl-3-(trifluormethyl)-1H-pyrazol-1-yl, 5-Chlor-2-methylphenyl oder für 2,5- Bis(difluoromethyl)phenyl steht,
- L¹: für -CH₂- oder -NH- steht,
- L²: für eine direkte Bindung steht,
- Y: für Sauerstoff steht,
- X: für Kohlenstoff steht,
- p: für 0 steht,
- G: für G¹ steht,
- R^{G1}: für Wasserstoff steht,
- Q: für Q²⁴-3 steht,
- R²: für Wasserstoff steht,
- R⁵: für Wasserstoff steht,
- R¹: für 3-Formylphenyl, 2-Formylphenyl oder 2-[(Methylsulfonyl)amino]phenyl steht.

Die zuvor genannten Reste-Definitionen können untereinander in beliebiger Weise kombiniert werden. Außerdem können einzelne Definitionen entfallen.

Je nach Art der oben definierten Substituenten weisen die Verbindungen der Formel **(I)** saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel **(I)** Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel **(I)** Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkyl-Gruppen, Mono-, Di- und Trialkanolamine von C₁-C₄-Alkanolen, Cholin sowie Chlorcholin.

Die so erhältlichen Salze weisen ebenfalls fungizide Eigenschaften auf.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, gesättigte oder einfach oder doppelt ungesättigte C₆-C₂₀-Fettsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Iod und vorzugsweise Fluor, Chlor, Brom und noch bevorzugter Fluor, Chlor.
**Alkyl:** gesättigte, geradkettige oder verzweigte Kohlenwasserstoffreste mit 1 bis 8 vorzugsweise 1 bis 6 und noch bevorzugter 1 bis 3 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Diese Definition gilt auch für Alkyl as Bestandteil eines zusammengesetzen Substitutenten wie z.B. Cycloalkylalkyl, Hydroxyalkyl etc. sofern an anderer Stelle nicht definiert wie z.B. Alkylthio, Alkylsufinyl, Alkylsulfonyl, Halogenalkyl bzw. Halogenalkylthio. Steht das Alkyl am Ende eines zusammengesetzten Substituenten. wie z.B bei Alkylcylcloalkyl, so kann der am Anfang stehende Bestandteil des zusammengesetzten Substituenten, z.B. das Cycloalkyl, ein- bzw. mehrfach, gleich oder verschieden und unabhängig voneinander mit Alkyl substituiert sein. Das gleiche gilt auch für zusammengesetzte Substituenten bei denen andere Reste wie z.B. Alkenyl, Alkinyl, Hydroxy, Halogen, Formyl etc. am Ende stehen.
**Alkenyl:** ungesättigte, geradkettige oder verzweigte Kohlenwasserstoffreste mit 2 bis 8 vorzugsweise 2 bis 6 Kohlenstoffatomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl. Diese Definition gilt auch für Alkenyl als Bestandteil eines zusammengesetzen Substitutenten wie z.B. Halogenalkenyl etc. sofern an anderer Stelle nicht definiert;
**Alkinyl:** geradkettige oder verzweigte Kohlenwasserstoffgruppen mit 2 bis 8 vorzugsweise 2 bis 6 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl. Diese Definition gilt auch für Alkinyl als Bestandteil eines zusammengesetzen Substitutenten wie z.B. Halogenalkinyl etc. sofern an anderer Stelle nicht definiert;
**Alkoxy:** gesättigte, geradkettige oder verzweigte Alkoxyreste mit 1 bis 8 vorzugsweise 1 bis 6 und noch bevorzugter 1 bis 3 Kohlenstoffatomen, z.B. (aber nicht beschränkt auf) C1-C6-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Dimethylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Diese Definition gilt auch für Alkoxy als Bestandteil eines zusammengesetzen Substitutenten wie z.B. Halogenalkoxy, Alkinylalkoxy etc. sofern an anderer Stelle nicht definiert;
**Alkylthio:** gesättigte, geradkettige oder verzweigte Alkylthioreste mit 1 bis 8 vorzugsweise 1 bis 6 und noch bevorzugter 1 bis 3 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio. Diese Definition gilt auch für Alkylthio als Bestandteil eines zusammengesetzen Substitutenten wie z.B. Halogenalkylthio etc. sofern an anderer Stelle nicht definiert;
**Alkoxycarbonyl:** eine Alkoxygruppe mit 1 bis 6 bevorzugt 1 bis 3 Kohlenstoffatomen (wie vorstehend genannt), welche über eine Carbonylgruppe (-CO-) an das Gerüst gebunden ist. Diese Definition gilt auch für Alkoxycarbonyl als Bestandteil eines zusammengesetzen Substitutenten wie z.B. Cycloalkylalkoxycarbonyl etc. sofern an anderer Stelle nicht definiert;
**Alkylsulfinyl:** gesättigte, geradkettige oder verzweigte Alkylsulfinylreste mit 1 bis 8 vorzugsweise 1 bis 6 und noch bevorzugter 1 bis 3 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, Hexylsulfinyl, 1,1-Dimethylpropylsulfinyl, 1,2-Dimethylpropylsulfinyl,1-Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl. Diese Definition gilt auch für Alkylsulfinyl als Bestandteil eines zusammengesetzen Substitutenten wie z.B. Halogenalkylsulfinyl etc. sofern an anderer Stelle nicht definiert;
**Alkylsulfonyl:** gesättigte, geradkettige oder verzweigte Alkylsulfonylreste mit 1 bis 8 vorzugsweise 1 bis 6 und noch bevorzugter 1 bis 3 Kohlenstoffatomen, z.B. (aber nicht beschränkt auf) C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentyl-sulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl. Diese Definition gilt auch für Alkylsulfonyl als Bestandteil eines zusammengesetzen Substitutenten wie z.B. Alkylsulfonylalkyl etc. sofern an anderer Stelle nicht definiert;
**Cycloalkyl:** monocyclische, gesättigte Kohlenwasserstoffgruppen mit 3 bis 10 vorzugsweise 3 bis 8 und noch bevorzugter 3 bis 6 Kohlenstoffringgliedern, z.B. (aber nicht beschränkt auf) Cyclopropyl, Cyclopentyl und Cyclohexyl. Diese Definition gilt auch für Cycloalkyl als Bestandteil eines zusammengesetzen Substitutenten wie z.B. Cycloalkylalkyl etc. sofern an anderer Stelle nicht definiert;
**Cycloalkenyl:** monocyclische, partiell ungesättigter Kohlenwasserstoffgruppen mit 3 bis 10 vorzugsweise 3 bis 8 und noch bevorzugter 3 bis 6 Kohlenstoffringgliedern, z.B. (aber nicht beschränkt auf) Cyclopropenyl, Cyclopenentyl und Cyclohexenyl. Diese Definition gilt auch für Cycloalkenyl als Bestandteil eines zusammengesetzen Substitutenten wie z.B. Cycloalkyenlalkyl etc. sofern an anderer Stelle nicht definiert;
**Cycloalkoxy:** monocyclische, gesättigte Cycloalkyloxyreste mit 3 bis 10 vorzugsweise 3 bis 8 und noch bevorzugter 3 bis 6 Kohlenstoffringgliedern, z.B. (aber nicht beschränkt auf) Cyclopropyloxy, Cyclopentyloxy und Cyclohexyloxy. Diese Definition gilt auch für Cycloalkoxy als Bestandteil eines zusammengesetzen Substitutenten wie z.B. Cycloalkoxyalkyl etc. sofern an anderer Stelle nicht definiert;
**Halogenalkyl:** geradkettige oder verzweigte Alkylgruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl. Diese Definition gilt auch für Halogenalkyl als Bestandteil eines zusammengesetzen Substitutenten wie z.B. Halogenalkylaminoalkyl etc. sofern an anderer Stelle nicht definiert;
   Halogenalkenyl und Halogenalkinyl sind analog wie Halogenalkyl definiert, wobei anstatt Alkylgruppen, Alkenyl und Alkinylgruppen als Bestandteil des Substituenten vorliegen.
**Halogenalkoxy:** geradkettige oder verzweigte Alkoxygruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy. Diese Definition gilt auch für Halogenalkoxy als Bestandteil eines zusammengesetzen Substitutenten wie z.B. Halogenalkoxyalkyl etc. sofern an anderer Stelle nicht definiert;
**Halogenalkylthio:** geradkettige oder verzweigte Alkylthiogruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio. Diese Definition gilt auch für Halogenalkylthio als Bestandteil eines zusammengesetzen Substitutenten wie z.B. Halogenalkylthioalkyl etc. sofern an anderer Stelle nicht definiert;
**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
**5-gliedriges Heteroaryl: enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Triazol-1-yl, 1-Imidazolyl, 1,2,3-Triazol-1-yl und 1,3,4-Triazol-1-yl;
**6-gliedriges Heteroaryl, enthaltend ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
**Benzokondensiertes 5-gliedriges Heteroaryl, enthaltend ein bis drei Stickstoffatome oder ein Stickstoffatom und ein Sauerstoff- oder Schwefelatom:** z.B. (aber nicht beschränkt auf) Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl und 1,3-Benzoxazol-7-yl,
**Benzokondensiertes 6-gliedriges Heteroaryl, enthaltend ein bis drei Stickstoffatome:** : z.B. (aber nicht beschränkt auf) Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, und Isochinolin-8-yl;
Diese Definition gilt auch für Heteroaryl als Bestandteil eines zusammengesetzen Substitutenten wie z.B. Heteroarylalkyl etc. sofern an anderer Stelle nicht definiert;
**Heterocyclyl:** drei- bis fünfzehngliedriger vorzugsweise ein drei- bis neungliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3 -yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl-Hexahydropyrimidinyl, 2-Piperazinyl, 1,3,5-Hexahydro-triazin-2-yl und 1,2,4-Hexahydrotriazin-3-yl. Diese Definition gilt auch für Heterocyclyl als Bestandteil eines zusammengesetzen Substitutenten wie z.B. Heterocyclylalkyl etc. sofern an anderer Stelle nicht definiert;
**Abgangsgruppe:** S_{N}1 oder S_{N}2-Abgangsgruppe, beispielsweise Chlor, Brom, Iod, Alkylsulfonate (-OSO₂-Alkyl, z.B. -OSO₂CH₃, -OSO₂CF₃) oder Arylsulfonate (-OSO₂-Aryl, z.B. -OSO₂Ph,-OSO₂PhMe);
Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Erläuterung der Herstellverfahren und Zwischenprodukte

Die Heteroarylpiperidin und -piperazinderivate der Formel **(I)** lassen sich auf unterschiedliche Weise herstellen. Im Folgenden sind die möglichen Verfahren zunächst schematisch dargestellt. Wenn nicht anders angegeben haben die angegebenen Reste die oben angegebenen Bedeutungen.

Die erfindungsgemäßen Verfahren zur Herstellung von Verbindungen der Formel **(I)** werden gegebenenfalls unter Verwendung eines oder mehrerer Reaktionshilfsmittel durchgeführt.

Als Reaktionshilfsmittel kommen gegebenenfalls anorganische oder organische Basen oder Säureakzeptoren in Betracht. Hierzu gehören vorzugsweise Alkalimetall- oder Erdalkalimetall- -acetate, - amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium-methanolat, -ethanolat, -n- oder -i-propanolat, -n-, -i-, -s- oder -tButanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl-und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die erfindungsgemäßen Verfahren werden gegebenenfalls unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methylisopropyl-oder Methyl-isobutyl-keton, Ester wie Essigsäuremethylester oder -ethylester, Nitrile wie z.B. Acetonitril oder Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid und DMPU.

Die Reaktionstemperaturen können bei den erfindungsgemäßen Verfahren in einem größeren Bereich variiert werden. Im Allgemeinen arbeitet man bei Temperaturen zwischen 0 °C und 250 °C, vorzugsweise bei Temperaturen zwischen 10 °C und 185 °C.

Die Reaktionszeit variiert in Abhängigkeit vom Maßstab der Reaktion und der Reaktionstemperatur, liegt aber im Allgemeinen zwischen einigen Minuten und 48 Stunden.

Die erfindungsgemäßen Verfahren werden im Allgemeinen unter Normaldruck durchgeführt. Es ist jedoch auch möglich, unter erhöhtem oder vermindertem Druck zu arbeiten.

Zur Durchführung der erfindungsgemäßen Verfahren werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuss zu verwenden.

### Verfahren A

| |
|---|
| 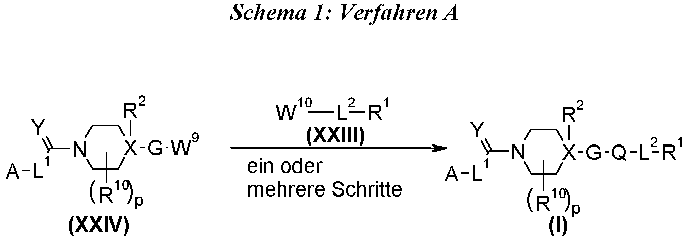 |
| W⁹ und W¹⁰ sind funktionelle Gruppen geeignet für das Bilden des gewünschten Heterocyclus und die Symbole A, G, Y, X, p, L¹, L², R¹, R² und R¹⁰ haben die in der Beschreibung angegebenen Bedeutungen. |

Im Allgemeinen ist es möglich Verbindungen der Formel **(I)** aus entsprechenden Verbindungen **(XXIII)** und **(XXIV)** mit geeigneten funktionellen Gruppen W⁹ und W¹⁰ **(I)** herzustellen (s. Schema 1, Verfahren A). Die möglichen funktionellen Gruppen für W⁹ und W¹⁰ sind z.B. Aldehyde, Ketone, Ester, Carbonsäuren, Amide, Thioamide, Nitrile, Alkohole, Thiole, Hydrazine, Oxime, Amidine, Amideoxidme, Olefine, Acetylene, Halide, Alkylhalide, Methansulfonate, Trifluormethansulfonate, Boronsäuren, Boronate u.s.w., die unter geeigneten Reaktionsbedingungen den gewünschten Heterocyclus **Q** bilden können. In der Literatur finden sich zahlreiche Methoden für die Darstellung von Heterocyclen (s. WO 2008/013622; Comprehensive Heterocyclic Chemistry Vol. 4-6, A. R. Katritzky and C. W. Rees editors, Pergamon Press, New York, 1984; Comprehensive Heterocyclic Chemistry II, Vol 2-4, A. R. Katritzky, C. W. Rees and E. F: Scriven editors, Pergamon Press, New York, 1996; The Chemistry of Heterocyclic Compounds, E. C. Taylor, editor, Wiley, New York; Rodd's Chemistry of Carbon Compounds, Vol. 2-4, Elsevier, New York; Synthesis, 1982, 6, 508-509; Tetrahedron, 2000, 56, 1057-1094; und darin zitierte Literatur).

### Verfahren B

| |
|---|
| 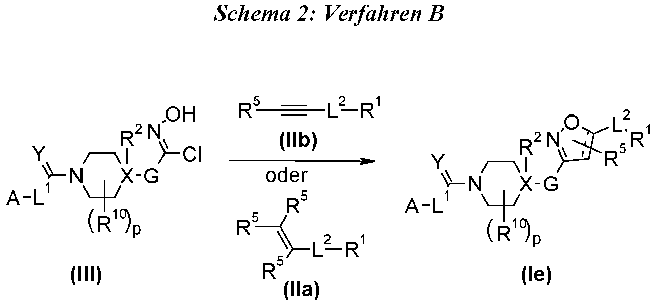 |
| Die Symbole A, G, X, Y, p, L¹, L², R¹, R², R⁵ und R¹⁰ haben die in der Beschreibung angegebenen Bedeutungen. |

Eine bestimmte Möglichkeit, Verbindungen der Formel **(Ie)** aus entsprechenden Verbindungen **(III)** durch Reaktion mit den Verbindungen **(IIa)** oder **(IIb)** darzustellen, ist in Schema 2 gezeigt.

Die Alkene und Alkine **(IIa)** und **(IIb)** sind kommerziell verfügbar oder können aus kommerziell erhältlichen Vorstufen nach literaturbeschriebenen Vorschriften hergestellt werden (z.B. aus Ketonen oder Aldehyden durch eine Wittig- oder Horner-Wadsworth-Emmons Olefinierung: Chem. Rev. 1989, 89, 863-927 und Julia Olefinierung: Tetrahedron Lett., 1973, 14, 4833-4836; Peterson-Olefinierung: J. Org. Chem. 1968, 33, 780; mit Bestmann-Ohira's Reagenz: Synthesis 2004, 1, 59-62).

Eine Verbindung der allgemeinen Formel **(Ie)** wird aus einem Alken der allgemeinen Formel **(IIa)** oder aus einem Alkin der Formel **(IIb)** und Verbindung **(III)** durch eine Zykloadditionsreaktion erhalten (s. z.B. WO 08/013622 und Synthesis, 1987, 11, 998-1001).

Das *Verfahren B* wird in Gegenwart einer geeigneten Base durchgeführt. Bevorzugte Basen sind tertiäre Amine (z.B. Triethylamin), Alkalimetall- oder Erdalkalimetallcarbonate, Hydrogencarbonate und Phosphate.

Das *Verfahren B* wird vorzugsweise unter Verwendung eines oder mehrer Verdünnungsmittel durchgeführt. Bei der Durchführung des *Verfahrens B* kommen vorzugsweise inerte organische Lösungsmittel infrage (wie z.B. Toluol und Hexan). Ebenso kommt Wasser als Lösungsmittel infrage. Alternativ kann das Verfahren A in einem Überschuß des Alkens **(IIa)** oder des Alkins **(IIb)** durchgeführt werden.

Die Aufarbeitung erfolgt nach üblichen Methoden. Falls notwendig werden die Verbindungen durch Umkristallisation oder Chromatographie gereinigt oder können gegebenenfalls auch im nächsten Schritt ohne vorhergehende Reinigung eingesetzt werden.

### Verfahren C

| |
|---|
| 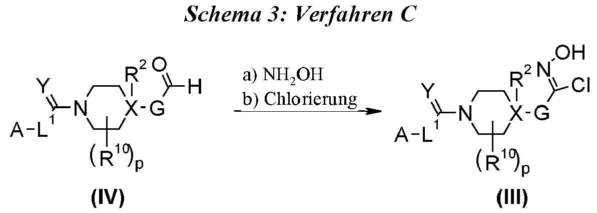 |
| Die Symbole A, G, Y, X, p, L¹, R¹, R², R⁵ und R¹⁰ haben die in der Beschreibung angegebenen Bedeutungen. |

Eine Möglichkeit, das Zwischenprodukt **(III)** aus Verbindung **(IV)** herzustellen, ist in Schema 3 (*Verfahren C*) gezeigt

Eine Verbindung der allgemeinen Formel **(III)** wird durch Kondensation eines Aldehyds der Formel **(IV)** mit Hydroxylamin und anschließender Chlorierung erhalten (s. z.B. WO 05/0040159, WO 08/013622 und Synthesis, 1987, 11, 998-1001).

Im *Verfahren C* werden zunächst Aldehyd **(IV)** und Hydroxylamin zur Reaktion gebracht (Schema 4, Schritt (a)). Das korrespondierende Oxim wird anschließend in Gegenwart eines geeingeneten Chlorierungsmittels chloriert. Bevorzugte Chlorierungsreagenzien sind N-Chlorsuccinimid, HClO und Chlor. Nach dem Schritt (a) des *Verfahrens C* kann die Reaktionsmischung nach üblichen Methoden aufgearbeitet oder direkt im Schritt (b) weiter umgesetzt werden.

Das *Verfahren C* wird vorzugsweise unter Verwendung eines oder mehrer Verdünnungsmittel durchgeführt. Im Schritt (a) des erfindungsgemäßen *Verfahrens C* werden bevorzugt protische Lösungsmittel, wie z.B. Ethanol, als Lösungsmittel verwendet. Nach der Bildung des korrespondierenden Oxims aus Verbindung **(IV)** wird die Reaktionsmischung im Schritt (b) mit einem weiteren Lösungsmittel verdünnt, z.B. Tetrahydrofuran, und anschließend mit wässrigem Natriumhypochlorit versehen. Ebenso kann die Chlorierung mit Hilfe von N-Chlorsuccinimid in DMF erfolgen.

Die Aufarbeitung erfolgt nach üblichen Methoden. Falls notwendig werden die Verbindungen durch Umkristallisation oder Chromatographie gereinigt oder können gegebenenfalls auch im nächsten Schritt ohne vorhergehende Reinigung eingesetzt werden.

### Verfahren D

| |
|---|
| 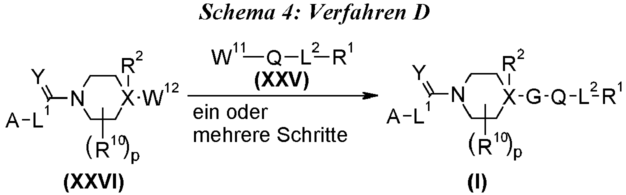 |
| W¹¹ und W¹² sind funktionelle Gruppen geeignet für das Bilden des gewünschten Heterocyclus und die Symbole A, G, Q, Y, p, L¹, L², R¹, R², R⁵ und R¹⁰ haben die in der Beschreibung angegebenen Bedeutungen. |

Im Allgemeinen ist es möglich das Zwischenprodukt der Formel **(I)** aus entsprechenden Verbindungen **(XXVI)** und **(XXV)** mit geeigneten funktionellen Gruppen, W¹¹ und W¹², **(I)** herzustellen (s. Schema 4, *Verfahren D*). Die mögliche funktionelle Gruppen für W¹¹ und W¹² sind z.B. Aldehyde, Ketone, Ester, Carbonsäuren, Amide, Thioamide, Nitrile, Alkohole, Thiole, Hydrazine, Oxime, Amidine, Amideoxidme, Olefine, Acetylene, Halide, Alkylhalide, Methansulfonate, Trifluormethansulfonate, Borsäure, Boronate u.s.w. Sie können unter geeigneten Reaktionsbedingungen den gewünschten 5-gliedrigen Heterocyclus **G** bilden. In der Literatur finden sich zahlreiche Methoden für die Darstellung von Heterocyclen (s. WO 2008/013622; Comprehensive Heterocyclic Chemistry Vol. 4-6, A. R. Katritzky and C. W. Rees editors, Pergamon Press, New York, 1984; Comprehensive Heterocyclic Chemistry II, Vol 2-4, A. R. Katritzky, C. W. Rees and E. F: Scriven editors, Pergamon Press, New York, 1996; The Chemistry of Heterocyclic Compounds, E. C. Taylor, editor, Wiley, New York; Rodd's Chemistry of Carbon Compounds, Vol. 2-4, Elsevier, New York).

### Verfahren E

| |
|---|
| 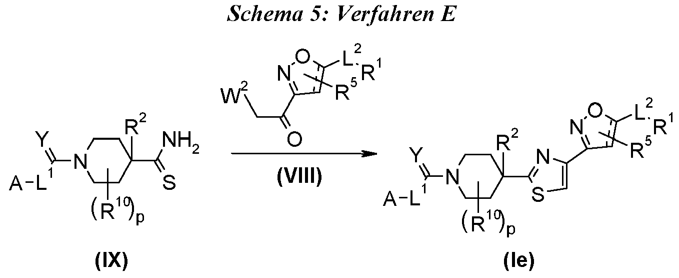 |
| W² steht für eine Abgangsgruppe und die Symbole A, Y, p, L¹, L², R¹, R², R⁵ und R¹⁰ haben die in der Beschreibung angegebenen Bedeutungen. |

Eine bestimmte Möglichkeit zur Synthese von Verbindungen der Formel **(Ie)** aus Verbindungen **(IX)** mit den Verbindungen **(VIII)** ist in Schema 5 (*Verfahren E*) gezeigt.

Thiocarboxamide **(IX)** sind nach literaturbekannten Methoden erhältlich, beispielsweise durch Schwefelung des kommerziell erhältlichen entsprechenden Carboxamids durch Verwendung von z.B. Lawesson's Reagenz (WO2008/013622, Org. Synth. Vol. 7, 1990, 372).

α-Haloketone oder entsprechende Ketone mit einer Abgangsgruppe (z.B. Toluolsulfonyloxyketone) sind auch nach literaturbekannten Methoden erhältlich (Beispiele WO2008/013622), (Schema 6).

| |
|---|
| 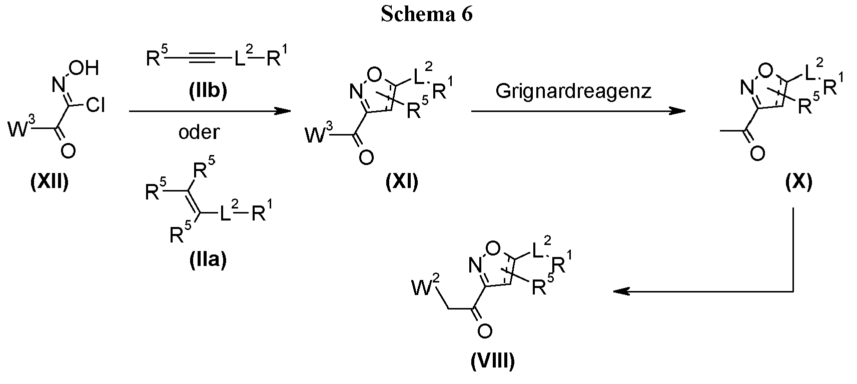 |
| W² steht für eine Abgabgsgruppe, W³ steht für N,N-Dimethylamino, N-Methoxy-N-methylamino oder Morpholin-1-yl und die Symbole L², R¹, R² und R⁵ haben die in der Beschreibung angegebenen Bedeutungen. |

Die Thiazole **(Ie)** werden durch eine Hantzsch-Thiazol-Synthese aus den Thiocarboxamiden **(IX)** und α-Haloketonen oder entsprechende Ketonen mit einer Abgangsgruppe **(VIII)** erhalten (siehe z.B. "Comprehensive Heterocyclic Chemistry", Pergamon Press, 1984; Vol 6, Seite 235-363, "Comprehensive Heterocyclic Chemistry II", Pergamon Press, 1996; Vol 3, Seite 373-474 und darin zitierte Referenzen, und WO 07/014290).

Das *Verfahren E* wird vorzugsweise unter Verwendung eines oder mehrer Verdünnungsmittel durchgeführt. Bei der Durchführung des *Verfahrens E* kommen vorzugsweise inerte organische Lösungsmittel infrage (wie z.B. sind N,N-Dimethylformamid und Ethanol).

Gegebenenfalls wird eine Hilfsbase, wie beispielsweise Triethylamin, verwendet.

Falls notwendig werden die Verbindungen durch Umkristallisation oder Chromatographie gereinigt oder können gegebenenfalls auch im nächsten Schritt ohne vorhergehende Reinigung eingesetzt werden.

### Verfahren F

| |
|---|
| 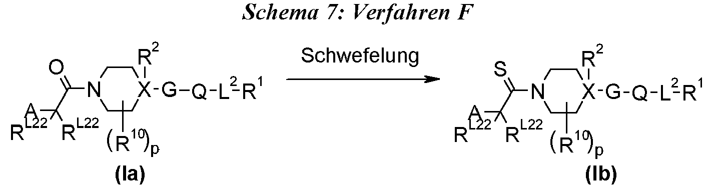 |
| Die Symbole A, Q, G, L², p, R¹, R², R^{L22} und R¹⁰ haben die in der Beschreibung angegebenen Bedeutungen. |

Die bei der Durchführung des erfindungsgemäßen *Verfahrens G* (Schema 8) erhaltenen Amide **(Ia)** lassen sich mittels literaturbeschriebener Methoden in die korrespondierenden Thioamide **(Ib)** überführen (z.B. Bioorganic & Medicinal Chemistry Letters, 2009, 19(2), 462-468). Hierbei werden die Verbindungen der Formel **(Ia)** typischerweise mit Phosphorpentasulfid oder 2,4-Bis(4-methoxyphenyl)-1,3-dithia-2,4-diphosphetan-2,4-disulfid (Lawesson Reagenz) umgesetzt (siehe Schema 7, *Verfahren F*).

Das erfindungsgemäße *Verfahren F* wird vorzugsweise unter Verwendung eines oder mehrerer Verdünnungsmittel durchgeführt. Die bevorzugten Lösungsmittel sind Toluol, Tetrahydrofuran und 1,2-Dimethoxyethan.

Nach Beendigung der Reaktion werden die Verbindungen **(Ib)** von der Reaktionsmischung durch eine der üblichen Trenntechniken getrennt. Falls notwendig werden die Verbindungen durch Umkristallisation oder Chromatographie gereinigt.

### Verfahren G

| |
|---|
| 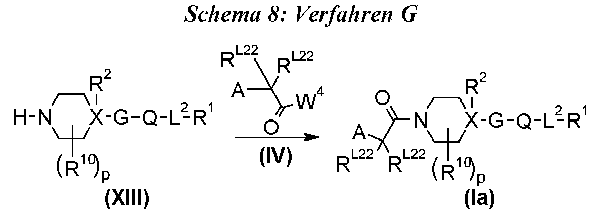 |
| W⁴ = OH oder Cl und die Symbole A, Q, G, L², p, R¹, R², R^{L22} und R¹⁰ haben die in der Beschreibung angegebenen Bedeutungen. |

Eine Möglichkeit, Verbindungen der Formel **(Ia)** aus entsprechenden Verbindungen **(XIII)** mit den Verbindungen **(IV)** darzustellen, ist in Schema 8 (*Verfahren G*) gezeigt

Verbindungen **(IV)** sind entweder kommerziell erhältlich oder durch in der Literatur beschriebene Prozesse darstellbar (s. z.B. WO 2008/013622 und WO 2008/013925).

Eine Verbindung mit der allgemeinen Formel **(Ia)** kann analog zu in der Literatur beschriebenen Vorschriften (s. z.B. WO 2007/147336) durch eine Kupplungsreaktion einer Verbindung mit der entsprechenden allgemeinen Formel **(XIII)** mit einem Substrat der allgemeinen Formel **(IV),** wobei W⁴ für Chlor steht, gegebenenfalls in der Gegenwart eines Säurefängers / Base synthetisiert werden.

Wenigstens ein Äquivalent eines Säurefängers/einer Base (z.B. Hünig Base, Triethylamin oder kommerziell erhältliche polymere Säurefänger) wird im Verhältnis zum Startmaterial der allgemeinen Formel **(XIII)** verwendet. Ist das Startmaterial ein Salz, werden wenigstens zwei Äquivalente des Säurefängers benötigt.

Alternativ kann eine Verbindung der Formel **(Ia)**, auch aus der entsprechenden Verbindung der Formel **(XIII)** mit einem Substrat der Formel **(IV),** wobei W⁴ für Hydroxy steht, in Gegenwart eines Kupplungsreagenzes analog zu in der Literatur beschriebenen Vorschriften synthetisiert werden (z.B. Tetrahedron 2005, 61, 10827-10852, und darin zitierte Referenzen).

Geeignete Kupplungsreagenzien sind beispielsweise Peptidkupplungsreagenzien zum Beispiel, N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-1-yl)-N,N,N',N'-tetramethyluroniumhexafluorophosphat, etc.

Nach Beendigung der Reaktion, werden die Verbindungen **(Ia)** von der Reaktionsmischung durch eine der üblichen Trenntechniken getrennt. Falls notwendig werden die Verbindungen durch Umkristallisation oder Chromatographie gereinigt.

### Verfahren H

| |
|---|
| 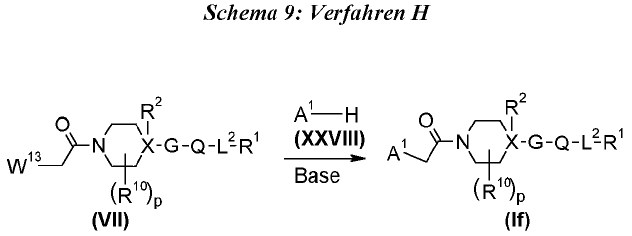 |
| A¹ steht für ein 5-gliedriges Heteroaryl, das bis zu vier Substituenten enthalten kann, wobei die Substituenten am Kohlenstoff unabhängig voneinander ausgewählt sind aus Z^{A-2} und wobei in A¹-H der Wasserstoff über ein Stickstoffatom angebunden ist, W¹³ = Abgangsgruppe und die Symbole A¹, Q, G, L², p, R¹, R², R⁵ und R¹⁰ haben die in der Beschreibung angegebenen Bedeutungen. |

Eine Möglichkeit, Verbindungen der Formel **(If)** aus entsprechenden Verbindungen **(VII)** mit den Verbindungen **(XXVIII)** darzustellen, ist in Schema 9 (*Verfahren H*) gezeigt
Die Ausgangstoffe **(VII),** worin W¹³ für eine Abgangsgruppe steht, lassen sich mittels literaturbeschriebener Methoden aus Verbindungen **(V), (XVIIe) (Abbildung 1)** oder aus **(XIII)** herstellen (s. z.B. Mesylierung: Organic Letters, 2003, 2539-2541; Tosylierung: JP60156601; Halogenierung: Australian Journal of Chemistry, 1983, 2095-2110;). Typischerweise werden die Verbindungen der Formel (**VII**, W¹³ = Chlor) ausgehend von einem Amin der Formel **(XIII)** und Chloracetylchlorid hergestellt. Die Verbindungen **(V)** in **(Abbildung 1)** werden analog zu *Verfahren G* mit Glycolsäure oder Hydroxyacetylchlorid aus **(XIII)** hergestellt (s. z.B. WO 2007103187, WO 2006117521, Bioorganic & Medicinal Chemistry Letters, 2007, 6326-6329).

| |
|---|
| 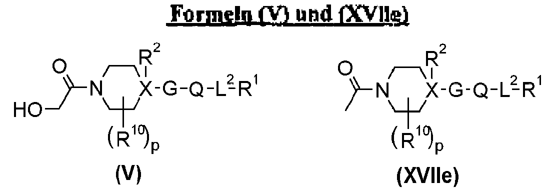 |
| Die Symbole Q, G, L², p, R¹, R², R⁵ und R¹⁰ haben die in der Beschreibung angegebenen Bedeutungen. |

Wenigstens ein Äquivalent einer Base (z.B. Natriumhydrid, Kaliumcarbonat) wird im Verhältnis zum Startmaterial der allgemeinen Formel **(XXVIII)** verwendet.

Nach Beendigung der Reaktion werden die Verbindungen **(If)** von der Reaktionsmischung durch eine der üblichen Trenntechniken getrennt. Falls notwendig werden die Verbindungen durch Umkristallisation oder Chromatographie gereinigt oder können, wenn gewünscht, auch im nächsten Schritt ohne vorhergehende Reinigung eingesetzt werden.

| |
|---|
| 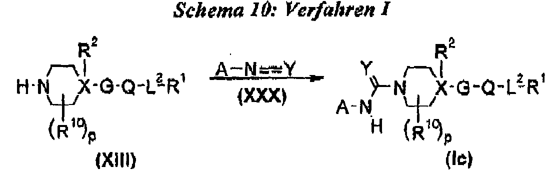 |
| Die Symbole A, Q, G, Y, L², p, R¹, R², R⁵ und R¹⁰ haben die in der Beschreibung angegebenen Bedeutungen. |

Eine Möglichkeit, Verbindungen der Formel **(Ic)** aus entsprechenden Verbindungen **(XIII)** mit den Verbindungen **(XXX)** darzustellen, ist in Schema 10 (*Verfahren 1*) gezeigt.

Eine Verbindung mit der allgemeinen Formel **(Ic)** kann analog zu in der Literatur beschriebenen Vorschriften (s. z.B. WO 2009/055514) durch eine Kupplungsreaktion einer Verbindung mit der entsprechenden allgemeinen Formel **(XIII)** mit einem Substrat der allgemeinen Formel **(XXX),** gegebenenfalls in der Gegenwart eines Säurefängers / Base, wie z.B. Triethylamin, 1,8-Diazabicyclo[5.4.0]undec-7-en oder HUnig-Base synthetisiert werden.

Nach Beendigung der Reaktion, werden die Verbindungen **(Ic)** von der Reaktionsmischung durch eine der üblichen Trenntechniken getrennt. Falls notwendig werden die Verbindungen durch Umkristallisation oder Chromatographie gereinigt.

### Verfahren J

| |
|---|
| 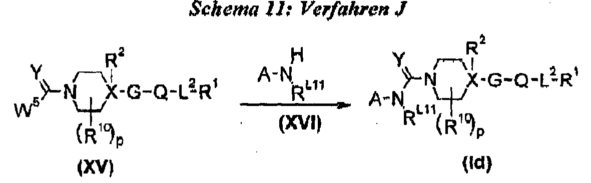 |
| W⁵ steht für Chlor oder Imidazol-1-yl und die Symbole A, G, Q, Y, L², p, R¹, R², R^{L11} und R¹⁰ haben die in der Beschreibung angegebenen Bedeutungen. |

Eine Möglichkeit, Verbindungen der Formel **(Id)** aus entsprechenden Verbindungen **(XVa,** siehe Abbildung 2) oder **(XVb,** siehe Abbildung 2) mit den Verbindungen **(XVI)** darzustellen, ist in Schema 11 (*Verfahren J*) gezeigt.

| |
|---|
| 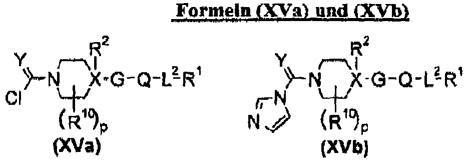 |
| Die Symbole Q, G, L², p, R¹, R², R⁵ und R¹⁰ haben die in der Beschreibung angegebenen Bedeutungen. |

Die Ausgangstoffe, Carbamoyl- und Thiocarbamoylchloride der Formel (**XVa**), lassen sich mittels literaturbeschriebener Methoden aus Verbindungen **(XIII)** herstellen (s. z.B. Tetrahedron, 2008, 7605; Journal of Organic Chemistry, 2004, 3787; Journal of Organic Chemistry, 1983, 4750; European Journal of Organic Chemistry, 2006, 1177). Typischerweise werden die Verbindungen der Formel **(XVa)** ausgehend von Aminen der Formel **(XIII)** und Phosgen, Thiophosgen oder deren Äquivalenten hergestellt.

Die alternativen Ausgangstoffe, Carbamoyl- und Thiocarbamoylimidazole der Formel **(XVb),** lassen sich mittels literaturbeschriebener Methoden herstellen (s. z.B. Tetrahedron Letters, 2008, 5279; Tetrahedron, 2005, 7153). Typischerweise werden die Verbindungen der Formel **(XVb,** W⁵ = imidazol-1-yl) ausgehend von Aminen der Formel **(XIII)** und 1,1'-Carbonyldiimidazole oder 1,1'-Thiocarbonyldimidazole hergestellt.

Das *Verfahren J* wird gegebenenfalls in Gegenwart eines geeigneten Säureakzeptors durchgeführt.

Die bei der Durchführung des erfindungsgemäßen *Verfahren J* erhaltenen Verbindungen **(Id)** können alternativ teilweise auch ohne Verwendung eines Säureakzeptors als korrespondierende Säurechloride [**(Id)**-HCl] erhalten werden. Bei Bedarf erfolgt die Freisetzung der Verbindungen **(Id)** nach üblichen Methoden.

Nach Beendigung der Reaktion, werden die Verbindungen **(Id)** von der Reaktionsmischung durch eine der üblichen Trenntechniken getrennt. Falls notwendig werden die Verbindungen durch Umkristallisation oder Chromatographie gereinigt.

### Verfahren K

| |
|---|
| 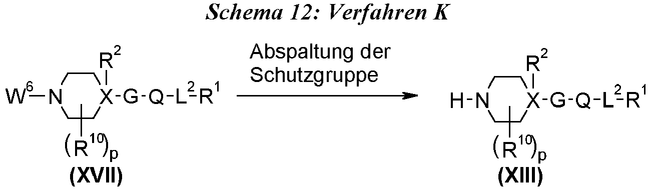 |
| W⁶ steht für Acetyl, C₁-C₄-Alkoxycarbonyl, Benzyl oder Benzyloxycarbonyl und die Symbole Q, L², p, R¹, R² und R¹⁰ haben die in der Beschreibung angegebenen Bedeutungen. |

Eine Möglichkeit Verbindungen der Formel **(XIII)** aus entsprechenden Verbindungen **(XVII)** darzustellen ist in Schema 12 (*Verfahren K*) gezeigt.

Eine Verbindung der Formel **(XVII)** wird in eine Verbindung der Formel **(XIII)** durch geeignete Methoden zur Entfernung von Schutzgruppen, die in der Literatur beschrieben sind ("Protective Groups in Organic Synthesis"; Theodora W. Greene, Peter G. M. Wuts; Wiley-Interscience; Third Edition; 1999; 494-653), überführt.
*tert*-Butoxycarbonyl- und Benzyloxycarbonylschutzgruppen können im sauren Medium entfernt werden (z.B mit Salzsäure oder der Trifluoressigsäure). Acetylschutzgruppen können unter basischen Bedingungen (z.B. mit Kaliumcarbonat oder Cäsiumcarbonat) entfernt werden. Benzylische Schutzgruppen können hydrogenolytisch mit Wasserstoff in Gegenwart eines Katalysators (z.B. Palladium auf Aktivkohle) entfernt werden.

Säuren, die für diese Reaktion, der Entschützung von *tert*-Butoxycarbonyl und Benzyloxycarbonyl Gruppen verwendet werden können, sind z.B. Trifluoressigsäure, Salzsäure oder andere Säuren, wie sie in der Literatur beschrieben sind (z.B. *"*Protective Groups in Organic Synthesis"; Theodora W. Greene, Peter G. M. Wuts; Wiley-Interscience; Third Edition; 1999; 494-653).

Nach Beendigung der Reaktion werden die Verbindungen **(XIII)** von der Reaktionsmischung durch eine der üblichen Trenntechniken getrennt. Falls notwendig werden die Verbindungen durch Umkristallisation oder Chromatographie gereinigt oder können, wenn gewünscht, auch im nächsten Schritt ohne vorhergehende Reinigung eingesetzt werden. Es ist außerdem möglich, die Verbindung der allgemeinen Formel **(XIII)** als Salz zu isolieren, z.B. als Salz der Salzsäure oder der Trifluoressigsäure.

### Verfahren L

| |
|---|
| 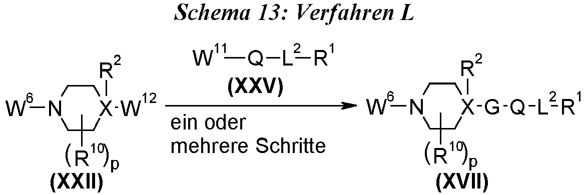 |
| W⁶ steht für Acetyl, C₁-C₄-Alkoxycarbonyl, Benzyl oder Benzyloxycarbonyl, W¹¹ und W¹² sind funktionelle Gruppen geeignet für das Bilden des gewünschten Heterocyclus und die Symbole Q, L², p, R¹, R² und R¹⁰ haben die in der Beschreibung angegebenen Bedeutungen. |

Im Allgemeinen ist es möglich, das Zwischenprodukt **(XVII)** aus entsprechenden Verbindungen **(XXII)** mit Verbindungen **(XXV)** herzustellen. *Verfahren L* (Schema 13) wird analog *Verfahren D* (Schema 4) durchgeführt.

### Verfahren M

| |
|---|
| 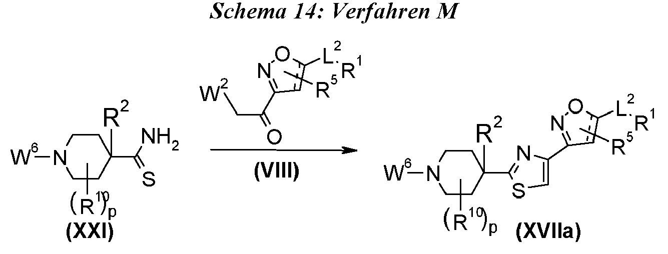 |
| W² steht für eine Abgangsgruppe, W⁶ steht für Acetyl, C₁-C₄-Alkoxycarbonyl, Benzyl oder Benzyloxycarbonyl und die Symbole L², p, R¹, R², R⁵ und R¹⁰ haben die in der Beschreibung angegebenen Bedeutungen. |

Eine andere Möglichkeit, das Zwischenprodukt der Formel **(XVIIa)** aus entsprechenden Verbindungen **(XXI)** herzustellen, ist in Schema 14 (*Verfahren M*) gezeigt. Verbindungen **(XXI)** sind entweder kommerziell erhältlich oder durch in der Literatur beschriebene Prozesse darstellbar (s. z.B. WO 2008/013622 und WO 2007/014290). *Verfahren M* wird analog *Verfahren E* (Schema 5) durchgeführt.

### Verfahren N

| |
|---|
| 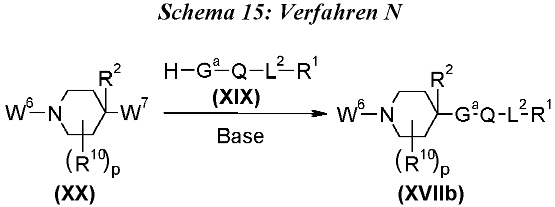 |
| G^{a} steht für 5-gliedriges Heteroaryl; bei den Verbindungen der Formel **(XVIIb)** ist das 5-gliedrige Heteroaryl über ein Stickstoffatom mit dem Piperidin-Rest verbunden, W⁶ steht für Acetyl, C₁-C₄-Alkoxycarbonyl, Benzyl oder Benzyloxycarbonyl, W⁷ steht für eine Abgangsgruppe und die Symbole Q, L², p, R¹, R² und R¹⁰ haben die in der Beschreibung angegebenen Bedeutungen. |

Eine Verbindung mit der allgemeinen Formel **(XVIIb)** kann analog zu in der Literatur beschriebenen Vorschriften durch eine Kupplungsreaktion einer Verbindung mit der entsprechenden allgemeinen Formel **(XX)** mit einem Substrat der allgemeinen Formel **(XIX)** gegebenenfalls in der Gegenwart einer Base synthetisiert werden (Schema 15, *Verfahren N*). (s. z.B. für Zn/Pd-Kupplung: WO2008/147831, WO 2006/106423 (Pyridin), Shakespeare, W. C. et al Chem. Biol. Drug Design 2008, 71, 97-105 (PyrimidinDerivate), Pasternak, A. et al Bioorg. Med. Chem. Lett. 2008, 18, 994-998 (Diazine); Coleridge, B. M.; Bello, C. S.; Leitner, A. Tetrahedron Lett. 2009, 50, 4475-4477; Bach, T., Heuser, S. Angew. Chem. Int. Ed. 2001, 40, 3184-3185. (Thiazole); für nucleophile Substitutionen: WO2008/104077; WO2006/084015 (Pyrazole mit N-Substitution)

Wenigstens ein Äquivalent einer Base (z.B. Natriumhydrid, Kaliumcarbonat) wird im Verhältnis zum Startmaterial der allgemeinen Formel **(XX)** verwendet.

Nach Beendigung der Reaktion werden die Verbindungen **(XVIIb)** von der Reaktionsmischung durch eine der üblichen Trenntechniken getrennt. Falls notwendig werden die Verbindungen durch Umkristallisation oder Chromatographie gereinigt oder können, wenn gewünscht, auch im nächsten Schritt ohne vorhergehende Reinigung eingesetzt werden.

### Verfahren O

| |
|---|
| 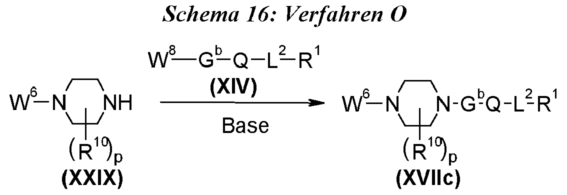 |
| G^{b} steht für 5-gliedriges Heteroaryl; bei den Verbindungen der Formel **(XVIIc)** ist das 5-gliedrige Heteroaryl über ein Kohlenstoffatom mit dem Piparazin-Ring verbunden, W⁶ steht für Acetyl, C₁-C₄-Alkoxycarbonyl, Benzyl oder Benzyloxycarbonyl, W⁸ steht für eine Abgangsgruppe und die Symbole Q, L², p, R¹ und R¹⁰ haben die in der Beschreibung angegebenen Bedeutungen. |

Eine Verbindung mit der allgemeinen Formel **(XVIIc)** kann analog zu in der Literatur beschriebenen Vorschriften (s. z.B. für nucleophile Substitutionen: Li, C. S., Belair, L., Guay, J. et al Bioorg. Med. Chem. Lett. 2009, 19, 5214-5217; WO 2008/062276; für Kupferkupplungen: Yeh, V. S. C.; Wiedeman, P. E. Tetrahedron Lett. 2006, 47, 6011-6016; für Palladiumkupplungen: WO 2008/157500) durch eine Kupplungsreaktion einer Verbindung mit der entsprechenden allgemeinen Formel **(XXIX)** mit einem Substrat der allgemeinen Formel **(XIV)** gegebenenfalls in der Gegenwart eines Base synthetisiert werden (Schema 16, *Verfahren O*).

Wenigstens ein Äquivalent einer Base (z.B. Natriumhydrid, Kaliumcarbonat) wird im Verhältnis zum Startmaterial der allgemeinen Formel **(XXIX)** verwendet.

Nach Beendigung der Reaktion werden die Verbindungen **(XVIIc)** von der Reaktionsmischung durch eine der üblichen Trenntechniken getrennt. Falls notwendig werden die Verbindungen durch Umkristallisation oder Chromatographie gereinigt oder können, wenn gewünscht, auch im nächsten Schritt ohne vorhergehende Reinigung eingesetzt werden.

### Verfahren P

| |
|---|
| 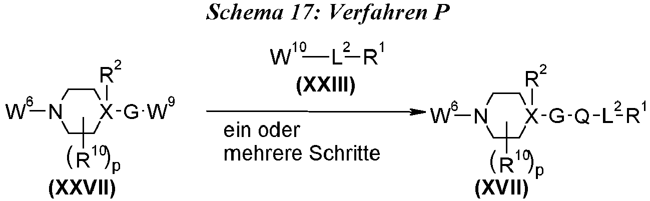 |
| W⁶ steht für Acetyl, C₁-C₄-Alkoxycarbonyl, Benzyl oder Benzyloxycarbonyl, W⁹ und W¹⁰ sind funktionelle Gruppen, die für das Bilden des gewünschten Heterocyclus geeignet sind und die Symbole Q, L², G, p, R¹, R² und R¹⁰ haben die in der Beschreibung angegebenen Bedeutunge. |

Im Allgemeinen ist es möglich, das Zwischenprodukt **(XVII)** aus entsprechenden Verbindungen **(XXVII)** und **(XXIII)** herzustellen. *Verfahren P* (Schema 17) wird analog *Verfahren A* (Schema 1) durchgeführt.

### Verfahren Q

| |
|---|
| 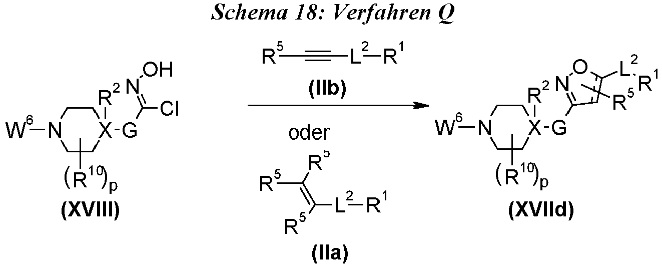 |
| W⁶ steht für Acetyl, C₁-C₄-Alkoxycarbonyl, Benzyl oder Benzyloxycarbonyl und in die Symbole X L², G, p, R¹, R², R⁵ und R¹⁰ haben die in der Beschreibung angegebenen Bedeutungen. |

Eine bestimmte Möglichkeit, das Zwischenprodukt **(XVIId)** aus entsprechenden Verbindungen **(XVIII)** herzustellen, ist in Schema 18 (*Verfahren Q*) gezeigt. Die Ausgangstoffe der Formel **(XVIII)** lassen sich mittels literaturbeschriebener Methoden herstellen (s. z.B. WO 2008/013622), und *Verfahren Q* wird analog *Verfahren B* (Schema 2) durchgeführt.

Ein weiterer Gegenstand der Erfindung betrifft die nichtmedizinische Verwendung der erfindungsgemäßen Heteroarylpiperidin und -piperazinderivate der Formel **(I)** zum Bekämpfen unerwünschter Mikroorganismen.

Neu sind Verbindungen der Formel **(VII),** 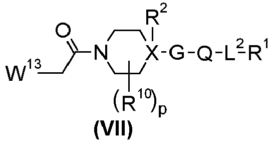 sowie Salze, Metallkomplexe und N-Oxide davon, in denen die Symbole R², X, G, Q, L², R¹, R¹⁰ und p die oben angegebenen allgemeinen, bevorzugten, besonders bevorzugten oder ganz besonders bevorzugten Bedeutungen haben und W¹³ für eine Abgangsgruppe steht.

Neu sind Verbindungen der Formel **(XVIId),** 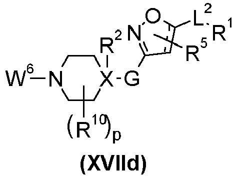 wie zum Beispiel **(XVIIf),** 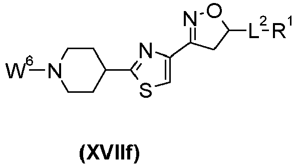 sowie Salze Metallkomplexe und N-Oxide davon, in denen die Symbole W⁶ für Acetyl, C₁-C₄ Alkoxycarbonyl, Benzyl oder Benzyloxycarbonyl steht und die Reste X, L², p, R¹, R², R⁵ und R¹⁰ die oben angegebenen allgemeinen, bevorzugten, besonders bevorzugten oder ganz besonders bevorzugten Bedeutungen haben.

Neu sind Verbindungen der Formel **(XIIIa),** 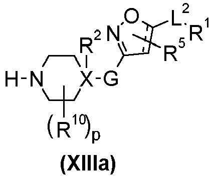 wie zum Beispiel **(XIIIb),** 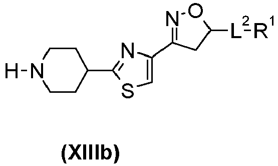 sowie Salze, Metallkomplexe und N-Oxide davon, in denen die Symbole X, L², p, G, R¹, R², R⁵ und R¹⁰ die oben angegebenen allgemeinen, bevorzugten, besonders bevorzugten oder ganz besonders bevorzugten Bedeutungen haben.

Ein weiterer Gegenstand der Erfindung betrifft ein Mittel zum Bekämpfen unerwünschter Mikroorganismen, umfassend wenigstens ein Heteroarylpiperidin und -piperazinderivat gemäß der vorliegenden Erfindung.

Außerdem betrifft die Erfindung ein Verfahren zum Bekämpfen unerwünschter Mikroorganismen, dadurch gekennzeichnet, dass die erfindungsgemäßen Heteroarylpiperidin und -piperazinderivate auf die Mikroorganismen und/oder in deren Lebensraum ausgebracht werden.

Weiterhin betrifft die Erfindung ein Saatgut, welches mit wenigstens einem erfindungsgemäßen Heteroarylpiperidin und -piperazinderivate behandelt wurde.

Ein letzter Gegenstand der Erfindung betrifft ein Verfahren zum Schutz von Saatgut vor unerwünschten Mikroorganismen durch Verwendung eines mit wenigstens einem Heteroarylpiperidin und - piperazinderivat gemäß der vorliegenden Erfindung behandelten Saatgutes.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die erfindungsgemäßen Heteroarylpiperidin und -piperazinderivate der Formel **(I)** besitzen sehr gute fungizide Eigenschaften und lassen sich im Pflanzenschutz beispielsweise zur Bekämpfung von Plasmodiophoromyceten, Oomyceten, Chytridiomyceten, Zygomyceten, Ascomyceten, Basidiomyceten und Deuteromyceten einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und - plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen..

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi oder Phakopsora meibomiae; Puccinia-Arten, wie beispielsweise Puccinia recondita, Puccinia graminis oder Puccinia striiformis; Uromyces-Arten, wie beispielsweise Uromyces appendiculatus;
Erkrankungen, hervorgerufen durch Erreger der Gruppe der Oomyceten wie z.B. Albugo-Arten, wie beispielsweise Albugo candida; Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladosporium-Arten, wie beispielsweise Cladosporium cucumerinum; Cochliobolus-Arten, wie beispielsweise Cochliobolus sativus (Konidienform: Drechslera, Syn: Helminthosporium) oder Cochliobolus miyabeanus; Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola, Mycosphaerella arachidicola oder Mycosphaerella fijiensis; Phaeosphaeria-Arten, wie beispielsweise Phaeosphaeria nodorum; Pyrenophora-Arten, wie beispielsweise Pyrenophora teres oder Pyrenophora tritici repentis; Ramularia-Arten, wie beispielsweise Ramularia collocygni oder Ramularia areola; Rhynchosporium-Arten, wie beispielsweise Rhynchosporium secalis; Septoria-Arten, wie beispielsweise Septoria apii oder Septoria lycopersici; Stagonospora-Arten, wie beispielsweise Stagonospora nodorum; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Plasmodiophora-Arten, wie beispielsweise Plasmodiophora brassicae; Rhizoctonia-Arten, wie beispielsweise Rhizoctonia solani; Sarocladium-Arten, wie beispielsweise Sarocladium oryzae; Sclerotium-Arten, wie beispielsweise Sclerotium oryzae; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Stagonospora-Arten, wie beispielsweise Stagonospora nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries oder Tilletia controversa; Urocystis-Arten, wie beispielsweise Urocystis occulta; Ustilago-Arten, wie beispielsweise Ustilago nuda;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum oder Penicillium purpurogenum; Rhizopus -Arten, wie beispielsweise Rhizopus stolonifer; Sclerotinia-Arten, wie beispielsweise Sclerotinia sclerotiorum; Verticilium-Arten, wie beispielsweise Verticilium alboatrum;
Samen- und bodenbürtige Fäulen und Welken, sowie Sämlingserkrankungen, hervorgerufen durch z.B. Alternaria-Arten, wie beispielsweise Alternaria brassicicola; Aphanomyces-Arten, wie beispielsweise Aphanomyces euteiches; Ascochyta-Arten, wie beispielsweise Ascochyta lentis; Aspergillus-Arten, wie beispielsweise Aspergillus flavus; Cladosporium-Arten, wie beispielsweise Cladosporium herbarum; Cochliobolus-Arten, wie beispielsweise Cochliobolus sativus (Konidienform: Drechslera, Bipolaris Syn: Helminthosporium); Colletotrichum-Arten, wie beispielsweise Colletotrichum coccodes; Fusarium-Arten, wie beispielsweise Fusarium culmorum; Gibberella-Arten, wie beispielsweise Gibberella zeae; Macrophomina-Arten, wie beispielsweise Macrophomina phaseolina; Microdochium-Arten, wie beispielsweise Microdochium nivale; Monographella-Arten, wie beispielsweise Monographella nivalis; Penicillium-Arten, wie beispielsweise Penicillium expansum; Phoma-Arten, wie beispielsweise Phoma lingam; Phomopsis-Arten, wie beispielsweise Phomopsis sojae; Phytophthora Arten, wie beispielsweise Phytophthora cactorum; Pyrenophora-Arten, wie beispielsweise Pyrenophora graminea; Pyricularia-Arten, wie beispielsweise Pyricularia oryzae; Pythium-Arten, wie beispielsweise Pythium ultimum; Rhizoctonia-Arten, wie beispielsweise Rhizoctonia solani; Rhizopus-Arten, wie beispielsweise Rhizopus oryzae; Sclerotium-Arten, wie beispielsweise Sclerotium rolfsii; Septoria-Arten, wie beispielsweise Septoria nodorum; Typhula-Arten, wie beispielsweise Typhula incarnata; Verticillium-Arten, wie beispielsweise Verticillium dahliae;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Exobasidium-Arten, wie beispielsweise Exobasidium vexans; Taphrina-Arten, wie beispielsweise Taphrina deformans;
Degenerationserkrankungen holziger pflanzen, hervorgerufen durch z.B. Esca-Arten, wie beispielsweise Phaeomoniella chlamydospora, Phaeoacremonium aleophilum oder Fomitiporia mediterranea; Ganoderma-Arten, wie beispielsweise Ganoderma boninense;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst-, Kartoffel- und Gemüseanbau, wie beispielsweise insbesondere gegen falsche Mehltaupilze, Oomyceten, wie beispielsweise Phytophthora-, Plasmopara-, Pseudoperonospora- und Pythium-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Insektizide verwenden. Sie lassen sich gegebenenfalls auch als Zwischen- oder Vorprodukte für die Synthese weiterer Wirkstoffe einsetzen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes in der Landwirtschaft, im Gartenbau, bei der Tierzucht, in Forsten, in Gärten und Freizeiteinrichtungen, im Vorrats- und Materialschutz sowie auf dem Hygienesektor. Sie können vorzugsweise als Pflanzenschutzmittel eingesetzt werden. Sie sind gegen normal sensible und resistente Arten sowie gegen alle oder einzelne Entwicklungsstadien wirksam.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von so genannte Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die vorliegende Erfindung betrifft weiterhin ein Mittel zum Bekämpfen unerwünschter Mikroorganismen, umfassend wenigstens eines der erfindungsgemäßen Heteroarylpiperidin und - piperazinderivate. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Die Wirkstoffe können als solche, in Form ihrer Formulierungen oder den daraus bereiteten Anwendungsformen, wie gebrauchsfertige Lösungen, Emulsionen, wasser- oder ölbasierte Suspensionen, Pulver, Spritzpulver, Pasten, lösliche Pulver, Stäubemittel, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die vorliegende Erfindung umfasst nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die zuvor beschriebenen Formulierungen können in einem erfindungsgemäßen Verfahren zum Bekämpfen unerwünschter Mikroorganismen verwendet werden, bei dem die erfindungsgemäßen Heteroarylpiperidin und -piperazinderivate auf die Mikroorganismen und/oder in deren Lebensraum ausgebracht werden.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 14th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Anwendung geschieht in einer den Anwendungsformen angepaßten üblichen Weise.

Die Erfindung umfasst weiterhin ein Verfahren zur Behandlung von Saatgut.

Ein weiterer Aspekt der vorliegenden Erfindung betrifft insbesondere ein Saatgut, welches mit wenigstens einem der erfindungsgemäßen Heteroarylpiperidin und -piperazinderivaten behandelt ist. Die erfindungsgemäßen Saatgüter finden Anwendung in Verfahren zum Schutz von Saatgut vor pflanzenpathogenen Schadpilzen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von pflanzenpathogenen Schadpilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von tierischen Schädlingen und/oder pflanzenpathogenen Schadpilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von tierischen Schädlingen und/oder pflanzenpathogenen Schadpilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor tierischen Schädlingen und/oder pflanzenpathogenen Schadpilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417, US 4,245,432, US4,808,430, US 5,876,739, US 2003/0176428, WO 2002/080675, WO 2002/028186.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Darüber hinaus weisen die erfindungsgemäßen Verbindungen der Formel **(I)** auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe der Formel **(I)** können daher sowohl in medizinischen als auch in nicht-medizinischen Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpem, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiterhin können die erfindungsgemäßen Verbindungen allein oder in Kombinationen mit anderen Wirkstoffen als Antifouling-Mittel eingesetzt werden.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte. In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosateacetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere Sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine , **die online bei**:
   http://www.lifesci.sussex.ac.uk/Home/Neil_Crickmore/Bt/ beschrieben sind, zusammengestellt wurden, oder insektizide Teile davon, z.B. Proteine der Cry-Proteinklassen CrylAb, CrylAc, CrylF, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bbl in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekommorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die aufgeführten Pflanzen können besonders vorteilhaft erfindungsgemäß mit den Verbindungen der allgemeinen Formel **(I)** bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Die Herstellung und die Verwendung der erfindungsgemäßen Wirkstoffe der Formeln **(I)** geht aus den folgenden Beispielen hervor. Die Erfindung ist jedoch nicht auf diese Beispiele beschränkt.

**Allgemeines:** Wenn nicht anders angegeben, werden alle chromatografischen Reinigungs- bzw. Trennungsschritte an Kieselgel und mit einem Lösungsmittelgradienten von 0:100 Essigsäure-ethylester/Cyclohexan zu 100:0 Essigsäure-ethylester/Cyclohexan durchgeführt.

### Herstellung von Verbindung (1-5)

### Schritt 1

### tert-Butyl-4-[4-(5-{2-[(methylsulfonyl)amino]phenyl}-4,5-dihydro-1,2-oxazol-3-yl)-1,3-thiazol-2-yl]piperidin-1-carboxylat (XVIIf-1)

Zu einer Lösung von *tert*-Butyl-4-{4-[(Z/E)-(hydroxyimino)methyl]-1,3-thiazol-2-yl}piperidin-1-carboxylat (1,35 g) und N-(2-Vinylphenyl)methansulfonamid (1,11 g) in Ethylacetat (20 mL) wurde bei Raumtemperatur Kaliumhydrogencarbonat (2,17 g) und N-Chlorsuccinimid (0,58 g) gegeben und anschließend drei Tropfen Wasser. Die Reaktionsmischung wurde über Nacht bei 50 °C gerührt, dann mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-[4-(5-{2-[(methylsulfonyl)amino]phenyl}-4,5-dihydro-1,2-oxazol-3-yl)-1,3-thiazol-2-yl]piperidin-1-carboxylat (0,94 g).

### Schritt 2

### 4-[4-(5-{2-[(Methylsulfonyl)ammonio]phenyl}-4,5-dihydro-1,2-oxazol-3-yl)-1,3-thiazol-2-yl]piperidiniumdichlorid (XIIIb-1)

Zu einer Lösung von *tert*-Butyl-4-[4-(5-{2-[(methylsulfonyl)amino]phenyl}-4,5-dihydro-1,2-oxazol-3-yl)-1,3-thiazol-2-yl]piperidin-1-carboxylat (900 mg) in 1,4-Dioxan (10 mL) wurde bei 0 °C eine 4-molare Lösung von Chlorwasserstoff in 1,4-Dioxan (6.66 mL) getropft. Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 4-[4-(5-{2-[(Methylsulfonyl)ammonio]phenyl}-4,5-dihydro-1,2-oxazol-3-yl)-1,3-thiazol-2-yl]piperidiniumdichlorid (1,00 g).

### Schritt 3

### N-(2-{3-[2-(1-{[5-Methyl-3-(trifluormethyl)-1H-pyrazol-1-yl]acetyl}piperidin-4-yl)-1,3-thiazol-4-yl]-4,5-dihydro-1,2-oxazol-5-yl}phenyl)methansulfonamid (I-5)

4-[4-(5-{2-[(Methylsulfonyl)ammonio]phenyl}-4,5-dihydro-1,2-oxazol-3-yl)-1,3-thiazol-2-yl]piperidiniumdichlorid (201 mg), [5-Methyl-3-(trifluormethyl)-1H-pyrazol-1-yl]essigsäure (104 mg) und Triethylamin (51 mg) werden in Dichlormethan (10 mL) gelöst und für 10 min gerührt. Das Gemisch weitere 10 min gerührt, bevor Brom-tris-pyrrolidino-phosphoniumhexafluorophosphat (254 mg) zugegeben wird. Die Reaktionsmischung wird für 2 Stunden bei Raumtemperatur gerührt. Nach Entfernen des Lösungsmittels unter vermindertem Druck wird der Rückstand chromatografisch gereinigt. Man erhält N-(2-{3-[2-(1-{[5-Methyl-3-(trifluormethyl)-1H-pyrazol-1-yl]acetyl}piperidin-4-yl)-1,3-thiazol-4-yl]-4,5-dihydro-1,2-oxazol-5-yl}phenyl)methansulfonamid (107 mg).

### Herstellung von Verbindung (1-4)

### Schritt 1

### N-(2-{3-[2-(1-{[2,5-Bis(difluormethyl)phenyl]acetyl}piperidin-4-yl)-1,3-thiazol-4-yl]-4,5-dihydro-1,2-oxazol-5-yl}phenyl)methansulfonamid (I-4)

4-[4-(5-{2-[(Methylsulfonyl)ammonio]phenyl}-4,5-dihydro-1,2-oxazol-3-yl)-1,3-thiazol-2-yl]piperidiniumdichlorid (298 mg) und [2,5-Bis(difluormethyl)phenyl]essigsäure (175 mg) wurden analog zu **I-5** (Schritt 3) umgesetzt. Man erhielt N-(2-{3-[2-(1-{[2,5-Bis(difluormethyl)phenyl]acetyl}piperidin-4-yl)-1,3-thiazol-4-yl]-4,5-dihydro-1,2-oxazol-5-yl}phenyl)methansulfonamid (170 mg).

### Herstellung von Verbindung (1-6)

### Schritt 1

### N-[2,5-Bis(difluormethyl)phenyl]-4-[4-(5-{2-[(methylsulfonyl)amino]phenyl}-4,5-dihydro-1,2-oxazol-3-yl)-1,3-thiazol-2-yl]piperidin-1-carboxamid (I-6)

Zu einer Suspension von 4-[4-(5-{2-[(Methylsulfonyl)ammonio]phenyl}-4,5-dihydro-1,2-oxazol-3-yl)-1,3-thiazol-2-yl]piperidiniumdichlorid (202 mg) in Dichlormethan (5 mL) und Triethylamin (51 mg) wurde bei Raumtemperatur 1,4-Bis(difluormethyl)-2-isocyanatobenzol (100 mg) und ein Tropfen 1,8-Diazabicyclo[5.4.0]undec-7-en (DBU) gegeben. Das Gemisch wurde bei Raumtemperatur für 2 Stunden gerührt und dann mit gesättigter Ammoniumchloridlösung versetzt. Die wässrige Phase wurde abgetrennt und mit Ethylacetat extrahiert. Die vereinigten organischen Phasen wurden über Natriumsulfat getrocknet und unter vermindertem Druck eingeengt. Der Rückstand wurde chromatographisch gereinigt. Man erhielt N-[2,5-Bis(difluormethyl)phenyl]-4-[4-(5-{2-[(methylsulfonyl)amino]phenyl}-4,5-dihydro-1,2-oxazol-3-yl)-1,3-thiazol-2-yl]piperidin-1-carboxamid (150 mg).

### Herstellung von Verbindungen der Formel (1-3)

### Schritt 1

### tert-Butyl-4-{4-[5-(2-formylphenyl)-4,5-dihydro-1,2-oxazol-3-yl]-1,3-thiazol-2-yl}piperidin-1-carboxylat (XVIIf-2)

Zu einer Lösung von *tert*-Butyl-4-{4-[(Z/E)-(hydroxyimino)methyl]-1,3-thiazol-2-yl}piperidin-1-carboxylat (5,6 g) und 2-Vinylbenzaldehyd (2,61 g) in Ethylacetat (70 mL) wurde bei Raumtemperatur Kaliumhydrogencarbonat (7,23 g) und N-Chlorsuccinimid (2,31 g) gegeben und anschließend drei Tropfen Wasser. Die Reaktionsmischung wurde für 3 Stunden bei 60 °C gerührt, dann mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. tert-Butyl-4-{4-[5-(2-formylphenyl)-4,5-dihydro-1,2-oxazol-3-yl]-1,3-thiazol-2-yl}piperidin-1-carboxylat (5,69 g).

### Schritt 2

### 4-{4-[5-(2-Formylphenyl)-4,5-dihydro-1,2-oxazol-3-yl]-1,3-thiazol-2-yl}piperidiniumchlorid (XIIIb-2)

*tert*-Butyl-4-{4-[5-(2-formylphenyl)-4,5-dihydro-1,2-oxazol-3-yl]-1,3-thiazol-2-yl}piperidin-1-carboxylat (5,1 g) wurde analog zu **I-5** (Schritt 2) umgesetzt. Man erhielt 4-{4-[5-(2-Formylphenyl)-4,5-dihydro-1,2-oxazol-3-yl]-1,3-thiazol-2-yl}piperidiniumchlorid (4,35 g).

### Schritt 3

### N-(5-Chlor-2-methylphenyl)-4-{4-[5-(2-formylphenyl)-4,5-dihydro-1,2-oxazol-3-yl]-1,3-thiazol-2-yl}piperidin-1-carboxamid (I-3)

Zu einer Suspension von 4-{4-[5-(2-Formylphenyl)-4,5-dihydro-1,2-oxazol-3-yl]-1,3-thiazol-2-yl}piperidiniumchlorid (1.44 g) in Dichlormethan (30 mL) und Diisopropylethylamin (0.73 ml) wurde bei Raumtemperatur 4-Chlor-2-isocyanato-1-methylbenzol (700 mg) und ein Tropfen 1,8-Diazabicyclo[5.4.0]undec-7-en (DBU) gegeben. Das Gemisch wurde bei Raumtemperatur über Nacht gerührt und dann mit Wasser versetzt. Die wässrige Phase wurde abgetrennt und mit Ethylacetat extrahiert. Die vereinigten organischen Phasen wurden über Natriumsulfat getrocknet und unter vermindertem Druck eingeengt. Der Rückstand wurde chromatographisch gereinigt. Man erhielt N-(5-Chlor-2-methylphenyl)-4-{4-[5-(2-formylphenyl)-4,5-dihydro-1,2-oxazol-3-yl]-1,3-thiazol-2-yl}piperidin-1-carboxamid (1.23 g).

### Herstellung von Verbindung (1-7)

### 2-{3-[2-(1-{[5-Methyl-3-(trifluormethyl)-1H-pyrazol-1-yl]acetyl}piperidin-4-yl)-1,3-thiazol-4-yl]-4,5-dihydro-1,2-oxazol-5-yl}benzaldehyd (I-7)

Zu einer Lösung von [5-Methyl-3-(trifluormethyl)-1H-pyrazol-1-yl]essigsäure (900 mg) in Dichlormethan (20 mL) werden bei 0 °C Oxalylchlorid (1.50 g) und ein Tropfen N,N-Dimethylformamid gegeben. Das Reaktionsgemisch wird bei Raumtemperatur für 2 Stunden gerührt. Das Lösungsmittel und das überschüssige Reagenz werden unter vermindertem Druck entfernt. Der feste Rückstand wird erneut in Dichlormethan gelöst und bei 0 °C tropfenweise zu einer Lösung von 4-{4-[5-(2-Formylphenyl)-4,5-dihydro-1,2-oxazol-3-yl]-1,3-thiazol-2-yl}piperidiniumchlorid (1.49 g) und Diisopropylethylamin (2,05 mL) in Dichlormethan (20 mL) gegeben. Die Reaktionsmischung wird über Nacht bei Raumtemperatur gerührt. Daraufhin wird sie mit konzentrierter Natriumhydrogencarbonatlösung versetzt, die wässrige Phase abgetrennt und mit Essigsäureethylester extrahiert. Die vereinigten organischen Phasen werden über Natriumsulfat getrocknet und eingeengt. Nach säulenchromatographischer Reinigung erhält man 2-{3-[2-(1-{[5-Methyl-3-(trifluormethyl)-1H-pyrazol-1-yl]acetyl}piperidin-4-yl)-1,3-thiazol-4-yl]-4,5-dihydro-1,2-oxazol-5-yl}benzaldehyd (2,00 g).

### Beispiele

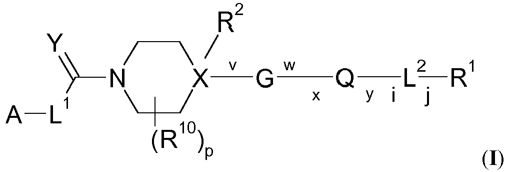

Die in Tablle 1 aufgelisteteten Strukturelemente G¹ und Q²⁴-3 sind wie folgt definiert:

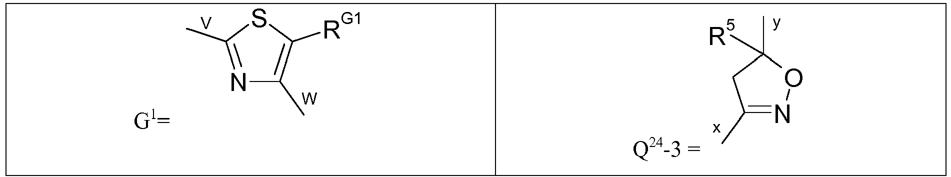

Für alle in Tabelle 1 aufgeführten Verbindungen, p=0 und L² = direkte Bindung, R² = H

**Tabelle 1:**

| **Bsp** | **A** | **L1** | **Y** | **X** | **G** | **R^{G1}** | **Q** | **R⁵** | **R¹** | **Log P** |
|---|---|---|---|---|---|---|---|---|---|---|
| 1 | 5-Methyl-3-(trifluormethyl)-1H-pyrazol-1-yl | CH₂ | O | C | G¹ | H | Q²⁴-3 | H | 3 -F ormylphenyl | 2,83[a] |
| 2 | 5-Chlor-2-methylphenyl | NH | O | C | G¹ | H | Q²⁴-3 | H | 3 -F ormylphenyl | 3,05[a] |
| 3 | 5-Chlor-2-methylphenyl | NH | O | C | G¹ | H | Q²⁴-3 | H | 2-Formylphenyl | 3,35[a] |
| 4 | 2,5-Bis(difluormethyl)phenyl | CH₂ | O | C | G¹ | H | Q²⁴-3 | H | 2-[(Methylsulfonyl)amino]phenyl | 2,81[a] |
| 5 | 5-Methyl-3-(trifluormethyl)-1H-pyrazol-1-yl | CH₂ | O | C | G¹ | H | Q²⁴-3 | H | 2-[(Methylsulfonyl)amino]phenyl | |
| 6 | 2,5-Bis(difluormethyl)phenyl | NH | O | C | G¹ | H | Q²⁴-3 | H | 2-[(Methylsulfonyl)amino]phenyl | 2,72[a] |
| 7 | 5-Methyl-3-(trifluormethyl)-1H-pyrazol-1-yl | CH₂ | O | C | G¹ | H | Q²⁴-3 | H | 2-Formylphenyl | 3,12[a] |
| 8* | 5-Methyl-3-(trifluormethyl)-1H-pyrazol-1-yl | CH₂ | O | C | G¹ | H | Q²⁴-3 | H | 2,6-Difluor-3-(prop-2-in-1-yloxy)phenyl | |
| 9* | 5-Methyl-3-(trifluormethyl)-1H-pyrazol-1-yl | CH₂ | O | C | G¹ | H | Q²⁴-3 | H | 3-Fluor-2-(prop-2-in-1-yloxy)phenyl | |
| 10* | 5-Methyl-3-(trifluormethyl)-1H-pyrazol-1-yl | CH₂ | O | C | G¹ | H | Q²⁴-3 | H | 3-(Prop-2-in-1-yloxy)phenyl | 3,49[a]; 3,3[b] |
| 11* | 5-Methyl-3-(trifluormethyl)-1H-pyrazol-1-yl | CH₂ | O | C | G¹ | H | Q²⁴-3 | H | 2-Chlor-6-(prop-2-in-1-yloxy)phenyl | 3,62[a]; 3,53[b] |
| 12* | 5-Methyl-3-(trifluormethyl)-1H-pyrazol-1-yl | CH₂ | O | C | G¹ | H | Q²⁴-3 | H | 2-Fluor-6-(prop-2-in-1-yloxy)phenyl | 3,35[a]; 3,27[b] |
| 13* | 5-Methyl-3-(trifluormethyl)-1H-pyrazol-1-yl | CH₂ | O | C | G¹ | H | Q²⁴-3 | H | 2-(Prop-2-in-1 -yloxy)phenyl | 3,47[a]; 3,37[b] |
| 14* | 3,5-Bis(Trifluormethyl)-1H-pyrazol-1-yl | CH₂ | O | C | G¹ | H | Q²⁴-3 | H | 2-[(Cyclopropylcar-bonyl)oxy]phenyl | 4,04[a] |
| 15* | 3-(Difluormethyl)-5-methyl-1H-pyrazol-1-yl | CH₂ | O | C | G¹ | H | Q²⁴-3 | H | 2-(But-2-in-1-yloxy)-4-methoxyphenyl | 3,28[a] |
| 16* | 3,5-Bis(Trifluormethyl)-1H-pyrazol-1-yl | CH₂ | O | C | G¹ | H | Q²⁴-3 | H | 2-(Cyclopropylmethoxy)phenyl | 4,59[a] |
| 17* | 5-Methyl-3-(trifluormethyl)-1H-pyrazol-1-yl | CH₂ | O | C | G¹ | H | Q²⁴-3 | H | 2 -(Cyclopropylmethoxy)phenyl | 4,03[a] |
| 18* | 5-(Difluormethyl)-3-methyl-1H-pyrazol-1-yl | CH₂ | O | C | G¹ | H | Q²⁴-3 | H | 2-(Allyloxy)phenyl | 3,42[a]; 3,33[b] |
| 19* | 5-Methyl-3-(trifluormethyl)-1H-pyrazol-1-yl | CH₂ | O | C | G¹ | H | Q²⁴-3 | H | 3 -(Allyloxy) -2,6 -difluorphenyl | |
| 20* | 3-(Difluormethyl)-5-methyl-1H-pyrazol-1-yl | CH₂ | O | C | G¹ | H | Q²⁴-3 | H | 4-Chlor-2-(prop-2-in-1-yloxy)phenyl | 3,37[a]; 3,36[b] |
| 21* | 3-(Difluormethyl)-5-methyl-1H-pyrazol-1-yl | CH₂ | O | C | G¹ | H | Q²⁴-3 | H | 2,4-Dichlor-6-(prop-2-in-1-yloxy)phenyl | 3,66[a]; 3,57[b] |
| 22* | 3 -(Difluormethyl)-5-methyl-1H-pyrazol-1-yl | CH₂ | O | C | G¹ | H | Q²⁴-3 | H | 2-(Allyloxy)-4-methylphenyl | 3,62[a]; 3,66[b]; |
| 23* | 3-(Difluormethyl)-5-methyl-1H-pyrazol-1-yl | CH₂ | O | C | G¹ | H | Q²⁴-3 | H | 4-Methyl-2-(prop-2-in-1-yloxy)phenyl | 3,23[a]; 3,23[b]; |
| 24* | 3-(Difluormethyl)-5-methyl-1H-pyrazol-1-yl | CH₂ | O | C | G¹ | H | Q²⁴-3 | H | 2-(Allyloxy)-4,6-dichlorphenyl | 3,95[a]; 3,97[b]; |
| 25* | 2,5-Dimethylphenyl | NH | O | C | G¹ | H | Q²⁴-3 | H | 2-(Allyloxy)-6-fluorphenyl | 3,64[a]; 3,62[b] |
| 26* | 5-Methyl-3-(trifluormethyl)-1H-pyrazol-1-yl | CH₂ | O | C | G¹ | H | Q²⁴-3 | H | 4-Methyl-2-(prop-2-in-1-yloxy)phenyl | 3,67[a]; 3,61[b] |
| 27* | 3-(Difluormethyl)-5-methyl-1H-pyrazol-1-yl | CH₂ | O | C | G¹ | H | Q²⁴-3 | H | 2-(Cyclopropylmethoxy)-4-methoxyphenyl | 3,5[a] |
| 28* | 3-(Difluormethyl)-5-methyl-1H-pyrazol-1-yl | CH₂ | O | C | G¹ | H | Q²⁴-3 | H | 2-(Cyclopropylmethoxy)-6-fluorphenyl | 3,44[a] |
| 29* | 5-(Difluormethyl)-3-methyl-1H-pyrazol-1-yl | CH₂ | O | C | G¹ | H | Q²⁴-3 | H | 2-(Prop-2-in-1-yloxy)phenyl | 3,03[a] |
| 30* | 5-Methyl-3-(trifluormethyl)-1H-pyrazol-1-yl | CH₂ | O | C | G¹ | H | Q²⁴-3 | H | 2-(Allyloxy)phenyl | 3,86[a]; 3,73[b] |
| 31* | 3-(Difluormethyl)-5-methyl-1H-pyrazol-1-yl | CH₂ | O | C | G¹ | H | Q²⁴-3 | H | 2-Fluor-6-(prop-2-in-1-yloxy)phenyl | 2,92[a] |
| 32^{*} | 3,5-Bis(Trifluormethyl)-1H-H-pyrazol-1-yl | CH₂ | O | C | G¹ | H | Q²⁴-3 | H | 2-(Prop-2-in-1-yloxy)phenyl | 3,91[a] |
| 33* | 3,5-Bis(Trifluormethyl)-1H-pyrazol-1-yl | CH₂ | O | C | G¹ | H | Q²⁴-3 | H | 2-(Allyloxy)phenyl | 4,38[a] |
| 34* | 5-Methyl-3-(trifluormethyl)-1H-pyrazol-1-yl | CH₂ | O | C | G¹ | H | Q²⁴-3 | H | 2-[(Methylsulfonyl)methyl]phenyl | 2,6[a] |
| 35* | 5-Methyl-3-(trifluormethyl)-1H-pyrazol-1-yl | CH₂ | O | C | G¹ | H | Q²⁴-3 | H | 2-[(Methylsulfinyl)methyl]phenyl | 2,34[a] |
| 36* | 5-Methyl-3-(trifluormethyl)-1H-pyrazol-1-yl | CH₂ | O | C | G¹ | H | Q²⁴-3 | H | 2-[(Ethylsulfinyl)methyl]phenyl | 2,53[a] |
| 37* | 5-(Difluormethyl)-3-methyl-1H-pyrazol-1-yl | CH₂ | O | C | G¹ | H | Q²⁴-3 | CH 3 | 2-Fluor-6-(prop-2-in-1-yloxy)phenyl | 3,11[a]; 3,12[b]; |
| 38* | 2,5-Dimethylphenyl | NH | O | C | G¹ | H | Q²⁴-3 | H | 2-Chlor-6-(prop-2-in-1-yloxy)phenyl | 3,68[a]; 3,57[b] |
| 39* | 5-Chlor-2-methylphenyl | NH | O | C | G¹ | H | Q²⁴-3 | H | 2-(Allyloxy)-6-chlorphenyl | 4,21[a]; 4,11[b] |
| 40* | 5-Methyl-3-(trifluormethyl)-1H-pyrazol-1-yl | CH₂ | O | C | G¹ | H | Q²⁴-3 | H | 3-[(Cyclopropylcar-bonyl)oxy]phenyl | 3,53[a]; 3,47[b] |
| 41* | 5-Methyl-3-(trifluormethyl)-H1-pyrazol-1-yl | CH₂ | O | C | G¹ | H | Q²⁴-3 | H | 3-(Allyloxy)phenyl | 3,62[a]; 3,68[b]; |
| 42* | 2,5-Dimethylphenyl | NH | O | C | G¹ | H | Q²⁴-3 | H | 2-(Allyloxy)-6-chlorphenyl | 3,98[a]; 3,91[b] |
| 43* | 5-Methyl-3-(trifluormethyl)-1H-pyrazol-1-yl | CH₂ | O | C | G¹ | H | Q²⁴-3 | H | 2-[(2,2,2-trifluorethoxy)methyl]phenyl | 3,77[a] |
| 44* | 5-Methyl-3-(trifluormethyl)-1H-pyrazol-1-yl | CH₂ | O | C | G¹ | H | Q²⁴-3 | H | 2-[(2-Methoxyethoxy)methyl]phenyl | 3,28[a] |
| 45* | 2,5-Dimethylphenyl | NH | O | C | G¹ | H | Q²⁴-3 | H | 2-(Prop-2-yn-1-yloxy)phenyl | 3,43[a]; 3,41[b] |
| 46* | 2,5-Dimethylphenyl | NH | O | C | G¹ | H | Q²⁴-3 | H | 2-(Allyloxy)phenyl | 3,91[a]; 3,81[b] |
| 47* | 5-Methyl-3-(trifluormethyl)-1H-pyrazol-1-yl | CH₂ | O | C | G¹ | H | Q²⁴-3 | H | 4-[(Cyclopropylcarbonyl)oxy]phenyl | 3,49[a]; 3,4[b] |
| 48* | 5-Methyl-3-(trifluormethyl)-1H-pyrazol-1-yl | CH₂ | O | C | G¹ | H | Q²⁴-3 | H | 2-Chlor-6-[(cyclopropylcarbonyl)oxy]phenyl | 3,73[a]; 3,64[b] |
| 49* | 5-Methyl-3-(trifluormethyl)-1H-pyrazol-1-yl | CH₂ | O | C | G¹ | H | Q²⁴-3 | H | 2-Fluor-6-(2-methoxyethoxy)phenyl | 3,28[a]; 3,17[b] |
| 50* | 5-Methyl-3-(trifluormethyl)-1H-pyrazol-1-yl | CH₂ | O | C | G¹ | H | Q²⁴-3 | H | 2-(Allyloxy)-6-chlorphenyl | 3,94[a]; 3,83[b] |
| 51* | 5-Methyl-3-(trifluormethyl)-1H-pyrazol-1-yl | CH₂ | O | C | G¹ | H | Q²⁴-3 | H | 2-(Allyloxy)-6-fluorphenyl | 3,7[a]; 3,61[b] |
| 52* | 5-Methyl-3-(trifluormethyl)-1H-pyrazol-1-yl | CH₂ | O | C | G¹ | H | Q²⁴-3 | H | 2 -(Allyloxy) -3 -fluorphenyl | 3,97[a]; 3,84[b] |
| 53 | 5-Methyl-3-(trifluormethyl)-1H-pyrazol-1-yl | CH₂ | O | C | G¹ | H | Q²⁴-3 | H | 2-Fluor-6-formylphenyl | 3,03[a]; 2,95[b] |
| 54* | 5-Methyl-3-(trifluormethyl)-H1-pyrazol-1-yl | CH₂ | O | C | G¹ | H | Q²⁴-3 | H | 2-[(Cyclopropylcarbonyl)oxy]-3-fluorphenyl | 3,62[a]; 3,5[b] |
| 55* | 5-Methyl-3-(trifluormethyl)-1H-pyrazol-1-yl | CH₂ | O | C | G¹ | H | Q²⁴-3 | H | 2-(2-Methoxyethoxy)phenyl | 3,37[a]; 3,25[b] |
| 56* | 5-Methyl-3-(trifluormethyl)-1H-pyrazol-1-yl | CH₂ | O | C | G¹ | H | Q²⁴-3 | H | 2-[(Cyclopropylcarbonyl)oxy]phenyl | 3,52[a]; 3,41[b] |

| | | | | | | | | | | |
|---|---|---|---|---|---|---|---|---|---|---|
| *:nicht erfindungsgemäße Verbindungen (Vergleichsbeispiele) | | | | | | | | | | |

**Die Messung der logP Werte** erfolgte gemäß EEC Directive 79/831 Annex V.A8 durch HPLC (High Performance Liquid Chromatography) an reversed-phase Säulen (C 18), mit nachfolgenden Methoden:
^{[a]}Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril
^{[b]}Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

### NMR-Daten ausgewählter Beispiele

NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensitätₙ)
**Bsp. 1, Lösungsmittel: DMSO, Spektrometer: 399.95 MHz**
   10.0344 (6.63); 8.0579 (0.79); 8.0400 (6.68); 7.9295 (3.74); 7.8988 (2.27); 7.8831 (2.00); 7.8801 (2.32); 7.7478 (1.88); 7.7285 (2.24); 7.6635 (2.24); 7.6446 (2.92); 7.6255 (1.19); 6.4922 (4.46); 5.8980 (1.40); 5.8784 (1.71); 5.8708 (1.52); 5.8515 (1.21); 5.3605 (0.36); 5.3405 (0.97); 5.2983 (2.90); 5.2496 (2.81); 5.2072 (0.87); 4.3847 (1.15); 4.3527 (1.13); 4.0571 (0.96); 4.0393 (2.31); 4.0216 (2.35); 4.0036 (1.55); 3.9837 (2.26); 3.9560 (2.66); 3.9405 (1.98); 3.9130 (1.45); 3.4474 (1.79); 3.4278 (2.00); 3.4041 (2.39); 3.3845 (3.03); 3.3262 (49.36); 3.3125 (313.38); 3.2424 (1.57); 2.8718 (0.91); 2.8449 (1.44); 2.8143 (0.81); 2.6743 (0.45); 2.6701 (0.49); 2.5053 (62.03); 2.5010 (65.38); 2.3326 (0.53); 2.3277 (0.56); 2.2097 (16.00); 2.1319 (1.48); 2.1068 (2.42); 2.0766 (1.41); 2.0049 (1.06); 2.0012 (1.21); 1.9971 (1.55); 1.9870 (8.57); 1.8437 (0.61); 1.8238 (1.08); 1.7936 (0.95); 1.7640 (0.44); 1.6093 (0.63); 1.5889 (1.03); 1.5799 (1.02); 1.5581 (0.98); 1.5287 (0.44); 1.1930 (2.83); 1.1854 (0.97); 1.1752 (4.77); 1.1574 (2.31); -0.0002 (1.06);
**Bsp. 2, Lösungsmittel: DMSO, Spektrometer: 399.95 MHz**
   10.0350 (7.37); 9.9023 (0.41); 8.6412 (0.39); 8.1288 (3.28); 8.0347 (7.77); 7.9318 (3.17); 7.9024 (1.25); 7.8992 (1.80); 7.8956 (1.04); 7.8836 (1.44); 7.8803 (2.04); 7.8768 (1.27); 7.7490 (1.35); 7.7328 (1.52); 7.7297 (1.99); 7.6636 (1.95); 7.6446 (2.84); 7.6256 (1.17); 7.3336 (3.23); 7.3281 (3.30); 7.1991 (2.18); 7.1786 (2.94); 7.0833 (2.27); 7.0777 (2.14); 7.0630 (1.58); 7.0574 (1.50); 5.8976 (1.13); 5.8781 (1.35); 5.8703 (1.29); 5.8507 (1.16); 4.1731 (1.81); 4.1396 (1.90); 4.0578 (1.32); 4.0400 (3.70); 4.0222 (3.72); 4.0044 (1.30); 3.9854 (1.31); 3.9578 (1.48); 3.9421 (1.68); 3.9147 (1.44); 3.4505 (1.74); 3.4309 (1.76); 3.4073 (1.67); 3.3878 (1.85); 3.3567 (1.71); 3.3193 (212.77); 3.0448 (1.07); 3.0392 (1.19); 3.0104 (2.19); 2.9815 (1.25); 2.5411 (0.60); 2.5108 (12.77); 2.5065 (22.65); 2.5021 (28.64); 2.4977 (19.62); 2.4934 (9.27); 2.1877 (0.40); 2.1557 (15.50); 2.0936 (1.51); 2.0679 (1.65); 1.9877 (16.00); 1.7197 (0.52); 1.7101 (0.66); 1.6880 (1.28); 1.6799 (1.42); 1.6579 (1.27); 1.6495 (1.19); 1.6282 (0.48); 1.6190 (0.39); 1.1935 (4.47); 1.1757 (8.72); 1.1579 (4.30); -0.0002 (0.43);
**Bsp. 3, Lösungsmittel: DMSO, Spektrometer: 399.95 MHz**
   10.1834 (6.76); 8.1195 (3.33); 8.0328 (1.90); 8.0289 (1.95); 8.0231 (0.70); 8.0126 (2.50); 8.0099 (2.53); 8.0020 (8.12); 7.7367 (0.79); 7.7335 (0.83); 7.7178 (2.01); 7.6990 (1.52); 7.6957 (1.42); 7.6241 (1.99); 7.6159 (2.62); 7.6074 (2.57); 7.5974 (1.90); 7.5894 (1.20); 7.3298 (3.23); 7.3243 (3.27); 7.1963 (2.20); 7.1758 (2.98); 7.0813 (2.24); 7.0758 (2.11); 7.0610 (1.60); 7.0554 (1.51); 6.4452 (1.19); 6.4290 (1.35); 6.4174 (1.33); 6.4011 (1.21); 4.1613 (1.78); 4.1278 (1.89); 4.0899 (1.41); 4.0620 (1.65); 4.0462 (1.79); 4.0401 (1.35); 4.0222 (1.56); 4.0185 (1.60); 4.0045 (0.46); 3.3155 (190.78); 3.2923 (3.19); 3.2755 (1.02); 3.2660 (0.58); 3.2268 (1.72); 3.2106 (1.68); 3.1831 (1.57); 3.1669 (1.55); 3.0251 (1.23); 2.9958 (2.25); 2.9668 (1.27); 2.5107 (11.92); 2.5065 (21.69); 2.5020 (27.90); 2.4977 (19.76); 2.4935 (9.77); 2.1503 (16.00); 2.0743 (1.51); 2.0485 (1.71); 1.9879 (4.94); 1.6994 (0.51); 1.6904 (0.61); 1.6686 (1.31); 1.6612 (1.39); 1.6386 (1.30); 1.6311 (1.21); 1.6090 (0.50); 1.5996 (0.40); 1.1935 (1.36); 1.1758 (2.65); 1.1580 (1.31); -0.0002 (2.28);
**Bsp. 4, Lösungsmittel: DMSO, Spektrometer: 399.95 MHz**
   9.2859 (0.81); 8.0295 (0.39); 8.0173 (6.58); 7.7187 (1.27); 7.6986 (1.91); 7.6011 (1.48); 7.5812 (1.08); 7.5052 (2.29); 7.4373 (1.17); 7.4208 (1.52); 7.4168 (1.40); 7.4057 (0.47); 7.3939 (0.45); 7.3784 (1.58); 7.3680 (2.66); 7.3618 (4.78); 7.3593 (4.89); 7.3491 (1.42); 7.3429 (1.39); 7.3367 (1.18); 7.3266 (0.51); 7.3204 (0.50); 7.2856 (1.04); 7.2187 (1.17); 7.1489 (2.16); 7.0796 (2.37); 7.0128 (1.09); 6.9403 (1.18); 6.1207 (0.97); 6.0995 (1.21); 6.0927 (1.20); 6.0723 (1.00); 5.7464 (4.98); 4.4407 (0.61); 4.4116 (0.63); 4.0778 (0.63); 4.0485 (0.87); 4.0392 (0.98); 4.0115 (2.08); 3.9904 (1.99); 3.9505 (1.30); 3.9228 (1.16); 3.9068 (1.34); 3.8789 (1.04); 3.3752 (1.57); 3.3642 (2.39); 3.3080 (1524.29); 3.2856 (11.89); 3.2635 (3.70); 3.2426 (2.20); 3.2286 (1.24); 3.2084 (0.77); 3.1559 (0.42); 3.1400 (0.52); 3.0603 (1.03); 3.0358 (16.00); 2.9393 (0.46); 2.8903 (1.36); 2.8331 (0.56); 2.7997 (0.92); 2.7683 (0.57); 2.7316 (1.19); 2.6739 (1.32); 2.6692 (1.64); 2.6647 (1.30); 2.5392 (2.28); 2.5221 (7.36); 2.5090 (93.05); 2.5046 (173.22); 2.5002 (226.03); 2.4958 (157.39); 2.4914 (75.79); 2.3316 (1.14); 2.3269 (1.48); 2.3224 (1.12); 2.0849 (1.22); 2.0691 (1.99); 1.9867 (1.36); 1.9079 (0.38); 1.7282 (0.53); 1.7038 (0.50); 1.5642 (0.54); 1.5398 (0.53); 1.2373 (0.48); 1.1925 (0.44); 1.1749 (0.77); 1.1571 (0.41); -0.0002 (4.82);
**Bsp. 5, Lösungsmittel: DMSO, Spektrometer: 399.95 MHz**
   9.2895 (1.04); 8.0347 (0.61); 8.0224 (6.52); 7.4399 (1.29); 7.4356 (0.88); 7.4228 (1.56); 7.4191 (1.32); 7.4070 (0.46); 7.4029 (0.38); 7.3981 (0.62); 7.3923 (0.44); 7.3782 (1.60); 7.3679 (3.08); 7.3624 (5.05); 7.3596 (4.74); 7.3499 (1.29); 7.3461 (0.94); 7.3426 (1.20); 7.3366 (0.93); 7.3268 (0.41); 7.3205 (0.35); 6.4907 (3.72); 6.1225 (1.00); 6.1019 (1.23); 6.0951 (1.18); 6.0744 (1.01); 5.3418 (0.63); 5.2993 (2.10); 5.2489 (2.11); 5.2062 (0.63); 4.3833 (0.64); 4.3517 (0.68); 4.0571 (0.33); 4.0393 (0.99); 4.0216 (1.04); 4.0036 (0.56); 3.9893 (0.65); 3.9556 (1.68); 3.9278 (1.16); 3.9117 (1.37); 3.8841 (1.04); 3.6914 (0.58); 3.4098 (0.54); 3.3999 (0.46); 3.3897 (0.68); 3.3809 (1.08); 3.3716 (0.76); 3.3639 (0.87); 3.3518 (0.89); 3.3415 (0.94); 3.3111 (259.73); 3.2902 (3.79); 3.2674 (2.30); 3.2469 (1.47); 3.2388 (0.80); 3.1410 (0.38); 3.0627 (1.24); 3.0367 (16.00); 2.8689 (0.47); 2.8397 (0.83); 2.8127 (0.49); 2.5227 (0.93); 2.5094 (15.18); 2.5051 (28.85); 2.5006 (38.17); 2.4962 (26.96); 2.4919 (13.31); 2.2395 (0.55); 2.2089 (13.78); 2.1942 (1.10); 2.1356 (0.66); 2.1070 (1.31); 2.0692 (0.96); 1.9868 (4.35); 1.8229 (0.53); 1.8181 (0.54); 1.7939 (0.54); 1.5861 (0.53); 1.5774 (0.55); 1.5554 (0.54); 1.5469 (0.49); 1.1928 (1.19); 1.1750 (2.37); 1.1572 (1.17); -0.0002 (3.61);
**Bsp. 6, Lösungsmittel: DMSO, Spektrometer: 399.95 MHz**
   9.2891 (0.72); 8.6090 (2.11); 8.0212 (5.44); 7.7010 (1.17); 7.6808 (1.36); 7.5331 (1.79); 7.4826 (1.13); 7.4629 (0.95); 7.4392 (0.99); 7.4219 (1.32); 7.3812 (0.58); 7.3765 (1.02); 7.3737 (0.84); 7.3657 (2.95); 7.3610 (4.08); 7.3486 (1.19); 7.3386 (0.95); 7.3329 (0.62); 7.3231 (0.41); 7.3165 (0.33); 7.2175 (1.04); 7.2125 (0.84); 7.0783 (2.26); 6.9389 (1.27); 6.1220 (0.82); 6.1014 (1.01); 6.0944 (0.95); 6.0738 (0.82); 5.7462 (2.07); 4.1756 (1.13); 4.1420 (1.16); 4.0571 (1.24); 4.0392 (3.64); 4.0215 (3.66); 4.0037 (1.25); 3.9569 (0.80); 3.9293 (0.95); 3.9132 (1.04); 3.8857 (0.86); 3.3658 (0.95); 3.3562 (1.26); 3.3115 (379.65); 3.2929 (3.24); 3.2881 (3.63); 3.2699 (1.15); 3.2492 (0.96); 3.0674 (0.78); 3.0335 (14.21); 3.0093 (0.89); 2.6696 (0.36); 2.5395 (0.51); 2.5227 (1.67); 2.5094 (21.20); 2.5050 (39.09); 2.5006 (50.66); 2.4961 (34.76); 2.4917 (16.36); 2.3273 (0.33); 2.2178 (0.36); 2.1093 (0.96); 2.0839 (1.04); 2.0692 (0.85); 1.9868 (16.00); 1.7223 (0.39); 1.7017 (0.78); 1.6925 (0.89); 1.6709 (0.81); 1.6622 (0.78); 1.6401 (0.32); 1.1928 (4.42); 1.1750 (8.83); 1.1572 (4.29); -0.0002 (3.01);
**Bsp. 7, Lösungsmittel: DMSO, Spektrometer: 399.95 MHz**
   10.1836 (3.67); 8.0340 (1.04); 8.0300 (1.06); 8.0234 (0.39); 8.0136 (1.65); 8.0089 (4.95); 7.7370 (0.43); 7.7337 (0.44); 7.7181 (1.11); 7.6992 (0.83); 7.6958 (0.77); 7.6247 (1.02); 7.6151 (1.42); 7.6082 (1.43); 7.5965 (0.99); 7.5902 (0.70); 6.4891 (2.20); 6.4455 (0.64); 6.4295 (0.73); 6.4178 (0.72); 6.4016 (0.65); 5.3357 (0.39); 5.2934 (1.36); 5.2448 (1.33); 5.2022 (0.40); 4.3745 (0.40); 4.3415 (0.42); 4.0904 (0.73); 4.0623 (0.98); 4.0582 (1.38); 4.0465 (1.15); 4.0403 (3.76); 4.0225 (3.92); 4.0047 (1.32); 3.9801 (0.39); 3.9464 (0.42); 3.3867 (0.33); 3.3675 (0.44); 3.3582 (0.71); 3.3486 (0.53); 3.3119 (67.25); 3.2901 (1.13); 3.2567 (0.61); 3.2245 (1.14); 3.2083 (0.92); 3.1808 (0.82); 3.1646 (0.81); 2.8299 (0.49); 2.5242 (0.45); 2.5109 (5.48); 2.5066 (9.98); 2.5022 (12.87); 2.4978 (8.97); 2.4934 (4.35); 2.2075 (8.36); 2.1937 (0.49); 2.1142 (0.38); 2.0859 (0.79); 2.0539 (0.45); 1.9879 (16.00); 1.8041 (0.33); 1.5593 (0.33); 1.1937 (4.44); 1.1759 (8.69); 1.1581 (4.32); -0.0002 (1.14);
**Bsp. 8*, Lösungsmittel: DMSO, Spektrometer: 399.95 MHz**
   8.0325 (7.66); 7.3371 (0.58); 7.3239 (0.66); 7.3135 (1.27); 7.3006 (1.28); 7.2903 (0.80); 7.2774 (0.69); 7.1557 (0.88); 7.1517 (0.92); 7.1310 (1.53); 7.1282 (1.53); 7.1075 (0.72); 7.1039 (0.75); 6.4985 (4.27); 6.0167 (1.08); 5.9957 (1.29); 5.9867 (1.24); 5.9655 (1.13); 5.7569 (5.74); 5.3556 (0.89); 5.3130 (2.67); 5.2626 (2.59); 5.2199 (0.87); 4.8983 (6.61); 4.8924 (6.85); 4.3934 (0.74); 4.3595 (0.79); 4.0554 (0.42); 4.0376 (1.27); 4.0198 (1.32); 4.0018 (0.93); 3.9614 (0.79); 3.9404 (0.88); 3.9094 (0.93); 3.8967 (1.11); 3.8666 (0.96); 3.6481 (1.57); 3.6423 (3.36); 3.6365 (1.60); 3.5741 (1.15); 3.5528 (1.15); 3.5305 (0.96); 3.5096 (0.94); 3.4245 (0.50); 3.4152 (0.35); 3.4055 (0.61); 3.3964 (1.06); 3.3870 (0.61); 3.3773 (0.38); 3.3676 (0.56); 3.3225 (46.65); 3.3203 (44.06); 3.2718 (1.05); 3.2434 (0.58); 2.8715 (0.52); 2.8411 (0.95); 2.8131 (0.55); 2.6702 (0.42); 2.5056 (48.88); 2.5012 (65.94); 2.4967 (48.92); 2.3322 (0.35); 2.3279 (0.48); 2.3234 (0.37); 2.2090 (16.00); 2.1483 (0.73); 2.1167 (1.47); 2.0843 (0.81); 1.9886 (5.44); 1.8305 (0.63); 1.8063 (0.59); 1.5951 (0.59); 1.5861 (0.66); 1.5648 (0.62); 1.5551 (0.59); 1.2350 (0.41); 1.1921 (1.52); 1.1744 (2.98); 1.1565 (1.49); -0.0002 (0.53);
**Bsp. 9*, Lösungsmittel: DMSO, Spektrometer: 601.6 MHz**
   8.7986 (0.66); 8.0310 (8.32); 7.2995 (0.69); 7.2946 (0.76); 7.2881 (0.78); 7.2832 (1.00); 7.2804 (0.82); 7.2719 (1.20); 7.2640 (0.95); 7.1948 (0.36); 7.1816 (1.61); 7.1749 (4.74); 7.1700 (3.35); 7.1620 (0.57); 6.5053 (4.59); 5.9817 (1.40); 5.9695 (1.56); 5.9631 (1.51); 5.9508 (1.40); 5.3490 (1.22); 5.3206 (2.49); 5.2629 (2.73); 5.2345 (1.36); 4.8465 (6.64); 4.8425 (6.72); 4.3803 (0.78); 4.3586 (0.79); 4.0454 (0.50); 4.0335 (1.51); 4.0217 (1.53); 4.0099 (0.52); 3.9788 (0.77); 3.9559 (0.83); 3.9467 (1.50); 3.9279 (1.43); 3.9180 (1.55); 3.8993 (1.28); 3.6568 (1.85); 3.6528 (3.82); 3.6489 (1.79); 3.4028 (0.55); 3.3968 (0.89); 3.3901 (0.81); 3.3837 (1.25); 3.3774 (2.09); 3.3501 (362.44); 3.3314 (3.04); 3.3265 (6.83); 3.3192 (2.05); 3.2812 (0.72); 3.2610 (1.14); 3.2424 (0.63); 3.2383 (0.57); 2.8500 (0.59); 2.8293 (1.02); 2.8118 (0.58); 2.6180 (0.80); 2.6150 (1.06); 2.6119 (0.84); 2.5955 (0.79); 2.5427 (0.99); 2.5242 (3.13); 2.5211 (3.99); 2.5180 (4.63); 2.5091 (55.45); 2.5062 (113.82); 2.5032 (153.80); 2.5002 (112.48); 2.4973 (52.77); 2.3904 (0.76); 2.3874 (1.01); 2.3844 (0.75); 2.2050 (16.00); 2.1921 (0.42); 2.1267 (1.03); 2.1005 (1.11); 2.0964 (1.11); 2.0782 (2.13); 1.9905 (6.69); 1.8367 (0.34); 1.8301 (0.39); 1.8160 (0.72); 1.8101 (0.76); 1.7955 (0.72); 1.7897 (0.64); 1.5690 (0.67); 1.5625 (0.69); 1.5483 (0.68); 1.5424 (0.63); 1.3968 (0.37); 1.2335 (0.74); 1.1860 (1.93); 1.1741 (4.07); 1.1623 (2.09); 1.0380 (0.32); 1.0270 (0.34); 0.0052 (1.52); -0.0002 (35.42); -0.0058 (1.08);
**Bsp. 10*, Lösungsmittel: CDCl3, Spektrometer: 601.6 MHz**
   7.6273 (0.91); 7.4326 (0.47); 7.3245 (1.12); 7.3113 (2.15); 7.2984 (1.22); 7.2614 (79.66); 7.0850 (0.46); 7.0266 (1.81); 7.0149 (3.61); 6.9414 (1.96); 6.9281 (1.90); 6.3350 (6.11); 5.7351 (0.78); 5.0298 (0.59); 5.0034 (1.60); 4.9737 (1.59); 4.9498 (0.67); 4.7006 (6.62); 4.6971 (6.67); 4.5817 (0.75); 4.5629 (0.74); 4.0571 (0.70); 4.0394 (0.71); 3.8349 (0.45); 3.8052 (0.32); 3.7893 (0.48); 3.6444 (0.47); 3.4187 (0.48); 3.3067 (1.20); 2.9123 (0.46); 2.8936 (0.76); 2.8745 (0.47); 2.5105 (2.38); 2.5071 (4.68); 2.5036 (2.42); 2.3239 (16.00); 2.2858 (0.44); 2.2145 (0.77); 2.1812 (0.75); 2.1629 (0.73); 1.7957 (0.89); 1.7805 (0.92); 1.5858 (3.62); 1.4787 (0.49); 1.4746 (0.46); 1.4321 (1.33); 1.4270 (0.89); 1.3860 (0.35); 1.3330 (0.38); 1.2928 (0.37); 1.2844 (0.63); 1.2542 (3.05); 0.8801 (0.63); 0.8683 (0.37); 0.0695 (1.41); 0.0051 (0.53); -0.0002 (16.59); -0.0057 (0.63);
**Bsp. 11*, Lösungsmittel: DMSO, Spektrometer: 399.95 MHz**
   7.9670 (8.64); 7.4133 (1.85); 7.3924 (3.34); 7.3720 (2.70); 7.1610 (3.06); 7.1564 (4.00); 7.1394 (2.50); 7.1365 (3.63); 6.5060 (4.36); 6.4950 (0.39); 6.2328 (1.37); 6.2083 (1.80); 6.2020 (1.66); 6.1773 (1.43); 5.7627 (11.00); 5.3613 (0.91); 5.3188 (2.57); 5.2700 (2.50); 5.2274 (0.88); 5.2032 (0.65); 4.8393 (0.34); 4.8331 (0.35); 4.7990 (2.74); 4.7910 (3.74); 4.7843 (2.83); 4.7503 (0.35); 4.7444 (0.36); 4.3942 (0.68); 4.3599 (0.74); 4.0366 (0.92); 4.0188 (0.97); 4.0009 (0.80); 3.9622 (0.72); 3.7667 (1.02); 3.7357 (1.26); 3.7247 (1.76); 3.6937 (1.42); 3.5797 (1.57); 3.5551 (1.60); 3.5376 (1.16); 3.5132 (1.16); 3.4596 (1.33); 3.4540 (3.13); 3.4482 (1.65); 3.4179 (0.79); 3.3720 (137.80); 3.3689 (145.30); 3.3659 (149.76); 3.3644 (142.87); 3.3627 (145.05); 3.3600 (158.31); 3.3560 (182.12); 3.3095 (0.97); 3.3026 (0.97); 3.2719 (1.19); 3.2437 (0.75); 3.0425 (1.70); 2.8727 (0.55); 2.8540 (1.66); 2.8448 (0.94); 2.8151 (0.55); 2.6774 (0.52); 2.6728 (0.75); 2.6682 (0.55); 2.5431 (0.37); 2.5262 (1.22); 2.5215 (1.91); 2.5128 (37.79); 2.5083 (83.02); 2.5037 (112.86); 2.4991 (80.19); 2.4945 (37.37); 2.3350 (0.54); 2.3304 (0.75); 2.3258 (0.59); 2.2082 (16.00); 2.1945 (0.98); 2.1899 (1.51); 2.1522 (0.65); 2.1249 (1.35); 2.0903 (0.76); 2.0774 (1.61); 1.9902 (4.31); 1.8271 (0.61); 1.8026 (0.55); 1.5943 (0.55); 1.5852 (0.62); 1.5638 (0.59); 1.5543 (0.58); 1.1920 (1.20); 1.1742 (2.43); 1.1564 (1.18); 0.0080 (0.86); -0.0002 (29.91); -0.0085 (1.06);
**Bsp. 12*, Lösungsmittel: DMSO, Spektrometer: 399.95 MHz**
   7.9807 (7.00); 7.9525 (2.03); 7.4372 (0.62); 7.4201 (0.81); 7.4161 (1.37); 7.3992 (1.36); 7.3953 (0.92); 7.3783 (0.71); 7.0116 (2.03); 6.9903 (1.83); 6.9152 (1.03); 6.8897 (1.23); 6.8681 (0.97); 6.5040 (3.54); 6.0725 (0.87); 6.0502 (1.06); 6.0421 (1.05); 6.0198 (0.93); 5.7619 (3.47); 5.3612 (0.79); 5.3185 (2.23); 5.2681 (2.18); 5.2254 (0.77); 5.2021 (0.39); 4.8549 (2.83); 4.8494 (4.94); 4.8440 (2.89); 4.3947 (0.60); 4.3602 (0.65); 3.9953 (0.56); 3.9617 (0.63); 3.8129 (0.58); 3.7816 (0.67); 3.7697 (0.84); 3.7394 (0.76); 3.5560 (1.45); 3.5502 (3.42); 3.5443 (1.54); 3.5246 (1.00); 3.5023 (1.02); 3.4818 (0.82); 3.4598 (0.76); 3.4187 (0.44); 3.4089 (0.41); 3.3998 (0.66); 3.3913 (1.02); 3.3798 (0.82); 3.3704 (1.02); 3.3457 (545.02); 3.3221 (4.34); 3.3102 (1.10); 3.3056 (1.13); 3.3023 (1.08); 3.2853 (0.48); 3.2711 (1.05); 3.2430 (0.56); 3.0425 (0.81); 2.8905 (16.00); 2.8710 (0.48); 2.8540 (0.86); 2.8400 (0.81); 2.8132 (0.46); 2.7305 (13.08); 2.6765 (0.51); 2.6719 (0.75); 2.6675 (0.56); 2.5251 (1.29); 2.5116 (41.13); 2.5073 (87.25); 2.5029 (117.49); 2.4984 (86.16); 2.4940 (42.72); 2.3339 (0.59); 2.3295 (0.81); 2.3250 (0.63); 2.2085 (13.04); 2.1948 (1.13); 2.1521 (0.59); 2.1218 (1.21); 2.0879 (0.69); 2.0769 (1.06); 1.8268 (0.53); 1.8037 (0.49); 1.5934 (0.48); 1.5834 (0.56); 1.5624 (0.51); 1.5537 (0.50); 1.2342 (0.40); -0.0002 (8.23); -0.0082 (0.39);
**Bsp. 13*, Lösungsmittel: DMSO, Spektrometer: 399.95 MHz**
   14.0244 (0.35); 8.7870 (0.54); 8.0352 (0.87); 8.0139 (7.56); 7.9916 (0.41); 7.4835 (0.44); 7.3543 (0.48); 7.3427 (0.95); 7.3228 (3.44); 7.3042 (2.77); 7.2237 (0.37); 7.1506 (1.98); 7.1313 (1.63); 7.0188 (1.03); 7.0022 (2.00); 6.9812 (0.94); 6.4963 (4.60); 6.4409 (0.35); 5.8865 (0.96); 5.8680 (1.10); 5.8584 (1.12); 5.8418 (0.94); 5.7554 (7.99); 5.3481 (0.81); 5.3051 (2.51); 5.2549 (2.40); 5.2126 (0.97); 4.9731 (0.39); 4.8801 (5.11); 4.8742 (5.27); 4.8150 (0.71); 4.3826 (0.80); 4.3501 (0.83); 4.0743 (0.38); 4.0556 (0.68); 4.0374 (1.57); 4.0200 (1.64); 4.0020 (0.67); 3.9809 (0.75); 3.9478 (1.00); 3.9202 (0.45); 3.8988 (1.16); 3.8703 (1.18); 3.8556 (1.32); 3.8277 (1.08); 3.6664 (0.48); 3.5857 (1.80); 3.5799 (3.73); 3.5739 (1.64); 3.4875 (0.36); 3.4448 (0.36); 3.3987 (0.82); 3.3776 (1.05); 3.3700 (1.49); 3.3581 (1.33); 3.3228 (817.78); 3.2695 (1.87); 3.2515 (1.78); 3.2263 (1.66); 3.2089 (1.19); 2.8589 (0.68); 2.8302 (1.05); 2.8000 (0.72); 2.6750 (1.40); 2.6704 (2.12); 2.6660 (1.35); 2.5407 (1.00); 2.5236 (3.21); 2.5096 (115.86); 2.5056 (225.44); 2.5012 (312.93); 2.4970 (220.11); 2.4928 (110.70); 2.3323 (1.37); 2.3278 (1.83); 2.3232 (1.36); 2.2160 (1.34); 2.2057 (16.00); 2.1935 (1.07); 2.1279 (0.79); 2.0993 (1.48); 2.0918 (1.41); 2.0733 (1.50); 2.0630 (0.99); 1.9884 (6.91); 1.9085 (0.84); 1.8351 (0.38); 1.8160 (0.73); 1.7837 (0.64); 1.6017 (0.35); 1.5716 (0.65); 1.5643 (0.65); 1.5516 (0.49); 1.5420 (0.71); 1.5104 (0.36); 1.2338 (0.91); 1.1922 (1.92); 1.1745 (3.72); 1.1567 (1.78); -0.0002 (5.87);
**Bsp. 14*, Lösungsmittel: DMSO, Spektrometer: 399.95 MHz**
   8.3198 (0.45); 8.0443 (16.00); 7.6053 (8.46); 7.4479 (2.82); 7.4440 (3.38); 7.4289 (3.83); 7.4249 (4.61); 7.4157 (1.89); 7.4115 (1.78); 7.3965 (3.75); 7.3921 (3.24); 7.3771 (3.07); 7.3727 (2.56); 7.3116 (2.76); 7.3090 (3.00); 7.2927 (4.30); 7.2903 (4.41); 7.2742 (1.69); 7.2714 (1.67); 7.1953 (0.33); 7.1890 (0.37); 7.1773 (4.76); 7.1747 (4.70); 7.1646 (0.64); 7.1572 (4.19); 7.1547 (3.90); 7.0597 (0.56); 7.0538 (0.44); 6.9641 (0.42); 6.9357 (0.32); 5.7610 (2.36); 5.7415 (2.76); 5.7327 (2.75); 5.7131 (2.43); 5.6385 (1.87); 5.5956 (4.12); 5.5268 (4.32); 5.4837 (1.93); 5.2693 (1.37); 5.1452 (0.97); 4.3548 (1.53); 4.3219 (1.65); 4.2712 (0.57); 4.2535 (0.57); 4.1720 (0.81); 4.1542 (0.81); 4.0376 (0.50); 4.0198 (0.52); 3.9738 (1.43); 3.9402 (1.61); 3.8975 (2.31); 3.8689 (2.63); 3.8543 (2.98); 3.8258 (2.50); 3.4591 (6.28); 3.4189 (2.28); 3.4100 (1.64); 3.3991 (1.84); 3.3896 (2.54); 3.3804 (1.64); 3.3705 (1.13); 3.3608 (1.37); 3.3524 (0.85); 3.3033 (1.33); 3.2908 (3.27); 3.2715 (5.00); 3.2551 (1.06); 3.2475 (3.40); 3.2283 (2.80); 2.8827 (1.10); 2.8529 (1.97); 2.8254 (1.12); 2.6763 (0.67); 2.6719 (0.93); 2.6674 (0.70); 2.5251 (2.77); 2.5116 (51.56); 2.5073 (103.29); 2.5028 (137.32); 2.4983 (103.49); 2.4940 (53.38); 2.3383 (0.39); 2.3341 (0.72); 2.3296 (0.98); 2.3250 (0.76); 2.1282 (1.37); 2.0962 (2.94); 2.0665 (1.68); 1.9896 (2.19); 1.8721 (0.77); 1.8557 (1.68); 1.8402 (2.83); 1.8344 (2.00); 1.8291 (2.06); 1.8213 (2.02); 1.8092 (1.64); 1.8011 (1.32); 1.7709 (1.16); 1.7423 (0.46); 1.5818 (0.52); 1.5725 (0.61); 1.5514 (1.21); 1.5424 (1.31); 1.5208 (1.42); 1.5096 (1.32); 1.5013 (0.75); 1.4976 (0.58); 1.4893 (1.15); 1.4832 (0.66); 1.4775 (0.66); 1.4697 (0.52); 1.3973 (6.91); 1.3362 (0.81); 1.2837 (0.58); 1.2659 (1.15); 1.2587 (0.35); 1.2486 (1.33); 1.2341 (0.81); 1.2155 (0.95); 1.1977 (1.80); 1.1929 (0.73); 1.1798 (1.22); 1.1751 (1.33); 1.1611 (0.85); 1.1573 (0.83); 1.1435 (0.42); 1.0719 (0.82); 1.0639 (1.00); 1.0562 (1.20); 1.0509 (2.43); 1.0454 (3.02); 1.0299 (5.26); 1.0181 (3.59); 1.0131 (5.32); 1.0065 (4.05); 1.0009 (2.53); 0.9945 (2.60); 0.9852 (2.73); 0.9784 (2.29); 0.9715 (1.96); 0.9603 (2.81); 0.9461 (1.55); 0.9397 (1.46); 0.9268 (0.72); 0.9190 (0.42); 0.8188 (0.79); 0.8116 (1.69); 0.8057 (0.80); 0.7989 (0.71); 0.7916 (1.70); 0.7858 (1.34); 0.7792 (1.57); 0.7723 (1.40); 0.7679 (1.82); 0.7604 (0.79); 0.7031 (0.49); 0.6996 (0.42); 0.6916 (0.93); 0.6717 (0.48); 0.1459 (0.40); 0.0080 (3.19); -0.0002 (88.57); -0.0083 (4.15); -0.1498 (0.41);
**Bsp. 15*, Lösungsmittel: DMSO, Spektrometer: 399.95 MHz**
   8.0022 (6.06); 7.2119 (2.20); 7.1906 (2.38); 7.0017 (1.16); 6.8645 (2.76); 6.7274 (1.32); 6.6819 (2.46); 6.6761 (2.69); 6.5676 (1.45); 6.5618 (1.32); 6.5464 (1.38); 6.5405 (1.27); 6.2950 (2.93); 5.8001 (0.91); 5.7812 (1.08); 5.7726 (1.04); 5.7536 (0.91); 5.2633 (0.66); 5.2211 (2.00); 5.1739 (1.99); 5.1317 (0.66); 4.7914 (3.28); 4.7856 (3.33); 4.3874 (0.60); 4.3555 (0.64); 4.0550 (0.61); 4.0372 (1.88); 4.0194 (2.07); 4.0016 (1.16); 3.9720 (0.63); 3.8129 (0.51); 3.8030 (0.90); 3.7752 (1.07); 3.7601 (1.52); 3.7498 (16.00); 3.7327 (1.19); 3.3994 (0.46); 3.3898 (0.41); 3.3804 (0.62); 3.3705 (1.06); 3.3443 (19.58); 3.2887 (1.45); 3.2698 (1.23); 3.2462 (1.44); 3.2269 (1.21); 2.8478 (0.42); 2.8168 (0.73); 2.7894 (0.42); 2.6759 (0.35); 2.6714 (0.50); 2.6667 (0.36); 2.5246 (1.35); 2.5111 (28.23); 2.5067 (57.28); 2.5022 (77.36); 2.4977 (59.44); 2.4933 (31.35); 2.3335 (0.45); 2.3290 (0.59); 2.3246 (0.45); 2.1850 (11.90); 2.1215 (0.63); 2.0914 (1.22); 2.0625 (0.73); 1.9894 (8.21); 1.9090 (6.30); 1.8492 (0.43); 1.8437 (0.49); 1.8203 (4.10); 1.8147 (8.12); 1.8090 (4.37); 1.7947 (0.89); 1.7870 (0.79); 1.7650 (0.65); 1.7334 (0.36); 1.7090 (0.40); 1.5650 (0.52); 1.5556 (0.57); 1.5342 (0.54); 1.5248 (0.51); 1.3972 (4.83); 1.1924 (2.19); 1.1746 (4.35); 1.1568 (2.16); -0.0002 (4.21);
**Bsp. 16*, Lösungsmittel: DMSO, Spektrometer: 399.95 MHz**
   8.0006 (2.31); 7.6034 (1.53); 7.2960 (0.90); 7.2769 (1.11); 7.2613 (0.46); 7.0222 (0.79); 7.0014 (0.64); 6.9487 (0.48); 6.9297 (0.81); 6.9115 (0.38); 5.8714 (0.37); 5.8527 (0.45); 5.8428 (0.41); 5.8247 (0.38); 5.6335 (0.34); 5.5902 (0.75); 5.5240 (0.77); 5.4812 (0.35); 4.0375 (0.46); 4.0198 (0.47); 3.9353 (0.34); 3.9227 (0.42); 3.9055 (0.37); 3.8967 (0.66); 3.8798 (0.68); 3.8683 (0.49); 3.8608 (0.68); 3.8417 (0.85); 3.8254 (0.59); 3.8173 (0.39); 3.7974 (0.51); 3.3817 (0.40); 3.3519 (0.84); 3.3277 (40.80); 3.3089 (0.72); 3.2908 (0.55); 3.2726 (0.39); 2.8544 (0.37); 2.5065 (34.94); 2.5021 (44.66); 2.4978 (32.56); 2.0951 (0.53); 1.9892 (1.91); 1.7901 (0.47); 1.3975 (16.00); 1.1926 (0.65); 1.1748 (1.22); 1.1635 (0.38); 1.1569 (0.69); 0.4971 (0.47); 0.4926 (0.48); 0.4770 (0.35); 0.4719 (0.39); 0.4383 (0.41); 0.4355 (0.42); 0.4156 (0.53); 0.3161 (0.39); 0.3042 (1.21); 0.2920 (1.16); 0.2798 (0.33); 0.0074 (0.34); -0.0002 (6.70);
**Bsp. 17*, Lösungsmittel: DMSO, Spektrometer: 399.95 MHz**
   7.9983 (5.82); 7.2945 (1.82); 7.2899 (1.35); 7.2782 (2.10); 7.2749 (2.41); 7.2605 (1.12); 7.2564 (0.65); 7.0215 (1.85); 7.0012 (1.44); 6.9475 (1.09); 6.9288 (1.88); 6.9100 (0.90); 6.4963 (3.32); 5.8720 (0.89); 5.8540 (1.03); 5.8439 (1.00); 5.8256 (0.91); 5.3524 (0.74); 5.3099 (2.04); 5.2561 (2.05); 5.2139 (0.74); 4.3815 (0.61); 4.3486 (0.65); 4.0554 (1.24); 4.0376 (3.73); 4.0198 (3.80); 4.0020 (1.38); 3.9778 (0.62); 3.9512 (0.66); 3.9230 (0.77); 3.9060 (0.81); 3.8974 (1.53); 3.8805 (1.59); 3.8675 (1.14); 3.8613 (1.62); 3.8436 (1.73); 3.8396 (1.60); 3.8247 (1.40); 3.8181 (0.87); 3.7966 (1.17); 3.4009 (0.48); 3.3909 (0.41); 3.3814 (0.57); 3.3722 (0.95); 3.3627 (0.70); 3.3497 (1.79); 3.3276 (79.63); 3.3070 (1.34); 3.2891 (1.39); 3.2626 (0.80); 3.2345 (0.46); 2.8634 (0.46); 2.8364 (0.77); 2.8052 (0.44); 2.6755 (0.35); 2.6711 (0.48); 2.6666 (0.37); 2.5413 (0.36); 2.5242 (1.94); 2.5109 (26.73); 2.5066 (52.99); 2.5020 (70.08); 2.4975 (51.35); 2.4931 (24.86); 2.3334 (0.36); 2.3287 (0.49); 2.3242 (0.35); 2.2060 (12.17); 2.1946 (1.38); 2.1218 (0.64); 2.0943 (1.15); 2.0628 (0.65); 1.9889 (16.00); 1.8140 (0.48); 1.8048 (0.53); 1.7901 (0.65); 1.7831 (0.48); 1.7735 (0.44); 1.5719 (0.45); 1.5629 (0.48); 1.5414 (0.44); 1.3975 (3.38); 1.2981 (0.32); 1.2585 (0.46); 1.1928 (4.73); 1.1823 (0.70); 1.1750 (9.35); 1.1655 (0.93); 1.1572 (4.79); 1.1484 (0.55); 0.5290 (0.41); 0.5213 (0.46); 0.5172 (0.45); 0.5093 (0.62); 0.5002 (1.09); 0.4953 (1.09); 0.4799 (0.79); 0.4747 (0.87); 0.4435 (0.90); 0.4399 (0.93); 0.4232 (1.00); 0.4197 (1.14); 0.4111 (0.52); 0.3980 (0.53); 0.3912 (0.48); 0.3410 (0.34); 0.3288 (0.49); 0.3178 (0.85); 0.3058 (2.78); 0.2987 (1.55); 0.2937 (2.71); 0.2884 (0.99); 0.2816 (0.72); 0.2705 (0.33); 0.0079 (0.51); -0.0002 (13.14); -0.0084 (0.45);
**Bsp. 18*, Lösungsmittel: DMSO, Spektrometer: 399.95 MHz**
   8.0100 (7.83); 7.3118 (1.40); 7.3083 (1.78); 7.3041 (1.62); 7.2943 (2.33); 7.2897 (2.40); 7.2853 (1.75); 7.2763 (1.44); 7.2721 (0.74); 7.2072 (1.33); 7.0725 (3.05); 7.0619 (2.31); 7.0512 (0.40); 7.0415 (1.80); 6.9747 (1.29); 6.9548 (2.22); 6.9378 (2.47); 6.3833 (3.14); 6.0804 (0.38); 6.0679 (0.83); 6.0545 (0.62); 6.0414 (0.95); 6.0372 (0.50); 6.0289 (0.50); 6.0246 (1.01); 6.0117 (0.70); 5.9981 (1.01); 5.9857 (0.48); 5.9181 (1.13); 5.9002 (1.30); 5.8903 (1.27); 5.8723 (1.15); 5.7570 (3.27); 5.4432 (0.61); 5.4389 (1.72); 5.4346 (1.75); 5.4303 (0.63); 5.3999 (0.53); 5.3956 (1.50); 5.3913 (1.52); 5.3870 (0.54); 5.2461 (1.93); 5.2421 (2.23); 5.2232 (0.68); 5.2196 (1.65); 5.2156 (1.65); 5.2119 (0.72); 5.1988 (2.50); 5.1594 (2.44); 5.1170 (0.61); 4.6326 (2.29); 4.6277 (2.51); 4.6207 (2.41); 4.6155 (2.15); 4.3629 (0.64); 4.3301 (0.66); 4.0380 (0.34); 4.0201 (0.36); 4.0020 (0.38); 3.9912 (0.60); 3.9560 (0.65); 3.8912 (1.23); 3.8632 (1.40); 3.8483 (1.59); 3.8205 (1.34); 3.3920 (0.47); 3.3822 (0.34); 3.3726 (0.57); 3.3633 (1.01); 3.3540 (0.59); 3.3439 (0.40); 3.3344 (0.69); 3.3217 (20.52); 3.2980 (1.00); 3.2864 (1.56); 3.2684 (1.90); 3.2434 (1.82); 3.2358 (0.93); 3.2257 (1.60); 3.2068 (0.46); 2.8344 (0.45); 2.8070 (0.80); 2.7767 (0.45); 2.5239 (0.38); 2.5104 (8.89); 2.5060 (18.38); 2.5015 (24.50); 2.4969 (17.47); 2.4925 (8.24); 2.1608 (16.00); 2.1069 (0.59); 2.0769 (1.25); 2.0475 (0.69); 1.9888 (1.44); 1.7741 (0.52); 1.7658 (0.56); 1.7431 (0.51); 1.7356 (0.48); 1.5590 (0.50); 1.5502 (0.54); 1.5283 (0.51); 1.5194 (0.49); 1.1923 (0.40); 1.1745 (0.79); 1.1567 (0.39); - 0.0002 (2.77);
**Bsp. 19*, Lösungsmittel: DMSO, Spektrometer: 399.95 MHz**
   8.0312 (8.75); 7.2693 (0.58); 7.2561 (0.65); 7.2458 (1.28); 7.2329 (1.27); 7.2224 (0.79); 7.2095 (0.70); 7.1053 (0.91); 7.1011 (0.94); 7.0806 (1.50); 7.0776 (1.46); 7.0573 (0.72); 7.0529 (0.72); 6.4986 (4.24); 6.0692 (0.43); 6.0559 (0.90); 6.0428 (0.88); 6.0295 (1.08); 6.0261 (0.60); 6.0129 (2.13); 5.9996 (1.15); 5.9927 (1.37); 5.9862 (1.78); 5.9732 (0.64); 5.9624 (1.15); 5.7570 (0.58); 5.4267 (0.69); 5.4226 (1.94); 5.4184 (1.98); 5.4144 (0.73); 5.3836 (0.61); 5.3795 (1.70); 5.3752 (1.75); 5.3711 (0.66); 5.3554 (0.86); 5.3128 (2.63); 5.2955 (0.92); 5.2922 (1.94); 5.2882 (1.83); 5.2621 (4.13); 5.2201 (0.87); 4.6447 (2.44); 4.6414 (4.03); 4.6380 (2.54); 4.6315 (2.56); 4.6281 (3.94); 4.6248 (2.36); 4.3925 (0.70); 4.3596 (0.74); 4.0555 (0.72); 4.0377 (2.18); 4.0199 (2.24); 4.0020 (1.21); 3.9612 (0.74); 3.9377 (0.84); 3.9073 (0.91); 3.8944 (1.09); 3.8642 (0.96); 3.5677 (3.27); 3.5469 (1.17); 3.5247 (0.95); 3.5037 (0.94); 3.4241 (0.52); 3.4148 (0.36); 3.4049 (0.61); 3.3955 (1.10); 3.3863 (0.63); 3.3760 (0.41); 3.3667 (0.58); 3.3573 (0.34); 3.3203 (81.65); 3.2966 (3.58); 3.2716 (0.97); 3.2433 (0.53); 3.0430 (0.53); 2.8705 (0.50); 2.8545 (0.64); 2.8441 (0.89); 2.8134 (0.52); 2.6748 (0.40); 2.6703 (0.56); 2.6657 (0.41); 2.5237 (1.15); 2.5102 (29.77); 2.5058 (61.50); 2.5012 (81.81); 2.4966 (58.12); 2.4922 (27.28); 2.3328 (0.40); 2.3279 (0.56); 2.3233 (0.41); 2.2095 (16.00); 2.1930 (0.82); 2.1485 (0.67); 2.1195 (1.36); 2.0862 (0.74); 1.9886 (9.64); 1.8292 (0.58); 1.8061 (0.53); 1.5944 (0.55); 1.5857 (0.61); 1.5651 (0.56); 1.5559 (0.55); 1.3975 (1.01); 1.1922 (2.65); 1.1744 (5.35); 1.1566 (2.61); -0.0002 (8.47);
**Bsp. 20*, Lösungsmittel: DMSO, Spektrometer: 601.6 MHz**
   8.0151 (9.12); 7.3155 (3.02); 7.3017 (3.39); 7.2357 (3.79); 7.2325 (3.96); 7.0807 (2.16); 7.0775 (2.10); 7.0671 (1.98); 7.0639 (1.94); 6.9503 (1.30); 6.8591 (3.31); 6.7680 (1.51); 6.2923 (3.51); 5.8374 (1.29); 5.8259 (1.41); 5.8191 (1.38); 5.8074 (1.30); 5.7541 (10.11); 5.2465 (1.00); 5.2183 (2.32); 5.1637 (2.28); 5.1354 (0.99); 4.9420 (6.32); 4.9380 (6.43); 4.3777 (0.68); 4.3555 (0.71); 4.0469 (0.32); 4.0350 (1.02); 4.0232 (1.03); 4.0113 (0.38); 3.9959 (0.64); 3.9729 (0.69); 3.8884 (1.32); 3.8699 (1.56); 3.8599 (1.67); 3.8414 (1.38); 3.6384 (1.82); 3.6345 (4.09); 3.6305 (1.81); 3.3844 (0.56); 3.3781 (0.34); 3.3717 (0.62); 3.3654 (1.17); 3.3590 (0.65); 3.3526 (0.40); 3.3463 (0.61); 3.3401 (0.36); 3.3188 (83.66); 3.2697 (0.51); 3.2545 (2.10); 3.2430 (1.81); 3.2303 (0.59); 3.2261 (1.89); 3.2145 (1.53); 2.8372 (0.48); 2.8168 (0.86); 2.7989 (0.49); 2.6162 (0.34); 2.6131 (0.51); 2.6102 (0.37); 2.5224 (0.87); 2.5194 (1.10); 2.5163 (1.02); 2.5074 (23.48); 2.5044 (53.22); 2.5014 (75.43); 2.4984 (54.67); 2.4954 (25.33); 2.3887 (0.34); 2.3856 (0.49); 2.3826 (0.36); 2.1853 (16.00); 2.1700 (0.43); 2.1154 (0.59); 2.0879 (0.88); 2.0601 (0.65); 1.9884 (4.41); 1.7835 (0.53); 1.7779 (0.58); 1.7633 (0.55); 1.7572 (0.54); 1.5559 (0.55); 1.5496 (0.60); 1.5358 (0.57); 1.5295 (0.54); 1.3359 (0.43); 1.2588 (0.34); 1.2497 (0.56); 1.1870 (1.23); 1.1751 (2.46); 1.1633 (1.19); 0.0052 (1.80); -0.0002 (65.36); -0.0058 (2.16);
**Bsp. 21*, Lösungsmittel: DMSO, Spektrometer: 399.95 MHz**
   8.3164 (0.44); 7.9600 (8.55); 7.3175 (3.41); 7.3128 (4.35); 7.2756 (4.09); 7.2709 (3.55); 7.0001 (1.52); 6.8629 (3.61); 6.7258 (1.69); 6.2950 (3.72); 6.1875 (1.31); 6.1633 (1.68); 6.1568 (1.59); 6.1324 (1.36); 5.7567 (11.60); 5.2624 (0.81); 5.2202 (2.75); 5.1767 (2.65); 5.1344 (0.80); 4.8983 (0.34); 4.8920 (0.36); 4.8581 (2.93); 4.8500 (3.96); 4.8434 (2.94); 4.8094 (0.35); 4.8036 (0.37); 4.3907 (0.77); 4.3577 (0.81); 4.0374 (0.66); 4.0195 (1.15); 4.0022 (0.65); 3.9783 (0.79); 3.7710 (1.02); 3.7399 (1.26); 3.7287 (1.71); 3.6977 (1.38); 3.5613 (1.55); 3.5370 (1.55); 3.5191 (1.13); 3.4949 (1.14); 3.4743 (1.69); 3.4685 (3.90); 3.4627 (1.72); 3.4108 (0.56); 3.4015 (0.45); 3.3911 (0.69); 3.3820 (1.22); 3.3725 (0.78); 3.3628 (0.64); 3.3258 (206.04); 3.3006 (0.81); 3.2947 (0.77); 3.2641 (1.06); 3.2355 (0.57); 2.8630 (0.55); 2.8332 (0.98); 2.8054 (0.54); 2.6753 (0.82); 2.6708 (1.17); 2.6663 (0.88); 2.5236 (6.05); 2.5106 (61.29); 2.5062 (121.93); 2.5017 (162.84); 2.4972 (123.45); 2.4930 (63.26); 2.3374 (0.43); 2.3329 (0.86); 2.3285 (1.16); 2.3240 (0.88); 2.1878 (16.00); 2.1455 (0.75); 2.1151 (1.49); 2.0825 (0.81); 1.9887 (2.69); 1.7973 (0.64); 1.7738 (0.59); 1.5841 (0.59); 1.5759 (0.65); 1.5544 (0.62); 1.5458 (0.58); 1.3354 (0.34); 1.2981 (0.40); 1.2586 (0.57); 1.2493 (0.45); 1.2332 (0.51); 1.1925 (0.74); 1.1747 (1.43); 1.1569 (0.71); 0.1460 (0.49); 0.0159 (0.96); 0.0079 (4.30); -0.0002 (106.55); -0.0083 (4.46); -0.1498 (0.50);
**Bsp. 22*, Lösungsmittel: DMSO, Spektrometer: 601.6 MHz**
   8.3142 (0.34); 7.9946 (7.84); 7.1678 (2.89); 7.1549 (3.09); 6.9506 (1.27); 6.8763 (3.42); 6.8594 (3.09); 6.7684 (3.08); 6.7568 (1.77); 6.2920 (3.60); 6.0513 (0.42); 6.0431 (0.92); 6.0340 (0.71); 6.0254 (0.99); 6.0226 (0.56); 6.0170 (0.53); 6.0143 (1.04); 6.0053 (0.74); 5.9966 (1.03); 5.9884 (0.49); 5.8617 (1.21); 5.8495 (1.34); 5.8433 (1.28); 5.8310 (1.21); 5.7546 (5.26); 5.4194 (0.67); 5.4167 (1.81); 5.4138 (1.84); 5.4110 (0.69); 5.3879 (1.61); 5.3850 (1.63); 5.3822 (0.61); 5.2475 (1.04); 5.2255 (1.92); 5.2228 (2.29); 5.2197 (2.66); 5.2079 (1.69); 5.2053 (1.70); 5.1630 (2.28); 5.1348 (1.00); 4.6045 (1.74); 4.5967 (3.07); 4.5888 (1.72); 4.5750 (0.34); 4.3765 (0.70); 4.3542 (0.74); 4.0348 (0.33); 4.0230 (0.35); 3.9958 (0.66); 3.9733 (0.72); 3.8315 (1.31); 3.8130 (1.54); 3.8032 (1.64); 3.7847 (1.38); 3.7377 (0.60); 3.3858 (0.59); 3.3795 (0.38); 3.3729 (0.67); 3.3666 (1.18); 3.3605 (0.69); 3.3536 (0.45); 3.3475 (0.71); 3.3222 (44.05); 3.3190 (116.77); 3.2722 (0.53); 3.2596 (1.82); 3.2475 (2.02); 3.2313 (1.88); 3.2191 (1.46); 2.8397 (0.50); 2.8194 (0.91); 2.8017 (0.50); 2.6159 (0.57); 2.6129 (0.82); 2.6098 (0.57); 2.5222 (1.54); 2.5191 (1.95); 2.5160 (1.99); 2.5071 (40.12); 2.5042 (87.09); 2.5011 (121.31); 2.4981 (87.79); 2.4951 (40.80); 2.3885 (0.58); 2.3853 (0.79); 2.3822 (0.55); 2.2873 (14.22); 2.1847 (16.00); 2.1701 (0.64); 2.1169 (0.68); 2.0884 (0.99); 2.0612 (0.69); 1.9883 (1.39); 1.7829 (0.56); 1.7775 (0.60); 1.7631 (0.57); 1.7575 (0.52); 1.5568 (0.55); 1.5504 (0.59); 1.5360 (0.55); 1.5300 (0.54); 1.2586 (0.33); 1.2494 (0.42); 1.2351 (0.37); 1.1867 (0.40); 1.1748 (0.77); 1.1630 (0.39); 1.1387 (0.54); 0.0840 (0.56); 0.0704 (0.41); 0.0052 (0.60); -0.0002 (18.89); - 0.0057 (0.67);
**Bsp. 23*, Lösungsmittel: DMSO, Spektrometer: 600.13 MHz**
   8.3129 (0.35); 7.9980 (8.12); 7.1849 (2.82); 7.1720 (3.04); 6.9642 (3.46); 6.9516 (1.32); 6.8602 (3.12); 6.8177 (1.81); 6.8048 (1.77); 6.7688 (1.43); 6.2935 (3.75); 5.8333 (1.17); 5.8212 (1.30); 5.8150 (1.26); 5.8027 (1.20); 5.7541 (9.04); 5.2463 (0.99); 5.2179 (2.31); 5.1632 (2.30); 5.1350 (1.00); 4.8484 (6.15); 4.8445 (6.19); 4.3766 (0.70); 4.3548 (0.73); 4.0350 (0.63); 4.0232 (0.64); 3.9961 (0.66); 3.9727 (0.71); 3.8409 (1.23); 3.8224 (1.43); 3.8124 (1.51); 3.7940 (1.30); 3.5695 (1.85); 3.5656 (3.91); 3.5617 (1.77); 3.3964 (0.35); 3.3923 (0.56); 3.3852 (0.84); 3.3793 (0.75); 3.3719 (1.08); 3.3667 (1.90); 3.3611 (1.61); 3.3534 (2.07); 3.3324 (1876.28); 3.3130 (1.81); 3.3099 (1.34); 3.3001 (0.44); 3.2903 (0.39); 3.2756 (0.58); 3.2711 (0.58); 3.2515 (1.06); 3.2438 (1.67); 3.2315 (2.00); 3.2152 (1.45); 3.2031 (1.41); 2.8391 (0.48); 2.8184 (0.91); 2.8009 (0.50); 2.6199 (0.75); 2.6170 (1.59); 2.6140 (2.18); 2.6110 (1.56); 2.6080 (0.72); 2.5412 (0.38); 2.5230 (3.90); 2.5199 (4.77); 2.5168 (4.61); 2.5079 (123.47); 2.5050 (264.64); 2.5019 (362.99); 2.4989 (259.62); 2.4960 (118.63); 2.3888 (1.53); 2.3858 (2.11); 2.3828 (1.48); 2.3007 (13.79); 2.1852 (16.00); 2.1711 (0.66); 2.1187 (0.61); 2.0909 (0.94); 2.0644 (0.69); 1.9885 (2.71); 1.7854 (0.55); 1.7792 (0.58); 1.7644 (0.57); 1.7587 (0.53); 1.5569 (0.56); 1.5507 (0.61); 1.5363 (0.57); 1.5305 (0.56); 1.3354 (0.58); 1.2981 (0.40); 1.2586 (0.54); 1.2494 (0.71); 1.1867 (0.75); 1.1748 (1.46); 1.1630 (0.72); 0.0969 (0.58); 0.0053 (4.51); -0.0001 (144.35); -0.0057 (4.44); -0.1002 (0.61);
**Bsp. 24*, Lösungsmittel: DMSO, Spektrometer: 399.95 MHz**
   7.9487 (9.14); 7.2585 (3.72); 7.2537 (4.34); 7.2009 (3.95); 7.1961 (3.40); 7.0001 (1.54); 6.8629 (3.75); 6.7258 (1.74); 6.2956 (3.55); 6.2149 (1.35); 6.1918 (1.71); 6.1839 (1.57); 6.1607 (1.42); 5.7985 (0.35); 5.7847 (0.74); 5.7717 (0.69); 5.7574 (14.11); 5.7415 (0.99); 5.7283 (0.83); 5.7152 (0.96); 5.7015 (0.48); 5.2670 (1.27); 5.2629 (2.06); 5.2589 (1.98); 5.2552 (0.77); 5.2269 (2.61); 5.2196 (1.95); 5.2156 (1.76); 5.2119 (0.71); 5.1747 (2.44); 5.1324 (0.84); 5.0184 (1.75); 5.0150 (1.72); 4.9921 (1.62); 4.9887 (1.61); 4.6251 (0.50); 4.6117 (0.52); 4.5934 (1.42); 4.5800 (1.39); 4.5623 (1.42); 4.5481 (1.38); 4.5305 (0.54); 4.5162 (0.50); 4.3903 (0.70); 4.3565 (0.74); 4.0375 (0.76); 4.0196 (1.20); 4.0021 (0.60); 3.9793 (0.72); 3.7660 (1.06); 3.7348 (1.32); 3.7238 (1.78); 3.6927 (1.43); 3.5552 (1.55); 3.5320 (1.56); 3.5130 (1.14); 3.4899 (1.13); 3.4182 (0.50); 3.4088 (0.34); 3.3989 (0.59); 3.3896 (1.07); 3.3802 (0.60); 3.3707 (0.38); 3.3608 (0.60); 3.3509 (0.37); 3.3264 (87.67); 3.2992 (0.64); 3.2682 (0.96); 3.2394 (0.52); 2.8672 (0.49); 2.8375 (0.88); 2.8104 (0.50); 2.6753 (0.41); 2.6707 (0.57); 2.6662 (0.41); 2.5242 (1.47); 2.5193 (2.32); 2.5108 (31.82); 2.5063 (63.80); 2.5017 (83.72); 2.4971 (60.62); 2.4926 (28.73); 2.3331 (0.43); 2.3285 (0.59); 2.3238 (0.42); 2.1880 (16.00); 2.1385 (0.65); 2.1047 (1.35); 2.0757 (0.75); 1.9889 (3.17); 1.8040 (0.56); 1.7956 (0.60); 1.7732 (0.58); 1.7656 (0.53); 1.5815 (0.56); 1.5726 (0.61); 1.5505 (0.58); 1.5420 (0.56); 1.3356 (0.49); 1.2584 (0.42); 1.2492 (0.63); 1.2338 (0.37); 1.1924 (0.89); 1.1746 (1.72); 1.1568 (0.85); 1.1382 (0.62); 0.0079 (1.93); -0.0002 (59.95); -0.0085 (1.93);
**Bsp. 25*, Lösungsmittel: DMSO, Spektrometer: 399.95 MHz**
   8.0279 (3.87); 7.9601 (9.47); 7.3975 (0.92); 7.3802 (1.19); 7.3765 (1.97); 7.3594 (2.01); 7.3557 (1.30); 7.3385 (1.02); 7.0489 (2.67); 7.0297 (3.22); 6.9979 (3.52); 6.9363 (2.80); 6.9151 (2.60); 6.8606 (2.26); 6.8413 (3.43); 6.8191 (1.39); 6.1066 (1.31); 6.0846 (1.60); 6.0760 (1.51); 6.0540 (1.37); 5.9311 (0.45); 5.9178 (0.95); 5.9047 (0.93); 5.8915 (1.12); 5.8882 (0.67); 5.8782 (0.65); 5.8747 (1.25); 5.8615 (1.08); 5.8482 (1.20); 5.8352 (0.57); 5.7593 (6.99); 5.3448 (0.79); 5.3410 (2.09); 5.3369 (2.15); 5.3330 (0.85); 5.2977 (1.81); 5.2936 (1.86); 5.2898 (0.74); 5.1204 (2.05); 5.1168 (2.04); 5.0941 (1.91); 5.0904 (1.92); 4.6232 (0.44); 4.6102 (0.41); 4.5907 (2.08); 4.5780 (3.26); 4.5707 (1.59); 4.5669 (1.99); 4.5480 (0.36); 4.5344 (0.33); 4.1790 (1.94); 4.1451 (2.08); 4.1246 (0.54); 4.1109 (1.03); 4.1069 (0.47); 4.0195 (0.32); 3.8018 (0.92); 3.7708 (1.07); 3.7589 (1.35); 3.7289 (1.23); 3.5378 (1.52); 3.5157 (1.59); 3.5033 (0.37); 3.4946 (1.62); 3.4734 (1.16); 3.3594 (0.67); 3.3299 (55.60); 3.3119 (0.73); 3.3021 (0.80); 3.2932 (0.41); 3.0164 (1.33); 2.9868 (2.49); 2.9582 (1.39); 2.6708 (0.39); 2.5240 (1.44); 2.5105 (23.49); 2.5062 (46.51); 2.5017 (60.90); 2.4971 (44.34); 2.4928 (21.71); 2.3331 (0.33); 2.3284 (0.42); 2.3240 (0.32); 2.2329 (16.00); 2.1107 (15.92); 2.0623 (1.95); 1.9891 (1.38); 1.7104 (0.55); 1.7005 (0.69); 1.6794 (1.45); 1.6707 (1.63); 1.6485 (1.53); 1.6407 (1.42); 1.6187 (0.63); 1.6094 (0.50); 1.2152 (0.41); 1.1974 (0.79); 1.1920 (0.42); 1.1795 (0.40); 1.1742 (0.75); 1.1564 (0.39); 1.0734 (0.43); 1.0561 (0.43); -0.0002 (1.81);
**Bsp. 26*, Lösungsmittel: DMSO, Spektrometer: 399.95 MHz**
   8.0044 (8.17); 7.1884 (2.93); 7.1691 (3.29); 6.9645 (3.82); 6.8199 (2.03); 6.8005 (1.84); 6.4986 (4.42); 5.8416 (1.17); 5.8235 (1.36); 5.8141 (1.34); 5.7957 (1.22); 5.7589 (1.32); 5.3524 (0.97); 5.3098 (2.81); 5.2586 (2.78); 5.2160 (0.98); 4.8515 (6.30); 4.8457 (6.52); 4.3839 (0.79); 4.3506 (0.83); 4.0554 (0.99); 4.0376 (3.04); 4.0198 (3.09); 4.0020 (1.13); 3.9834 (0.74); 3.9487 (0.81); 3.8553 (1.21); 3.8275 (1.40); 3.8124 (1.59); 3.7847 (1.32); 3.5799 (1.54); 3.5741 (3.36); 3.5683 (1.55); 3.3983 (0.53); 3.3890 (0.37); 3.3788 (0.65); 3.3696 (1.11); 3.3605 (0.68); 3.3502 (0.45); 3.3406 (0.78); 3.3264 (27.97); 3.3027 (1.83); 3.2875 (0.62); 3.2541 (2.36); 3.2356 (1.71); 3.2111 (1.44); 3.1929 (1.37); 2.8556 (0.60); 2.8258 (0.99); 2.7983 (0.57); 2.5240 (0.54); 2.5104 (14.51); 2.5061 (30.55); 2.5016 (42.55); 2.4972 (32.69); 2.4931 (16.62); 2.3331 (0.34); 2.3283 (0.36); 2.3001 (13.88); 2.2054 (16.00); 2.1934 (1.03); 2.1257 (0.70); 2.0918 (1.50); 2.0643 (0.82); 1.9891 (12.98); 1.8182 (0.63); 1.8094 (0.68); 1.7873 (0.62); 1.7788 (0.58); 1.5707 (0.60); 1.5614 (0.67); 1.5404 (0.63); 1.5310 (0.61); 1.3972 (0.35); 1.2582 (0.37); 1.1922 (3.50); 1.1744 (6.96); 1.1566 (3.45); 0.0080 (0.98); -0.0002 (31.85); -0.0084 (1.34);
**Bsp. 27*, Lösungsmittel: DMSO, Spektrometer: 600.13 MHz**
   7.9752 (5.68); 7.1878 (2.27); 7.1737 (2.38); 6.9533 (0.98); 6.8618 (2.31); 6.7705 (1.08); 6.5563 (2.43); 6.5524 (2.73); 6.5035 (1.49); 6.4997 (1.31); 6.4895 (1.43); 6.4856 (1.29); 6.2946 (2.97); 5.7876 (0.95); 5.7750 (1.07); 5.7689 (1.05); 5.7561 (0.99); 5.2512 (0.82); 5.2229 (1.82); 5.1646 (1.72); 5.1363 (0.80); 4.3757 (0.58); 4.3538 (0.62); 4.0472 (0.33); 4.0353 (0.98); 4.0234 (1.01); 4.0115 (0.42); 3.9985 (0.58); 3.9781 (0.66); 3.8896 (0.83); 3.8784 (0.88); 3.8726 (1.31); 3.8614 (1.28); 3.8251 (0.53); 3.8178 (1.20); 3.8059 (1.26); 3.8008 (0.88); 3.7889 (0.83); 3.7765 (0.42); 3.7713 (0.94); 3.7523 (1.13); 3.7426 (2.44); 3.7381 (16.00); 3.7241 (1.06); 3.3894 (0.46); 3.3831 (0.34); 3.3766 (0.55); 3.3703 (0.93); 3.3640 (0.57); 3.3550 (1.29); 3.3423 (1.27); 3.3254 (75.46); 3.3141 (1.28); 3.2785 (0.47); 3.2587 (0.83); 3.2393 (0.45); 2.8902 (2.01); 2.8476 (0.43); 2.8276 (0.75); 2.8094 (0.44); 2.7313 (1.69); 2.5226 (0.40); 2.5196 (0.50); 2.5164 (0.55); 2.5046 (24.76); 2.5016 (33.47); 2.4987 (24.64); 2.1971 (0.62); 2.1869 (11.90); 2.1769 (0.73); 2.1141 (0.58); 2.0870 (0.88); 2.0616 (0.61); 1.9888 (4.15); 1.7792 (0.49); 1.7767 (0.49); 1.7618 (0.46); 1.7564 (0.43); 1.5543 (0.49); 1.5344 (0.46); 1.3971 (1.43); 1.1867 (1.12); 1.1748 (2.20); 1.1629 (1.09); 1.1303 (0.48); 1.1266 (0.45); 1.1186 (0.74); 1.1106 (0.48); 1.1069 (0.50); 0.4787 (0.33); 0.4766 (0.35); 0.4704 (0.57); 0.4684 (0.53); 0.4629 (0.74); 0.4573 (0.64); 0.4508 (0.44); 0.4483 (0.49); 0.4445 (0.38); 0.3860 (0.37); 0.3824 (0.50); 0.3806 (0.50); 0.3766 (0.47); 0.3735 (0.54); 0.3672 (0.75); 0.3604 (0.58); 0.3524 (0.38); 0.3467 (0.37); 0.2926 (0.41); 0.2853 (0.67); 0.2771 (1.19); 0.2721 (1.47); 0.2688 (1.34); 0.2649 (1.55); 0.2602 (1.06); 0.2519 (0.61); 0.2448 (0.36); 0.0053 (0.32); -0.0001 (7.89);
**Bsp. 28*, Lösungsmittel: DMSO, Spektrometer: 399.95 MHz**
   7.9638 (7.36); 7.3700 (0.76); 7.3492 (1.67); 7.3319 (1.71); 7.3111 (0.82); 7.0001 (1.52); 6.8633 (4.36); 6.8462 (2.41); 6.8378 (1.50); 6.8131 (1.71); 6.7911 (1.22); 6.7259 (1.68); 6.2950 (3.99); 6.0878 (1.17); 6.0666 (1.43); 6.0570 (1.34); 6.0360 (1.20); 5.2707 (0.96); 5.2284 (2.60); 5.1725 (2.60); 5.1300 (0.96); 4.3861 (0.85); 4.3540 (0.91); 4.0552 (0.55); 4.0373 (1.66); 4.0195 (2.13); 4.0018 (1.14); 3.9758 (0.92); 3.9028 (1.21); 3.8858 (1.35); 3.8773 (1.72); 3.8606 (1.63); 3.7919 (0.92); 3.7547 (1.94); 3.7504 (1.87); 3.7364 (1.68); 3.7301 (1.42); 3.7187 (1.47); 3.7115 (1.31); 3.6042 (1.39); 3.5831 (1.39); 3.5620 (0.94); 3.5412 (1.00); 3.4136 (0.63); 3.4040 (0.49); 3.3938 (0.78); 3.3847 (1.22); 3.3754 (0.83); 3.3650 (0.65); 3.3556 (0.93); 3.3272 (76.50); 3.3019 (0.92); 3.2641 (1.16); 3.2353 (0.64); 2.8622 (0.60); 2.8337 (1.08); 2.8044 (0.62); 2.6754 (0.57); 2.6707 (0.75); 2.6665 (0.57); 2.5061 (82.21); 2.5017 (106.35); 2.4974 (80.71); 2.3331 (0.68); 2.3285 (0.80); 2.3241 (0.62); 2.1873 (16.00); 2.1616 (0.34); 2.1234 (0.87); 2.0923 (1.72); 2.0629 (0.98); 1.9890 (6.69); 1.8252 (0.35); 1.8156 (0.36); 1.7896 (0.95); 1.7628 (0.71); 1.7556 (0.67); 1.7450 (0.44); 1.5902 (0.34); 1.5619 (0.70); 1.5334 (0.66); 1.1924 (1.85); 1.1746 (3.58); 1.1567 (1.78); 1.0251 (0.36); 1.0100 (0.71); 0.9934 (0.97); 0.9767 (0.83); 0.9598 (0.43); 0.3804 (0.36); 0.3721 (0.52); 0.3545 (0.94); 0.3450 (1.17); 0.3397 (0.98); 0.3306 (0.59); 0.3261 (0.66); 0.3208 (0.53); 0.2060 (0.47); 0.1965 (0.59); 0.1787 (2.77); 0.1712 (2.46); 0.1613 (4.41); 0.1471 (1.81); 0.1306 (0.69); 0.1239 (0.52); -0.0002 (18.65);
**Bsp. 29*, Lösungsmittel: DMSO, Spektrometer: 399.95 MHz**
   8.0135 (7.84); 7.3476 (0.67); 7.3434 (0.92); 7.3236 (4.06); 7.3047 (3.74); 7.2072 (1.33); 7.1516 (2.20); 7.1317 (1.88); 7.0725 (2.95); 7.0206 (1.25); 7.0019 (2.21); 6.9833 (1.03); 6.9380 (1.52); 6.3834 (3.20); 5.8869 (1.10); 5.8691 (1.27); 5.8593 (1.24); 5.8414 (1.13); 5.7569 (7.59); 5.2407 (0.62); 5.1983 (2.49); 5.1598 (2.44); 5.1435 (0.40); 5.1174 (0.61); 4.8819 (6.04); 4.8760 (5.98); 4.3626 (0.64); 4.3296 (0.68); 4.0378 (0.65); 4.0200 (0.68); 4.0021 (0.48); 3.9904 (0.61); 3.9553 (0.67); 3.8993 (1.18); 3.8714 (1.36); 3.8563 (1.52); 3.8286 (1.27); 3.5890 (1.60); 3.5831 (3.49); 3.5772 (1.54); 3.3906 (3.30); 3.3733 (1.28); 3.3630 (1.26); 3.3537 (0.71); 3.3437 (0.44); 3.3342 (0.58); 3.2694 (1.85); 3.2515 (1.58); 3.2327 (0.95); 3.2266 (1.72); 3.2086 (1.62); 2.8326 (0.45); 2.8049 (0.82); 2.7747 (0.46); 2.5235 (0.47); 2.5103 (10.63); 2.5059 (21.60); 2.5013 (28.45); 2.4968 (20.20); 2.4923 (9.50); 2.1611 (16.00); 2.1079 (0.61); 2.0790 (1.27); 2.0498 (0.70); 1.9888 (2.84); 1.7766 (0.52); 1.7681 (0.55); 1.7455 (0.52); 1.7377 (0.48); 1.5600 (0.52); 1.5504 (0.56); 1.5287 (0.52); 1.5202 (0.50); 1.1922 (0.79); 1.1744 (1.56); 1.1566 (0.77); -0.0002 (2.27);
**Bsp. 30*, Lösungsmittel: DMSO, Spektrometer: 399.95 MHz**
   8.0106 (9.66); 7.3122 (1.38); 7.3070 (1.86); 7.3034 (1.80); 7.2943 (2.51); 7.2886 (2.37); 7.2843 (2.01); 7.2764 (1.66); 7.2723 (0.83); 7.0618 (2.49); 7.0508 (0.46); 7.0417 (1.89); 6.9745 (1.36); 6.9548 (2.36); 6.9377 (1.12); 6.4962 (4.47); 6.0804 (0.43); 6.0677 (0.97); 6.0547 (0.71); 6.0413 (1.09); 6.0370 (0.58); 6.0288 (0.60); 6.0245 (1.18); 6.0115 (0.82); 5.9980 (1.18); 5.9855 (0.55); 5.9178 (1.18); 5.8999 (1.37); 5.8903 (1.39); 5.8721 (1.23); 5.7554 (3.77); 5.4426 (0.62); 5.4384 (1.88); 5.4341 (1.98); 5.4300 (0.73); 5.3993 (0.58); 5.3952 (1.62); 5.3909 (1.68); 5.3871 (0.62); 5.3490 (0.87); 5.3064 (2.59); 5.2534 (2.66); 5.2461 (2.16); 5.2420 (2.00); 5.2195 (1.83); 5.2153 (2.05); 5.2117 (1.56); 4.6321 (2.51); 4.6276 (2.73); 4.6198 (2.65); 4.6153 (2.44); 4.3814 (0.71); 4.3488 (0.71); 4.0557 (0.89); 4.0380 (2.66); 4.0202 (2.74); 4.0024 (1.04); 3.9835 (0.69); 3.9517 (0.73); 3.8909 (1.34); 3.8629 (1.47); 3.8482 (1.72); 3.8203 (1.43); 3.3995 (0.56); 3.3894 (0.39); 3.3797 (0.67); 3.3702 (1.17); 3.3609 (0.78); 3.3268 (115.28); 3.3247 (244.25); 3.3015 (0.91); 3.2864 (2.05); 3.2682 (2.05); 3.2607 (1.05); 3.2435 (1.55); 3.2256 (1.84); 2.8595 (0.57); 2.8298 (0.90); 2.7999 (0.55); 2.6748 (0.44); 2.6703 (0.54); 2.6664 (0.41); 2.5237 (0.95); 2.5058 (64.08); 2.5015 (86.96); 2.4974 (61.07); 2.3329 (0.35); 2.3282 (0.54); 2.2059 (16.00); 2.1941 (0.82); 2.1281 (0.63); 2.0918 (1.32); 2.0736 (0.76); 2.0642 (0.77); 1.9885 (11.91); 1.8144 (0.55); 1.8073 (0.61); 1.7843 (0.55); 1.7789 (0.51); 1.5723 (0.55); 1.5625 (0.61); 1.5404 (0.55); 1.5319 (0.53); 1.2345 (0.56); 1.1924 (3.16); 1.1746 (6.25); 1.1568 (3.04); 0.0079 (0.33); -0.0002 (11.83); -0.0085 (0.37);
**Bsp. 31*, Lösungsmittel: DMSO, Spektrometer: 399.95 MHz**
   8.3169 (0.34); 7.9747 (8.93); 7.9522 (0.97); 7.4350 (0.80); 7.4180 (1.07); 7.4139 (1.72); 7.3971 (1.75); 7.3930 (1.11); 7.3762 (0.89); 7.0110 (2.57); 7.0008 (1.74); 6.9899 (2.33); 6.9122 (1.26); 6.8907 (1.42); 6.8870 (1.50); 6.8637 (4.65); 6.7266 (1.72); 6.2950 (3.82); 6.0724 (1.10); 6.0500 (1.32); 6.0419 (1.26); 6.0194 (1.13); 5.2657 (0.82); 5.2232 (2.61); 5.1774 (2.57); 5.1348 (0.81); 4.8525 (3.55); 4.8470 (6.21); 4.8414 (3.66); 4.3937 (0.74); 4.3602 (0.80); 4.0551 (0.76); 4.0373 (2.39); 4.0195 (2.94); 4.0018 (1.14); 3.9812 (0.77); 3.8099 (0.72); 3.7790 (0.84); 3.7698 (0.99); 3.7669 (1.02); 3.7372 (0.91); 3.5476 (1.82); 3.5417 (4.04); 3.5358 (1.85); 3.5253 (1.31); 3.5026 (1.25); 3.4824 (0.95); 3.4600 (0.95); 3.4161 (0.53); 3.4070 (0.38); 3.3965 (0.62); 3.3873 (1.11); 3.3783 (0.68); 3.3685 (0.47); 3.3585 (0.75); 3.3498 (0.69); 3.3283 (104.73); 3.2962 (0.61); 3.2647 (0.99); 3.2365 (0.56); 2.8903 (8.29); 2.8628 (0.53); 2.8318 (0.94); 2.8049 (0.53); 2.7307 (6.62); 2.6753 (0.50); 2.6709 (0.72); 2.6662 (0.52); 2.5406 (0.38); 2.5384 (0.40); 2.5241 (2.78); 2.5193 (4.33); 2.5107 (39.51); 2.5063 (79.45); 2.5018 (104.79); 2.4972 (76.32); 2.4928 (37.44); 2.3332 (0.55); 2.3286 (0.74); 2.3241 (0.57); 2.1883 (16.00); 2.1484 (0.75); 2.1130 (1.45); 2.0836 (0.83); 1.9888 (10.32); 1.8011 (0.62); 1.7797 (0.58); 1.5861 (0.59); 1.5773 (0.65); 1.5554 (0.62); 1.5467 (0.60); 1.3974 (2.06); 1.1925 (2.75); 1.1747 (5.55); 1.1569 (2.69); 0.0079 (0.70); -0.0002 (20.09); -0.0085 (0.85);
**Bsp. 32*, Lösungsmittel: DMSO, Spektrometer: 399.95 MHz**
   8.0176 (3.53); 7.6012 (1.82); 7.3438 (0.44); 7.3239 (1.89); 7.3050 (1.73); 7.1513 (1.01); 7.1313 (0.86); 7.0203 (0.58); 7.0018 (1.01); 6.9830 (0.46); 5.8875 (0.50); 5.8696 (0.59); 5.8598 (0.57); 5.8420 (0.53); 5.6352 (0.42); 5.5923 (0.93); 5.5228 (0.92); 5.4798 (0.41); 4.8815 (2.80); 4.8756 (2.78); 4.3512 (0.34); 4.3165 (0.35); 3.9344 (0.35); 3.9003 (0.52); 3.8724 (0.60); 3.8573 (0.67); 3.8295 (0.57); 3.5881 (0.71); 3.5822 (1.50); 3.5764 (0.68); 3.3790 (0.49); 3.3500 (0.35); 3.3277 (40.02); 3.2700 (1.02); 3.2523 (0.72); 3.2272 (0.67); 3.2094 (0.60); 2.8910 (2.35); 2.8506 (0.43); 2.7317 (1.82); 2.6713 (0.43); 2.5246 (1.46); 2.5198 (2.36); 2.5112 (24.23); 2.5068 (48.55); 2.5023 (63.46); 2.4977 (45.99); 2.4933 (22.44); 2.3289 (0.43); 2.0945 (0.63); 2.0639 (0.35); 1.3977 (16.00); - 0.0002 (1.78);
**Bsp. 33*, Lösungsmittel: DMSO, Spektrometer: 399.95 MHz**
   8.3173 (0.68); 8.0139 (16.00); 7.6009 (9.09); 7.3088 (4.66); 7.3047 (4.26); 7.2943 (5.83); 7.2901 (6.33); 7.2765 (3.38); 7.0614 (5.55); 7.0397 (4.29); 6.9733 (3.12); 6.9546 (5.42); 6.9358 (2.50); 6.0796 (0.81); 6.0671 (1.68); 6.0536 (1.40); 6.0405 (1.96); 6.0366 (1.26); 6.0280 (1.17); 6.0238 (2.13); 6.0105 (1.61); 5.9973 (2.06); 5.9849 (1.00); 5.9180 (2.65); 5.9001 (3.04); 5.8902 (3.03); 5.8722 (2.72); 5.6336 (2.15); 5.5908 (4.85); 5.5228 (4.85); 5.4798 (2.17); 5.4387 (3.78); 5.4344 (3.96); 5.4155 (0.38); 5.3954 (3.29); 5.3911 (3.43); 5.3759 (0.33); 5.2445 (3.75); 5.2407 (3.86); 5.2180 (3.49); 5.2142 (3.58); 4.6664 (0.38); 4.6541 (0.44); 4.6318 (5.77); 4.6274 (6.54); 4.6202 (6.39); 4.6156 (5.84); 4.5935 (0.50); 4.5812 (0.41); 4.3499 (1.84); 4.3167 (1.94); 3.9705 (1.72); 3.9352 (1.91); 3.8923 (2.66); 3.8643 (2.99); 3.8494 (3.43); 3.8215 (2.94); 3.4082 (1.20); 3.3989 (0.91); 3.3887 (1.47); 3.3795 (2.48); 3.3704 (1.57); 3.3599 (1.12); 3.3504 (1.69); 3.3269 (87.89); 3.3030 (7.09); 3.2871 (3.80); 3.2691 (5.63); 3.2441 (3.72); 3.2264 (3.21); 2.8786 (1.28); 2.8514 (2.32); 2.8211 (1.31); 2.6757 (0.86); 2.6713 (1.15); 2.6670 (0.87); 2.5414 (0.76); 2.5066 (123.19); 2.5022 (163.13); 2.4979 (124.07); 2.3335 (0.90); 2.3290 (1.20); 2.3248 (0.94); 2.1240 (1.68); 2.0916 (3.54); 2.0619 (1.97); 1.9891 (0.84); 1.8341 (0.60); 1.8240 (0.72); 1.8030 (1.46); 1.7945 (1.59); 1.7721 (1.44); 1.7641 (1.35); 1.7424 (0.61); 1.7329 (0.47); 1.5797 (0.59); 1.5699 (0.72); 1.5492 (1.44); 1.5399 (1.58); 1.5186 (1.47); 1.5092 (1.41); 1.4889 (0.62); 1.4785 (0.50); 1.3976 (8.31); 1.3512 (0.60); 1.3361 (1.33); 1.2587 (0.34); 1.2495 (0.99); 1.2251 (1.13); 1.1750 (0.46); 0.1460 (0.57); 0.0079 (5.94); - 0.0002 (128.33); -0.0083 (6.23); -0.1496 (0.60);
**Bsp. 34*, Lösungsmittel: CD3CN, Spektrometer: 399.95 MHz**
   7.7083 (6.06); 7.5436 (1.24); 7.5244 (2.00); 7.4725 (0.75); 7.4677 (0.83); 7.4555 (1.21); 7.4509 (1.48); 7.4360 (0.76); 7.4314 (1.48); 7.4274 (1.02); 7.4129 (2.75); 7.4081 (1.86); 7.4023 (2.05); 7.3989 (1.79); 7.3852 (1.24); 7.3825 (1.31); 7.3662 (0.41); 7.3628 (0.37); 6.3912 (3.38); 6.0855 (1.21); 6.0633 (1.45); 6.0581 (1.40); 6.0359 (1.26); 5.4478 (5.74); 5.1282 (0.63); 5.0861 (3.18); 5.0549 (3.16); 5.0129 (0.64); 4.5808 (1.36); 4.5455 (2.82); 4.4959 (0.73); 4.4847 (3.37); 4.4639 (0.73); 4.4494 (1.65); 3.9738 (1.43); 3.9462 (2.02); 3.9303 (1.85); 3.9137 (0.75); 3.9028 (1.91); 3.3696 (0.71); 3.3643 (1.81); 3.3508 (0.73); 3.3420 (2.72); 3.3317 (0.72); 3.3208 (1.99); 3.3123 (1.18); 3.2988 (1.58); 3.2815 (0.95); 3.2526 (0.53); 3.2460 (0.49); 2.9728 (0.42); 2.9425 (16.00); 2.8825 (0.46); 2.8759 (0.51); 2.8498 (0.86); 2.8452 (0.88); 2.8188 (0.51); 2.8123 (0.47); 2.2256 (16.31); 2.1817 (12.52); 2.1786 (12.84); 2.1203 (0.72); 1.9638 (0.83); 1.9575 (0.80); 1.9519 (5.16); 1.9457 (9.51); 1.9396 (13.00); 1.9333 (8.89); 1.9272 (4.50); 1.8732 (0.60); 1.8635 (0.62); 1.8421 (0.57); 1.8324 (0.55); 1.7053 (0.63); 1.6953 (0.64); 1.6740 (0.59); 1.6643 (0.56); -0.0002 (3.21);
**Bsp. 35*, Lösungsmittel: CD3CN, Spektrometer: 399.95 MHz**
   7.7009 (3.52); 7.6949 (4.85); 7.5121 (1.27); 7.5082 (1.60); 7.4918 (2.04); 7.4223 (0.34); 7.4175 (0.72); 7.4104 (0.57); 7.4006 (1.27); 7.3950 (1.13); 7.3861 (1.41); 7.3812 (1.44); 7.3763 (1.15); 7.3677 (2.28); 7.3640 (2.54); 7.3617 (1.79); 7.3585 (2.13); 7.3522 (2.26); 7.3487 (2.27); 7.3418 (1.45); 7.3377 (1.01); 7.3235 (0.47); 6.3904 (4.15); 6.0731 (0.94); 6.0512 (1.11); 6.0456 (1.19); 6.0237 (1.08); 6.0196 (0.87); 6.0142 (0.75); 5.9917 (0.66); 5.4467 (3.81); 5.1259 (0.73); 5.0839 (3.90); 5.0530 (3.81); 5.0317 (0.67); 5.0109 (0.74); 4.4964 (0.77); 4.4626 (0.80); 4.2284 (1.10); 4.1947 (1.44); 4.1428 (0.66); 4.1094 (3.41); 4.0957 (3.50); 4.0623 (0.68); 3.9871 (1.62); 3.9533 (1.80); 3.9362 (1.72); 3.9104 (1.93); 3.9087 (2.05); 3.8950 (1.38); 3.8929 (1.62); 3.8654 (1.49); 3.3778 (0.36); 3.3664 (1.37); 3.3556 (1.62); 3.3489 (0.89); 3.3437 (1.36); 3.3395 (1.57); 3.3337 (1.73); 3.3228 (1.26); 3.3121 (2.35); 3.3008 (1.28); 3.2903 (1.64); 3.2817 (1.12); 3.2527 (0.60); 3.2462 (0.56); 2.8823 (0.50); 2.8759 (0.55); 2.8456 (0.98); 2.8188 (0.56); 2.8124 (0.52); 2.5676 (16.00); 2.5203 (10.63); 2.2257 (18.95); 2.2113 (1.50); 2.2100 (1.47); 2.1508 (8.92); 2.1450 (7.66); 2.1193 (0.89); 2.1068 (0.44); 1.9635 (1.26); 1.9573 (1.46); 1.9516 (10.81); 1.9454 (20.09); 1.9392 (27.69); 1.9330 (18.99); 1.9268 (9.78); 1.9026 (0.38); 1.8925 (0.39); 1.8717 (0.71); 1.8624 (0.72); 1.8409 (0.66); 1.8313 (0.65); 1.7249 (0.36); 1.7047 (0.71); 1.6945 (0.74); 1.6736 (0.67); 1.6638 (0.65); -0.0002 (6.43);
**Bsp. 36*, Lösungsmittel: CD3CN, Spektrometer: 399.95 MHz**
   7.6978 (9.56); 7.6946 (9.81); 7.5034 (2.47); 7.4953 (1.98); 7.4871 (3.22); 7.4786 (2.25); 7.4120 (1.37); 7.4058 (1.29); 7.4041 (1.31); 7.3961 (2.49); 7.3894 (3.75); 7.3832 (1.79); 7.3780 (2.98); 7.3709 (3.94); 7.3642 (5.25); 7.3592 (7.40); 7.3502 (4.68); 7.3463 (4.48); 7.3432 (4.31); 7.3389 (3.50); 7.3255 (0.77); 7.3199 (0.36); 6.3904 (9.09); 6.0559 (2.04); 6.0490 (1.54); 6.0337 (2.46); 6.0279 (3.38); 6.0213 (1.74); 6.0064 (2.11); 5.9989 (1.53); 5.4461 (6.12); 5.1259 (1.72); 5.0837 (8.68); 5.0527 (8.53); 5.0108 (1.70); 4.4966 (1.86); 4.4630 (1.92); 4.1672 (2.48); 4.1336 (3.50); 4.0704 (16.00); 3.9820 (3.65); 3.9483 (4.37); 3.9368 (3.21); 3.9335 (3.07); 3.9090 (4.03); 3.9059 (3.83); 3.8938 (2.76); 3.8900 (2.90); 3.8659 (2.47); 3.8624 (2.68); 3.3719 (4.90); 3.3580 (1.20); 3.3497 (5.82); 3.3392 (3.46); 3.3289 (5.33); 3.3192 (2.12); 3.3068 (4.84); 3.2814 (2.60); 3.2524 (1.42); 3.2458 (1.31); 2.8770 (1.70); 2.8581 (2.13); 2.8442 (3.34); 2.8396 (3.03); 2.8252 (3.07); 2.8203 (2.47); 2.8172 (2.63); 2.8064 (3.31); 2.8032 (2.20); 2.7871 (1.40); 2.7842 (1.81); 2.7653 (0.57); 2.7494 (0.72); 2.7307 (2.29); 2.7121 (2.41); 2.6978 (1.98); 2.6937 (0.98); 2.6795 (2.88); 2.6611 (2.11); 2.6468 (1.21); 2.6427 (0.66); 2.6283 (1.11); 2.6097 (0.36); 2.2254 (41.56); 2.2087 (1.66); 2.2029 (1.75); 2.1611 (2.89); 2.1390 (18.09); 2.1203 (2.04); 1.9630 (1.87); 1.9511 (16.20); 1.9450 (29.63); 1.9388 (40.14); 1.9327 (27.43); 1.9265 (14.03); 1.9023 (0.82); 1.8919 (0.82); 1.8718 (1.68); 1.8617 (1.74); 1.8404 (1.58); 1.8309 (1.54); 1.8101 (0.64); 1.7999 (0.57); 1.7351 (0.72); 1.7243 (0.80); 1.7045 (1.69); 1.6944 (1.75); 1.6737 (1.59); 1.6637 (1.55); 1.6429 (0.64); 1.6325 (0.58); 1.3112 (7.82); 1.2978 (7.09); 1.2924 (15.87); 1.2792 (12.26); 1.2737 (8.02); 1.2605 (5.50); 0.0078 (0.36); -0.0002 (8.65); -0.0086 (0.33);
**Bsp. 37*, Lösungsmittel: DMSO, Spektrometer: 399.95 MHz**
   8.0640 (0.47); 7.9724 (5.06); 7.9511 (0.92); 7.3757 (0.61); 7.3547 (1.09); 7.3396 (1.18); 7.3184 (0.67); 7.0292 (1.63); 7.0201 (0.65); 7.0080 (1.47); 7.0004 (1.26); 6.8829 (1.40); 6.8633 (2.63); 6.8505 (0.94); 6.8289 (0.81); 6.7465 (0.55); 6.7262 (1.23); 6.3413 (1.14); 6.3002 (3.02); 5.3924 (0.49); 5.2614 (0.62); 5.2194 (1.88); 5.1719 (1.92); 5.1299 (0.63); 5.1053 (3.31); 4.9229 (3.83); 4.9172 (3.95); 4.3945 (0.59); 4.3594 (0.64); 4.2667 (0.94); 4.2556 (1.08); 4.2437 (1.05); 4.0560 (0.58); 4.0383 (1.66); 4.0204 (2.05); 4.0029 (0.99); 3.9777 (0.72); 3.7070 (1.48); 3.7025 (1.60); 3.6939 (2.25); 3.6801 (3.32); 3.6706 (2.57); 3.6521 (5.30); 3.5928 (2.49); 3.5858 (2.97); 3.3779 (5389.76); 2.8920 (7.63); 2.8662 (0.93); 2.8312 (1.23); 2.8031 (0.88); 2.7325 (6.18); 2.6785 (3.59); 2.6739 (4.83); 2.6694 (3.59); 2.5409 (3.16); 2.5272 (16.53); 2.5224 (24.16); 2.5137 (251.32); 2.5094 (502.31); 2.5049 (656.43); 2.5003 (472.57); 2.4960 (230.06); 2.3692 (1.31); 2.3360 (3.51); 2.3315 (4.73); 2.3269 (3.82); 2.2414 (5.87); 2.2071 (0.91); 2.1905 (12.67); 2.1730 (1.01); 2.1055 (1.36); 2.0721 (16.00); 1.9889 (6.86); 1.9101 (1.98); 1.7874 (0.66); 1.7687 (0.63); 1.6120 (1.12); 1.5918 (6.80); 1.5472 (0.60); 1.4104 (0.83); 1.2356 (0.72); 1.1928 (1.94); 1.1750 (3.75); 1.1572 (1.90); -0.0002 (2.02);
**Bsp. 38*, Lösungsmittel: DMSO, Spektrometer: 399.95 MHz**
   8.0193 (3.81); 7.9536 (9.31); 7.9287 (0.71); 7.4111 (1.90); 7.3903 (3.70); 7.3699 (2.77); 7.1618 (3.41); 7.1554 (4.01); 7.1404 (2.83); 7.1355 (3.52); 7.0500 (2.52); 7.0308 (3.07); 6.9990 (3.46); 6.8590 (1.95); 6.8395 (1.66); 6.2332 (1.49); 6.2085 (1.94); 6.2024 (1.88); 6.1777 (1.58); 5.7531 (5.37); 4.8383 (0.39); 4.8325 (0.42); 4.7981 (3.37); 4.7912 (5.19); 4.7841 (3.81); 4.7499 (0.40); 4.7439 (0.43); 4.1809 (1.91); 4.1469 (2.02); 4.0379 (0.72); 4.0201 (0.75); 3.7672 (1.17); 3.7363 (1.43); 3.7251 (2.07); 3.6943 (1.70); 3.5841 (1.95); 3.5593 (1.99); 3.5420 (1.43); 3.5173 (1.40); 3.5040 (0.41); 3.4453 (2.54); 3.4396 (4.79); 3.4338 (2.75); 3.3583 (517.73); 3.3548 (636.88); 3.3514 (650.80); 3.3476 (856.85); 3.1850 (0.58); 3.1755 (0.57); 3.1584 (0.59); 3.1523 (0.69); 3.0153 (1.49); 2.9857 (2.67); 2.9568 (1.50); 2.9081 (0.89); 2.6766 (1.32); 2.6721 (1.80); 2.6676 (1.36); 2.5393 (1.01); 2.5254 (5.57); 2.5206 (8.48); 2.5119 (93.76); 2.5075 (189.89); 2.5030 (252.19); 2.4984 (187.91); 2.4941 (94.93); 2.3343 (1.28); 2.3297 (1.75); 2.3252 (1.31); 2.2726 (0.33); 2.2336 (15.38); 2.1124 (16.00); 2.0727 (3.08); 1.9886 (3.35); 1.7027 (0.68); 1.6747 (1.49); 1.6473 (1.35); 1.6187 (0.51); 1.2360 (0.51); 1.1925 (0.89); 1.1747 (1.77); 1.1569 (0.86); -0.0002 (4.40);
**Bsp. 39*, Lösungsmittel: DMSO, Spektrometer: 399.95 MHz**
   8.1588 (3.15); 7.9410 (9.29); 7.3715 (1.93); 7.3509 (4.10); 7.3300 (4.13); 7.3227 (3.11); 7.2026 (2.15); 7.1817 (3.00); 7.1038 (2.36); 7.1016 (3.27); 7.0883 (2.72); 7.0832 (6.50); 7.0681 (1.94); 7.0626 (3.65); 6.2591 (1.36); 6.2356 (1.73); 6.2282 (1.60); 6.2046 (1.40); 5.8317 (0.37); 5.8181 (0.77); 5.8051 (0.72); 5.7917 (0.90); 5.7888 (0.58); 5.7782 (0.52); 5.7748 (0.98); 5.7617 (0.88); 5.7533 (7.80); 5.7488 (1.23); 5.7350 (0.48); 5.2725 (0.61); 5.2686 (1.79); 5.2645 (1.88); 5.2607 (0.73); 5.2291 (0.52); 5.2253 (1.53); 5.2212 (1.58); 5.2173 (0.60); 5.0153 (1.67); 5.0116 (1.68); 4.9889 (1.55); 4.9852 (1.59); 4.5819 (0.45); 4.5687 (0.48); 4.5502 (1.39); 4.5369 (1.39); 4.5231 (1.42); 4.5090 (1.36); 4.4913 (0.49); 4.4809 (0.36); 4.4771 (0.45); 4.1741 (1.49); 4.1408 (1.58); 4.0559 (0.39); 4.0381 (1.22); 4.0202 (1.23); 4.0025 (0.42); 3.7617 (1.06); 3.7306 (1.30); 3.7197 (1.87); 3.6887 (1.52); 3.5785 (1.71); 3.5549 (1.74); 3.5365 (1.20); 3.5130 (1.25); 3.3616 (206.47); 3.3582 (217.30); 3.3552 (181.25); 3.3479 (336.94); 3.0516 (1.16); 3.0221 (2.01); 2.9935 (1.15); 2.9264 (0.94); 2.6768 (0.56); 2.6722 (0.78); 2.6677 (0.55); 2.5257 (2.16); 2.5210 (3.11); 2.5123 (41.85); 2.5078 (87.47); 2.5032 (117.61); 2.4986 (86.11); 2.4940 (42.20); 2.3344 (0.60); 2.3299 (0.82); 2.3253 (0.61); 2.1553 (16.00); 2.1044 (1.29); 2.0730 (1.96); 1.9887 (5.62); 1.7272 (0.42); 1.7174 (0.54); 1.6955 (1.13); 1.6874 (1.27); 1.6656 (1.19); 1.6575 (1.10); 1.6361 (0.48); 1.6262 (0.40); 1.2350 (0.36); 1.1926 (1.55); 1.1748 (3.07); 1.1570 (1.50); -0.0002 (2.02);
**Bsp. 40*, Lösungsmittel: DMSO, Spektrometer: 399.95 MHz**
   8.0340 (9.26); 7.4541 (1.53); 7.4344 (3.45); 7.4147 (2.25); 7.2915 (1.97); 7.2722 (1.59); 7.1628 (1.73); 7.1577 (2.93); 7.1533 (2.10); 7.1184 (1.43); 7.1162 (1.62); 7.1127 (1.30); 7.1104 (1.26); 7.0983 (1.34); 7.0961 (1.38); 7.0927 (1.25); 7.0903 (1.15); 6.4980 (4.43); 5.7802 (1.13); 5.7604 (1.37); 5.7531 (1.33); 5.7332 (1.17); 5.3497 (0.88); 5.3073 (2.72); 5.2577 (2.67); 5.2151 (0.88); 5.1965 (0.85); 4.3847 (0.72); 4.3507 (0.77); 4.0557 (0.72); 4.0379 (2.19); 4.0201 (2.25); 4.0023 (0.93); 3.9883 (0.68); 3.9532 (0.75); 3.9232 (1.23); 3.8957 (1.41); 3.8800 (1.64); 3.8527 (1.33); 3.4089 (1.99); 3.3998 (0.57); 3.3890 (2.04); 3.3807 (1.25); 3.3707 (0.96); 3.3655 (1.83); 3.3456 (3.26); 3.3292 (241.15); 3.3261 (302.53); 3.2659 (1.26); 3.2371 (0.70); 3.0429 (1.96); 2.8659 (0.52); 2.8546 (1.70); 2.8352 (0.92); 2.8076 (0.53); 2.6753 (0.51); 2.6707 (0.71); 2.6662 (0.53); 2.5409 (0.35); 2.5241 (1.21); 2.5192 (1.89); 2.5106 (36.46); 2.5061 (78.71); 2.5015 (111.02); 2.4969 (83.92); 2.4925 (41.55); 2.3329 (0.55); 2.3283 (0.77); 2.3238 (0.58); 2.2070 (16.00); 2.1924 (1.81); 2.1325 (0.67); 2.1004 (1.39); 2.0735 (1.03); 1.9885 (9.76); 1.9183 (0.43); 1.9063 (0.85); 1.8989 (0.95); 1.8956 (0.73); 1.8870 (1.65); 1.8813 (0.75); 1.8750 (0.97); 1.8676 (1.00); 1.8557 (0.74); 1.8447 (0.33); 1.8248 (0.61); 1.8157 (0.64); 1.7930 (0.58); 1.7849 (0.55); 1.5824 (0.56); 1.5734 (0.62); 1.5523 (0.58); 1.5439 (0.58); 1.3976 (0.58); 1.2355 (0.40); 1.1923 (2.67); 1.1745 (5.40); 1.1567 (2.63); 1.0807 (0.55); 1.0670 (1.78); 1.0595 (3.41); 1.0558 (1.68); 1.0530 (1.79); 1.0470 (1.69); 1.0392 (3.51); 1.0334 (2.18); 1.0311 (2.14); 1.0249 (3.40); 1.0179 (3.09); 1.0139 (3.90); 1.0060 (1.80); 0.9919 (0.44); 0.0080 (1.09); -0.0002 (35.44); -0.0085 (1.38);
**Bsp. 41*, Lösungsmittel: DMSO, Spektrometer: 399.95 MHz**
   8.0259 (9.47); 7.3231 (1.61); 7.3190 (0.50); 7.3027 (3.67); 7.2952 (0.37); 7.2825 (2.20); 6.9632 (4.18); 6.9580 (3.28); 6.9534 (2.20); 6.9445 (1.95); 6.9304 (1.59); 6.9278 (1.49); 6.9245 (1.19); 6.9221 (1.11); 6.9097 (1.41); 6.9079 (1.39); 6.9036 (1.22); 6.4980 (4.40); 6.0822 (0.43); 6.0692 (0.96); 6.0560 (0.94); 6.0428 (1.11); 6.0390 (0.59); 6.0298 (0.60); 6.0259 (1.19); 6.0128 (1.05); 5.9996 (1.20); 5.9866 (0.57); 5.7179 (1.17); 5.6978 (1.40); 5.6911 (1.37); 5.6706 (1.21); 5.4168 (0.67); 5.4126 (1.94); 5.4082 (2.03); 5.4039 (0.75); 5.3737 (0.62); 5.3694 (1.73); 5.3650 (1.80); 5.3607 (0.77); 5.3504 (0.89); 5.3076 (2.74); 5.2632 (2.49); 5.2590 (4.21); 5.2407 (0.78); 5.2370 (1.73); 5.2328 (1.74); 5.2294 (0.81); 5.2153 (0.92); 5.1968 (0.86); 4.5810 (2.25); 4.5772 (4.03); 4.5735 (2.51); 4.5680 (2.46); 4.5642 (4.01); 4.5606 (2.36); 4.3853 (0.73); 4.3525 (0.77); 4.0377 (0.73); 4.0200 (0.78); 4.0021 (0.45); 3.9890 (0.69); 3.9532 (0.73); 3.8941 (1.27); 3.8670 (1.46); 3.8512 (1.69); 3.8239 (1.42); 3.4095 (0.54); 3.3929 (1.96); 3.3809 (1.27); 3.3725 (2.16); 3.3619 (0.42); 3.3500 (2.09); 3.3262 (194.10); 3.3243 (226.61); 3.3220 (303.73); 3.2983 (3.23); 3.2653 (1.26); 3.2367 (0.64); 3.2314 (0.57); 3.0428 (2.05); 2.8659 (0.53); 2.8544 (1.75); 2.8381 (0.92); 2.8086 (0.52); 2.6792 (0.39); 2.6748 (0.88); 2.6702 (1.21); 2.6657 (0.89); 2.6612 (0.41); 2.5406 (0.42); 2.5237 (2.26); 2.5188 (3.67); 2.5102 (61.32); 2.5057 (131.73); 2.5011 (185.44); 2.4966 (139.12); 2.4921 (67.67); 2.3326 (0.93); 2.3280 (1.24); 2.3234 (0.94); 2.3188 (0.50); 2.2068 (16.00); 2.1923 (1.83); 2.1313 (0.66); 2.1023 (1.37); 2.0736 (1.16); 1.9884 (3.47); 1.8230 (0.57); 1.8150 (0.63); 1.7916 (0.57); 1.7835 (0.54); 1.5831 (0.57); 1.5736 (0.63); 1.5521 (0.59); 1.5421 (0.57); 1.3976 (0.32); 1.2583 (0.39); 1.2493 (0.59); 1.2354 (0.71); 1.1922 (0.94); 1.1744 (1.90); 1.1566 (0.93); 0.0080 (2.08); -0.0002 (68.81); -0.0085 (2.53);
**Bsp. 42*, Lösungsmittel: DMSO, Spektrometer: 601.6 MHz**
   8.0198 (3.29); 7.9396 (10.59); 7.3640 (2.27); 7.3503 (4.58); 7.3366 (2.94); 7.1002 (2.64); 7.0987 (3.30); 7.0868 (2.70); 7.0852 (2.96); 7.0813 (2.45); 7.0671 (2.24); 7.0451 (2.24); 7.0324 (2.55); 6.9980 (2.70); 6.8566 (1.49); 6.8547 (1.50); 6.8439 (1.31); 6.8421 (1.31); 6.2494 (1.52); 6.2337 (1.82); 6.2287 (1.73); 6.2131 (1.56); 5.8156 (0.47); 5.8065 (0.92); 5.7978 (0.85); 5.7890 (1.03); 5.7869 (0.64); 5.7799 (0.59); 5.7777 (1.10); 5.7690 (0.94); 5.7602 (1.06); 5.7555 (1.13); 5.7512 (0.57); 5.2645 (0.67); 5.2618 (2.03); 5.2591 (2.05); 5.2564 (0.74); 5.2357 (0.62); 5.2331 (1.79); 5.2303 (1.85); 5.2277 (0.64); 5.0126 (0.70); 5.0104 (1.86); 5.0079 (1.83); 5.0057 (0.74); 4.9951 (0.68); 4.9929 (1.76); 4.9904 (1.76); 4.9882 (0.71); 4.5755 (0.35); 4.5732 (0.62); 4.5708 (0.38); 4.5667 (0.38); 4.5644 (0.63); 4.5621 (0.37); 4.5544 (0.78); 4.5521 (1.36); 4.5497 (0.85); 4.5456 (0.80); 4.5433 (1.33); 4.5410 (0.78); 4.5214 (0.78); 4.5192 (1.35); 4.5170 (0.83); 4.5120 (0.80); 4.5098 (1.34); 4.5077 (0.79); 4.5004 (0.38); 4.4981 (0.66); 4.4958 (0.40); 4.4910 (0.37); 4.4887 (0.63); 4.4865 (0.37); 4.1690 (1.42); 4.1468 (1.48); 4.0466 (1.14); 4.0348 (3.48); 4.0229 (3.47); 4.0111 (1.16); 3.7493 (1.28); 3.7286 (1.61); 3.7214 (1.98); 3.7008 (1.62); 3.5691 (1.84); 3.5535 (1.86); 3.5413 (1.45); 3.5257 (1.45); 3.3579 (0.37); 3.3517 (0.85); 3.3481 (0.61); 3.3454 (0.84); 3.3251 (591.42); 3.3079 (1.11); 3.3012 (4.55); 3.0160 (0.87); 3.0123 (1.06); 2.9927 (1.95); 2.9738 (1.08); 2.9701 (0.92); 2.6190 (0.40); 2.6160 (0.88); 2.6130 (1.25); 2.6099 (0.90); 2.6069 (0.41); 2.5405 (0.37); 2.5223 (3.07); 2.5192 (3.82); 2.5161 (3.46); 2.5074 (64.00); 2.5043 (141.18); 2.5012 (196.75); 2.4981 (141.80); 2.4951 (65.39); 2.3915 (0.37); 2.3885 (0.85); 2.3854 (1.21); 2.3823 (0.86); 2.3793 (0.38); 2.2327 (12.96); 2.1103 (13.21); 2.0890 (1.16); 2.0837 (1.23); 2.0734 (1.26); 2.0670 (1.33); 1.9884 (16.00); 1.6966 (0.39); 1.6905 (0.47); 1.6763 (1.08); 1.6704 (1.18); 1.6561 (1.12); 1.6504 (1.09); 1.6365 (0.44); 1.6299 (0.37); 1.3976 (0.40); 1.1862 (4.45); 1.1744 (9.23); 1.1626 (4.25); 0.0053 (0.64); -0.0002 (24.12); -0.0057 (0.68);
**Bsp. 43*, Lösungsmittel: CD3CN, Spektrometer: 399.95 MHz**
   7.7017 (16.00); 7.5291 (2.62); 7.5095 (4.66); 7.4599 (1.87); 7.4556 (2.02); 7.4419 (3.09); 7.4379 (4.17); 7.4302 (2.69); 7.4189 (4.14); 7.4148 (6.16); 7.3934 (4.06); 7.3899 (3.96); 7.3753 (3.19); 7.3569 (1.29); 7.3536 (1.16); 6.4212 (7.95); 6.0279 (2.81); 6.0066 (3.23); 6.0002 (3.19); 5.9789 (2.93); 5.1548 (1.55); 5.1129 (7.73); 5.0841 (7.77); 5.0630 (0.76); 5.0419 (1.47); 4.8696 (4.32); 4.8408 (6.69); 4.7412 (7.05); 4.7123 (4.54); 4.5215 (1.52); 4.4876 (1.67); 4.1147 (1.76); 4.0968 (5.36); 4.0790 (5.57); 4.0678 (1.89); 4.0612 (2.11); 4.0560 (2.15); 4.0534 (1.43); 4.0451 (5.27); 4.0334 (5.47); 4.0225 (5.45); 4.0107 (5.47); 4.0023 (1.59); 3.9998 (2.14); 3.9880 (2.51); 3.9798 (2.25); 3.9570 (0.81); 3.9327 (4.54); 3.9049 (3.73); 3.8896 (4.22); 3.8619 (4.00); 3.4070 (0.67); 3.3974 (1.35); 3.3878 (0.87); 3.3783 (1.53); 3.3687 (2.87); 3.3591 (1.67); 3.3494 (2.09); 3.3407 (2.44); 3.3303 (1.21); 3.3227 (4.99); 3.3136 (2.36); 3.3017 (4.58); 3.2797 (4.61); 3.2586 (3.78); 2.9157 (1.05); 2.9093 (1.15); 2.8782 (2.03); 2.8520 (1.17); 2.8454 (1.08); 2.4976 (0.34); 2.2545 (40.20); 2.2208 (828.11); 2.2177 (745.43); 2.1497 (2.66); 2.1436 (2.69); 2.1374 (2.27); 2.1312 (1.52); 2.1250 (0.99); 2.0912 (0.59); 2.0015 (24.43); 1.9943 (7.48); 1.9880 (7.97); 1.9823 (68.62); 1.9762 (130.30); 1.9700 (182.81); 1.9638 (126.48); 1.9576 (65.15); 1.9303 (1.19); 1.9197 (1.10); 1.8997 (1.68); 1.8900 (1.75); 1.8680 (1.55); 1.8584 (1.52); 1.8382 (0.87); 1.8278 (0.69); 1.8108 (0.60); 1.8046 (0.94); 1.7984 (1.25); 1.7922 (0.94); 1.7861 (0.56); 1.7660 (0.75); 1.7553 (0.82); 1.7355 (1.55); 1.7253 (1.60); 1.7042 (1.49); 1.6942 (1.45); 1.6744 (0.70); 1.6635 (0.66); 1.4653 (1.05); 1.3891 (0.33); 1.2986 (1.87); 1.2504 (6.62); 1.2326 (12.99); 1.2147 (6.49);
**Bsp. 44*, Lösungsmittel: CD3CN, Spektrometer: 399.95 MHz**
   7.6732 (4.41); 7.4745 (0.94); 7.4565 (1.13); 7.4523 (1.04); 7.3852 (0.53); 7.3812 (0.64); 7.3687 (1.08); 7.3640 (1.38); 7.3565 (1.61); 7.3526 (1.54); 7.3477 (1.18); 7.3262 (1.14); 7.3226 (1.11); 7.3066 (0.84); 6.3930 (2.25); 6.0032 (0.78); 5.9814 (0.93); 5.9756 (0.90); 5.9538 (0.80); 5.1262 (0.46); 5.0841 (2.18); 5.0558 (2.16); 5.0139 (0.41); 4.6676 (1.34); 4.6387 (2.06); 4.5397 (2.16); 4.5108 (1.47); 4.4929 (0.48); 4.4592 (0.51); 4.0683 (0.55); 4.0504 (0.54); 3.9493 (1.39); 3.9217 (1.31); 3.9062 (1.51); 3.8785 (1.11); 3.6257 (0.85); 3.6130 (1.73); 3.6025 (3.17); 3.5924 (1.34); 3.5316 (2.71); 3.5217 (2.79); 3.5177 (1.62); 3.5106 (1.21); 3.5076 (1.14); 3.3701 (0.38); 3.3510 (0.45); 3.3413 (0.82); 3.3317 (0.51); 3.3194 (0.56); 3.3126 (0.88); 3.2965 (16.00); 3.2833 (1.32); 3.2790 (1.78); 3.2675 (0.34); 3.2573 (1.34); 3.2482 (0.40); 3.2357 (1.04); 3.2141 (1.01); 2.8782 (0.34); 2.8476 (0.60); 2.8214 (0.34); 2.8155 (0.32); 2.4691 (0.44); 2.2258 (12.47); 2.2245 (12.61); 2.2048 (274.24); 2.1995 (217.79); 2.1214 (0.81); 2.1153 (0.79); 2.1091 (0.70); 2.1029 (0.44); 1.9729 (2.73); 1.9659 (2.55); 1.9540 (26.83); 1.9478 (51.05); 1.9417 (70.86); 1.9355 (49.44); 1.9293 (25.39); 1.9048 (0.46); 1.8942 (0.37); 1.8736 (0.51); 1.8642 (0.53); 1.8424 (0.46); 1.8331 (0.47); 1.7763 (0.36); 1.7701 (0.48); 1.7639 (0.36); 1.7087 (0.45); 1.6988 (0.48); 1.6775 (0.44); 1.6675 (0.44); 1.2699 (0.61); 1.2218 (0.68); 1.2040 (1.33); 1.1862 (0.67); -0.0002 (0.39);
**Bsp. 45*, Lösungsmittel: DMSO, Spektrometer: 399.95 MHz**
   8.0061 (9.48); 7.9826 (0.77); 7.6598 (0.38); 7.3440 (1.15); 7.3246 (5.02); 7.3058 (4.59); 7.1517 (2.63); 7.1318 (2.19); 7.0473 (2.59); 7.0283 (2.99); 7.0207 (2.26); 7.0000 (4.37); 6.9832 (1.47); 6.8561 (1.85); 6.8393 (1.79); 5.8864 (1.24); 5.8686 (1.50); 5.8588 (1.54); 5.8407 (1.30); 5.7552 (11.14); 4.8813 (6.84); 4.8756 (7.31); 4.1688 (1.86); 4.1351 (1.96); 4.0375 (0.44); 4.0198 (0.43); 3.9005 (1.28); 3.8727 (1.45); 3.8576 (1.70); 3.8298 (1.38); 3.5843 (1.93); 3.5785 (3.88); 3.5727 (1.85); 3.3549 (49.56); 3.3360 (230.21); 3.3284 (217.08); 3.3256 (287.42); 3.2867 (2.54); 3.2724 (2.74); 3.2544 (2.19); 3.2403 (0.76); 3.2293 (1.76); 3.2115 (1.70); 3.1978 (0.45); 3.1549 (0.35); 3.1491 (0.47); 2.9978 (1.36); 2.9676 (2.48); 2.9391 (1.36); 2.9078 (7.08); 2.6750 (0.71); 2.6706 (0.96); 2.6662 (0.71); 2.5408 (0.63); 2.5060 (108.78); 2.5015 (142.02); 2.4971 (107.62); 2.3326 (0.81); 2.3282 (1.05); 2.3239 (0.84); 2.2620 (0.49); 2.2319 (14.26); 2.1266 (0.83); 2.1063 (16.00); 2.0734 (2.22); 2.0429 (2.16); 1.9884 (1.83); 1.6864 (0.67); 1.6787 (0.78); 1.6565 (1.51); 1.6485 (1.61); 1.6261 (1.49); 1.6187 (1.39); 1.5966 (0.58); 1.5873 (0.47); 1.2581 (0.37); 1.2346 (1.05); 1.1923 (0.52); 1.1745 (0.99); 1.1567 (0.51); 0.0079 (1.58); -0.0002 (26.90); -0.0083 (1.28);
**Bsp. 46*, Lösungsmittel: DMSO, Spektrometer: 399.95 MHz**
   8.0114 (3.98); 8.0029 (11.23); 7.3168 (0.64); 7.3102 (2.52); 7.3057 (2.06); 7.2948 (3.13); 7.2911 (3.67); 7.2869 (2.38); 7.2770 (1.89); 7.2727 (1.12); 7.0621 (2.97); 7.0470 (3.07); 7.0424 (2.86); 7.0400 (2.62); 7.0278 (3.25); 6.9937 (3.44); 6.9741 (1.81); 6.9554 (2.92); 6.9385 (1.31); 6.9367 (1.32); 6.8563 (1.94); 6.8404 (1.62); 6.8372 (1.60); 6.0807 (0.50); 6.0682 (1.08); 6.0546 (0.80); 6.0417 (1.23); 6.0374 (0.69); 6.0293 (0.63); 6.0249 (1.34); 6.0114 (0.91); 5.9984 (1.32); 5.9860 (0.64); 5.9166 (1.45); 5.8987 (1.67); 5.8889 (1.66); 5.8708 (1.52); 5.4429 (0.74); 5.4386 (2.25); 5.4343 (2.36); 5.4300 (0.91); 5.3996 (0.64); 5.3953 (1.95); 5.3910 (2.07); 5.3867 (0.80); 5.2483 (0.83); 5.2445 (2.18); 5.2405 (2.21); 5.2369 (0.89); 5.2218 (0.75); 5.2180 (2.01); 5.2140 (2.08); 5.2104 (0.84); 4.6375 (1.28); 4.6331 (2.95); 4.6284 (3.27); 4.6249 (2.68); 4.6210 (3.20); 4.6161 (3.05); 4.1680 (1.90); 4.1345 (1.99); 4.0556 (1.04); 4.0378 (3.12); 4.0200 (3.15); 4.0022 (1.05); 3.8925 (1.59); 3.8645 (1.82); 3.8497 (2.08); 3.8218 (1.73); 3.3907 (0.37); 3.3748 (0.51); 3.3267 (710.58); 3.2892 (3.58); 3.2791 (1.56); 3.2711 (2.91); 3.2574 (0.53); 3.2464 (2.07); 3.2284 (2.00); 3.0001 (1.35); 2.9703 (2.48); 2.9418 (1.39); 2.6795 (0.44); 2.6752 (0.98); 2.6706 (1.42); 2.6660 (1.05); 2.6614 (0.52); 2.5408 (0.73); 2.5240 (2.28); 2.5193 (3.48); 2.5105 (71.10); 2.5060 (152.70); 2.5014 (209.79); 2.4969 (155.79); 2.4924 (77.49); 2.3373 (0.50); 2.3328 (1.07); 2.3282 (1.49); 2.3237 (1.12); 2.3191 (0.57); 2.2321 (15.82); 2.1056 (16.00); 2.0735 (3.20); 2.0413 (1.86); 1.9885 (13.82); 1.6890 (0.55); 1.6799 (0.66); 1.6586 (1.41); 1.6501 (1.59); 1.6278 (1.45); 1.6193 (1.40); 1.5978 (0.57); 1.5879 (0.48); 1.3976 (1.15); 1.2978 (0.39); 1.2583 (0.59); 1.2348 (1.38); 1.1923 (3.77); 1.1745 (7.51); 1.1567 (3.71); -0.0002 (6.96);
**Bsp. 47*, Lösungsmittel: DMSO, Spektrometer: 399.95 MHz**
   8.7845 (0.35); 8.0356 (1.18); 8.0319 (7.37); 8.0173 (0.49); 7.5869 (0.42); 7.4420 (0.59); 7.4347 (3.88); 7.4182 (1.40); 7.4133 (4.68); 7.3994 (0.34); 7.3507 (0.44); 7.1707 (0.68); 7.1642 (5.09); 7.1593 (1.82); 7.1477 (1.45); 7.1427 (4.51); 7.1361 (0.67); 6.4984 (4.27); 5.7729 (1.07); 5.7530 (1.32); 5.7460 (1.23); 5.7256 (1.00); 5.3488 (0.82); 5.3061 (2.51); 5.2560 (2.62); 5.2135 (0.86); 4.3851 (0.72); 4.3514 (0.78); 4.0558 (0.62); 4.0380 (1.84); 4.0202 (1.89); 4.0024 (0.80); 3.9879 (0.71); 3.9524 (0.75); 3.9109 (1.03); 3.8837 (1.26); 3.8679 (1.39); 3.8406 (1.14); 3.4604 (0.37); 3.4400 (0.63); 3.4132 (2.35); 3.3927 (3.49); 3.3525 (353.69); 3.3485 (459.15); 3.3461 (516.01); 3.3421 (735.82); 3.2661 (2.04); 3.2380 (1.15); 3.2318 (1.10); 3.1922 (0.43); 2.8668 (0.59); 2.8363 (0.98); 2.8095 (0.58); 2.6807 (0.41); 2.6763 (0.84); 2.6717 (1.18); 2.6672 (0.88); 2.6628 (0.45); 2.5419 (0.62); 2.5251 (2.21); 2.5204 (3.20); 2.5117 (56.02); 2.5072 (119.61); 2.5027 (163.85); 2.4981 (121.68); 2.4936 (60.83); 2.3385 (0.42); 2.3340 (0.83); 2.3294 (1.15); 2.3249 (0.87); 2.3202 (0.44); 2.2069 (16.00); 2.1340 (0.75); 2.1017 (1.45); 2.0861 (1.25); 2.0729 (1.73); 1.9886 (8.30); 1.9241 (0.45); 1.9123 (0.85); 1.9045 (0.99); 1.9016 (0.74); 1.8929 (1.48); 1.8887 (0.92); 1.8812 (0.96); 1.8732 (0.96); 1.8615 (0.56); 1.8252 (0.61); 1.8148 (0.66); 1.7924 (0.59); 1.7839 (0.57); 1.7603 (0.33); 1.6043 (0.33); 1.5838 (0.59); 1.5743 (0.65); 1.5525 (0.63); 1.5443 (0.61); 1.3976 (1.15); 1.2349 (0.77); 1.1925 (2.26); 1.1747 (4.49); 1.1569 (2.23); 1.1158 (0.39); 1.0957 (0.36); 1.0830 (0.77); 1.0704 (1.81); 1.0631 (3.57); 1.0553 (2.15); 1.0505 (1.85); 1.0431 (2.83); 1.0368 (1.75); 1.0246 (1.74); 1.0175 (3.08); 1.0106 (3.01); 1.0063 (3.12); 0.9984 (2.10); 0.9854 (0.50); 0.0080 (0.33); -0.0002 (11.67); -0.0084 (0.46);
**Bsp. 48*, Lösungsmittel: DMSO, Spektrometer: 399.95 MHz**
   8.0329 (7.22); 7.4658 (0.49); 7.4552 (8.31); 7.4454 (4.85); 7.4417 (4.76); 7.4214 (0.51); 7.2027 (0.35); 7.1921 (2.05); 7.1817 (1.81); 7.1789 (1.98); 7.1687 (1.79); 6.5052 (4.35); 6.1109 (1.31); 6.0867 (1.67); 6.0798 (1.60); 6.0555 (1.36); 5.7630 (9.67); 5.3651 (0.98); 5.3226 (2.46); 5.2634 (2.42); 5.2208 (0.98); 4.3907 (0.71); 4.3580 (0.75); 4.0544 (0.35); 4.0367 (1.07); 4.0189 (1.11); 4.0010 (0.69); 3.9922 (0.67); 3.9571 (0.72); 3.9209 (1.11); 3.8896 (1.34); 3.8776 (1.54); 3.8464 (1.23); 3.4331 (1.62); 3.4211 (0.73); 3.4089 (1.99); 3.4016 (1.17); 3.3900 (3.25); 3.3692 (105.50); 3.3662 (108.97); 3.3627 (125.98); 3.3573 (125.25); 3.3535 (153.94); 3.3133 (0.59); 3.2996 (0.80); 3.2691 (1.11); 3.2411 (0.58); 3.0424 (0.57); 2.8682 (0.53); 2.8540 (0.58); 2.8380 (0.90); 2.8106 (0.52); 2.6772 (0.44); 2.6726 (0.62); 2.6680 (0.44); 2.5261 (1.16); 2.5214 (1.66); 2.5126 (31.65); 2.5081 (69.43); 2.5035 (94.42); 2.4989 (67.38); 2.4944 (31.70); 2.3347 (0.46); 2.3303 (0.64); 2.3257 (0.48); 2.2067 (16.00); 2.1915 (0.73); 2.1337 (0.69); 2.1264 (0.52); 2.1033 (1.36); 2.0774 (1.16); 1.9902 (4.94); 1.8206 (0.56); 1.8121 (0.62); 1.7897 (0.56); 1.7816 (0.52); 1.6306 (0.37); 1.6183 (0.69); 1.6112 (0.91); 1.6056 (0.71); 1.5993 (1.52); 1.5876 (1.00); 1.5796 (1.26); 1.5678 (1.05); 1.5464 (0.60); 1.5370 (0.58); 1.1920 (1.38); 1.1742 (2.75); 1.1564 (1.35); 0.9614 (0.64); 0.9531 (0.62); 0.9425 (2.08); 0.9294 (1.10); 0.9219 (2.04); 0.9102 (0.90); 0.9030 (0.72); 0.8210 (0.39); 0.8110 (0.77); 0.8005 (0.70); 0.7932 (0.89); 0.7835 (0.72); 0.7686 (0.74); 0.7586 (0.60); 0.7470 (0.93); 0.7375 (0.80); 0.7268 (0.86); 0.7176 (0.60); 0.0080 (0.72); -0.0002 (27.69); -0.0085 (1.03);
**Bsp. 49*, Lösungsmittel: DMSO, Spektrometer: 399.95 MHz**
   7.9775 (7.21); 7.9529 (0.74); 7.3985 (0.59); 7.3812 (0.71); 7.3775 (1.25); 7.3604 (1.27); 7.3567 (0.82); 7.3395 (0.66); 6.9326 (1.66); 6.9116 (1.52); 6.8657 (0.89); 6.8415 (1.10); 6.8200 (0.86); 6.5035 (3.21); 6.0604 (0.81); 6.0378 (1.02); 6.0299 (0.96); 6.0070 (0.85); 5.3679 (0.77); 5.3253 (1.93); 5.2637 (1.85); 5.2211 (0.75); 4.3962 (0.51); 4.3632 (0.54); 4.1114 (1.81); 4.1006 (2.89); 4.0887 (1.95); 4.0548 (0.57); 4.0370 (1.71); 4.0192 (1.75); 4.0013 (0.96); 3.9626 (0.51); 3.7633 (0.56); 3.7322 (0.67); 3.7208 (0.99); 3.6903 (0.88); 3.5957 (1.03); 3.5730 (1.06); 3.5536 (0.67); 3.5309 (0.66); 3.4820 (1.31); 3.4783 (1.25); 3.4727 (2.14); 3.4652 (2.14); 3.4542 (1.04); 3.4264 (0.43); 3.4069 (0.40); 3.3973 (0.75); 3.3870 (0.38); 3.3688 (0.48); 3.3607 (0.74); 3.3419 (218.73); 3.3183 (1.43); 3.3079 (0.72); 3.3033 (0.71); 3.2721 (0.80); 3.2654 (0.64); 3.2445 (0.46); 3.0407 (0.36); 3.0169 (16.00); 2.8906 (6.62); 2.8700 (0.39); 2.8386 (0.74); 2.8125 (0.39); 2.7310 (5.14); 2.6763 (0.36); 2.6718 (0.52); 2.6673 (0.37); 2.5252 (0.78); 2.5206 (1.20); 2.5118 (26.94); 2.5073 (58.44); 2.5028 (78.90); 2.4982 (55.83); 2.4937 (26.07); 2.3342 (0.39); 2.3295 (0.54); 2.3249 (0.40); 2.2087 (12.09); 2.1393 (0.48); 2.1331 (0.49); 2.1257 (0.45); 2.1104 (0.99); 2.0768 (0.66); 1.9899 (7.95); 1.9076 (0.84); 1.8267 (0.44); 1.8211 (0.47); 1.7982 (0.43); 1.7900 (0.40); 1.5843 (0.41); 1.5753 (0.46); 1.5538 (0.44); 1.5450 (0.43); 1.1920 (2.10); 1.1742 (4.23); 1.1564 (2.04); 0.0080 (0.40); -0.0002 (14.85); -0.0085 (0.51);
**Bsp. 50*, Lösungsmittel: DMSO, Spektrometer: 399.95 MHz**
   7.9530 (8.91); 7.3744 (1.93); 7.3538 (4.10); 7.3332 (2.69); 7.1062 (2.47); 7.1041 (3.28); 7.0859 (4.76); 7.0839 (4.63); 7.0648 (2.13); 6.5062 (4.45); 6.2598 (1.34); 6.2363 (1.75); 6.2289 (1.61); 6.2053 (1.41); 5.8253 (0.33); 5.8117 (0.67); 5.7985 (0.66); 5.7852 (0.82); 5.7684 (0.90); 5.7616 (0.80); 5.7552 (0.78); 5.7420 (0.86); 5.7285 (0.44); 5.3676 (1.03); 5.3250 (2.60); 5.2642 (3.94); 5.2243 (2.34); 5.2207 (2.27); 5.0140 (1.59); 5.0105 (1.62); 4.9877 (1.50); 4.9843 (1.52); 4.5816 (0.47); 4.5687 (0.49); 4.5500 (1.39); 4.5367 (1.37); 4.5208 (1.37); 4.5066 (1.34); 4.4889 (0.48); 4.4748 (0.47); 4.3923 (0.71); 4.3596 (0.76); 4.0547 (0.87); 4.0369 (2.63); 4.0191 (2.70); 4.0012 (1.40); 3.9622 (0.72); 3.7620 (1.05); 3.7309 (1.29); 3.7201 (1.84); 3.6890 (1.47); 3.5752 (1.59); 3.5516 (1.58); 3.5332 (1.13); 3.5097 (1.10); 3.4242 (0.83); 3.3788 (255.26); 3.3768 (255.06); 3.3727 (209.45); 3.3699 (232.41); 3.3125 (1.04); 3.3074 (1.07); 3.2773 (1.18); 3.2489 (0.69); 3.2435 (0.63); 2.8774 (0.54); 2.8465 (0.94); 2.8206 (0.55); 2.6783 (0.38); 2.6737 (0.53); 2.6691 (0.39); 2.5271 (0.84); 2.5224 (1.36); 2.5136 (27.60); 2.5092 (60.18); 2.5046 (80.99); 2.5000 (57.47); 2.4955 (26.85); 2.3358 (0.40); 2.3313 (0.55); 2.3267 (0.40); 2.2088 (16.00); 2.1502 (0.67); 2.1123 (1.37); 2.0865 (0.77); 2.0771 (0.87); 1.9903 (12.30); 1.8314 (0.58); 1.8229 (0.64); 1.8004 (0.59); 1.7919 (0.55); 1.5907 (0.55); 1.5814 (0.64); 1.5603 (0.59); 1.5511 (0.58); 1.1921 (3.28); 1.1743 (6.54); 1.1565 (3.22); -0.0002 (4.51);
**Bsp. 51*, Lösungsmittel: DMSO, Spektrometer: 399.95 MHz**
   7.9706 (8.74); 7.3989 (0.78); 7.3817 (0.97); 7.3779 (1.64); 7.3609 (1.66); 7.3571 (1.04); 7.3400 (0.84); 6.9372 (2.31); 6.9160 (2.12); 6.8675 (1.21); 6.8422 (1.42); 6.8207 (1.13); 6.5037 (4.21); 6.1077 (1.07); 6.0857 (1.30); 6.0770 (1.24); 6.0548 (1.13); 5.9293 (0.34); 5.9160 (0.74); 5.9029 (0.73); 5.8895 (0.86); 5.8864 (0.53); 5.8729 (0.95); 5.8596 (0.86); 5.8465 (0.92); 5.8334 (0.45); 5.3659 (1.00); 5.3406 (1.76); 5.3366 (1.84); 5.3327 (0.85); 5.3235 (2.65); 5.3012 (0.59); 5.2974 (1.51); 5.2933 (1.53); 5.2895 (0.62); 5.2665 (2.53); 5.2239 (0.98); 5.2032 (0.61); 5.1221 (1.65); 5.1185 (1.63); 5.0957 (1.55); 5.0922 (1.53); 4.5906 (1.63); 4.5869 (1.21); 4.5786 (2.74); 4.5662 (1.56); 4.3942 (0.70); 4.3609 (0.75); 4.0550 (1.00); 4.0372 (3.03); 4.0194 (3.09); 4.0015 (1.49); 3.9625 (0.71); 3.8005 (0.77); 3.7702 (0.88); 3.7578 (1.10); 3.7280 (1.01); 3.5352 (1.27); 3.5132 (1.28); 3.4928 (0.96); 3.4709 (0.98); 3.4237 (0.44); 3.4120 (0.35); 3.4037 (0.62); 3.3943 (1.11); 3.3854 (0.68); 3.3772 (0.39); 3.3744 (0.40); 3.3657 (0.78); 3.3620 (0.95); 3.3602 (0.99); 3.3556 (1.40); 3.3418 (302.46); 3.3264 (1.23); 3.3180 (1.55); 3.3079 (0.78); 3.3038 (0.71); 3.2744 (0.93); 3.2461 (0.52); 3.2401 (0.47); 3.0429 (1.33); 2.8736 (0.50); 2.8543 (1.43); 2.8467 (0.90); 2.8166 (0.51); 2.6763 (0.42); 2.6719 (0.59); 2.6673 (0.42); 2.5253 (0.96); 2.5206 (1.55); 2.5119 (31.01); 2.5074 (66.11); 2.5029 (88.42); 2.4984 (62.76); 2.4940 (29.40); 2.3344 (0.41); 2.3297 (0.58); 2.3251 (0.42); 2.2091 (16.00); 2.1913 (1.25); 2.1498 (0.67); 2.1466 (0.63); 2.1125 (1.31); 2.0862 (0.71); 2.0776 (0.71); 1.9900 (13.71); 1.8331 (0.55); 1.8264 (0.66); 1.8031 (0.54); 1.7952 (0.52); 1.5914 (0.55); 1.5826 (0.61); 1.5608 (0.58); 1.5508 (0.56); 1.2349 (0.34); 1.1921 (3.53); 1.1743 (7.13); 1.1564 (3.43); 0.0081 (0.47); -0.0002 (15.63); -0.0084 (0.51);
**Bsp. 52*, Lösungsmittel: DMSO, Spektrometer: 601.6 MHz**
   8.0358 (7.61); 7.2774 (0.74); 7.2742 (0.77); 7.2644 (0.94); 7.2612 (1.03); 7.2580 (0.86); 7.2551 (0.81); 7.2446 (0.93); 7.2418 (0.95); 7.1645 (0.94); 7.1617 (1.01); 7.1515 (2.51); 7.1487 (1.98); 7.1409 (1.34); 7.1331 (1.26); 7.1279 (1.36); 7.1200 (1.37); 7.1150 (0.49); 7.1069 (0.50); 6.5053 (4.35); 6.0851 (0.60); 6.0763 (0.47); 6.0669 (0.85); 6.0568 (0.89); 6.0476 (0.52); 6.0393 (0.68); 6.0293 (0.35); 5.9261 (1.26); 5.9136 (1.43); 5.9074 (1.36); 5.8949 (1.27); 5.3966 (1.73); 5.3940 (1.69); 5.3915 (0.67); 5.3681 (1.60); 5.3654 (1.56); 5.3630 (0.64); 5.3509 (1.29); 5.3225 (2.53); 5.2618 (2.61); 5.2407 (1.82); 5.2382 (1.83); 5.2361 (1.33); 5.2336 (1.44); 5.2235 (1.62); 4.6113 (2.93); 4.6016 (2.75); 4.3806 (0.76); 4.3588 (0.79); 4.0335 (0.46); 4.0217 (0.46); 3.9801 (0.75); 3.9567 (0.77); 3.9224 (1.21); 3.9035 (1.38); 3.8938 (1.51); 3.8750 (1.27); 3.3986 (0.64); 3.3926 (0.49); 3.3855 (0.79); 3.3793 (1.41); 3.3493 (580.12); 3.3257 (12.73); 3.3089 (1.64); 3.2826 (0.66); 3.2634 (1.08); 3.2439 (0.62); 3.2396 (0.52); 2.8516 (0.54); 2.8309 (0.97); 2.8134 (0.56); 2.6178 (1.20); 2.6149 (1.62); 2.6119 (1.14); 2.5426 (0.65); 2.5241 (2.96); 2.5211 (4.06); 2.5179 (4.89); 2.5087 (96.04); 2.5061 (192.28); 2.5031 (255.09); 2.5002 (185.23); 2.4804 (1.02); 2.4773 (0.92); 2.3903 (1.26); 2.3873 (1.66); 2.3843 (1.20); 2.2055 (16.00); 2.1928 (0.47); 2.1259 (0.69); 2.0981 (1.15); 2.0783 (2.81); 1.9905 (2.01); 1.8291 (0.33); 1.8150 (0.64); 1.8090 (0.69); 1.7944 (0.66); 1.7890 (0.62); 1.5684 (0.65); 1.5622 (0.69); 1.5482 (0.67); 1.5419 (0.64); 1.3969 (0.47); 1.2337 (1.34); 1.1860 (0.57); 1.1741 (1.09); 1.1623 (0.56); 1.1365 (0.56); 0.0052 (2.96); -0.0002 (72.51); -0.0057 (2.46);
**Bsp. 53, Lösungsmittel: DMSO, Spektrometer: 601.6 MHz**
   10.2595 (5.04); 8.0507 (7.33); 7.7701 (1.91); 7.7685 (2.00); 7.7574 (2.47); 7.7558 (2.50); 7.6824 (0.70); 7.6738 (0.84); 7.6690 (1.38); 7.6607 (1.40); 7.6562 (0.94); 7.6476 (0.77); 7.6120 (1.19); 7.5949 (1.38); 7.5811 (0.83); 6.5807 (1.13); 6.5629 (1.65); 6.5441 (1.15); 6.5072 (4.42); 5.3547 (1.33); 5.3263 (2.70); 5.2687 (2.59); 5.2404 (1.30); 4.3893 (0.87); 4.3673 (0.90); 3.9893 (0.85); 3.9787 (1.05); 3.9761 (1.10); 3.9663 (0.96); 3.9589 (1.23); 3.9501 (1.01); 3.9476 (0.99); 3.9280 (0.85); 3.5858 (1.07); 3.5688 (1.10); 3.5567 (0.95); 3.5400 (0.94); 3.4250 (0.34); 3.4189 (0.62); 3.4124 (0.47); 3.4059 (0.76); 3.3998 (1.23); 3.3935 (0.82); 3.3865 (0.63); 3.3799 (1.01); 3.3550 (256.99); 3.3314 (3.80); 3.2926 (0.68); 3.2728 (1.16); 3.2537 (0.62); 3.2502 (0.55); 2.8615 (0.60); 2.8411 (1.10); 2.8235 (0.61); 2.6182 (0.47); 2.6154 (0.64); 2.6125 (0.50); 2.5244 (1.20); 2.5214 (1.70); 2.5065 (75.93); 2.5036 (101.45); 2.5007 (79.23); 2.3906 (0.48); 2.3877 (0.64); 2.3850 (0.50); 2.2082 (16.00); 2.1456 (0.76); 2.1201 (1.12); 2.0913 (0.88); 2.0788 (6.21); 1.8307 (0.72); 1.8107 (0.69); 1.5836 (0.67); 1.5643 (0.66); 0.0049 (0.68); -0.0002 (13.17);
**Bsp. 54*, Lösungsmittel: DMSO, Spektrometer: 601.6 MHz**
   8.7986 (0.80); 8.0657 (0.34); 8.0560 (7.35); 7.3883 (0.52); 7.3857 (0.58); 7.3745 (1.21); 7.3720 (1.50); 7.3582 (1.39); 7.3556 (1.99); 7.3466 (1.11); 7.3429 (1.41); 7.3341 (1.38); 7.3293 (0.67); 7.3204 (0.56); 7.2762 (1.73); 7.2643 (1.25); 6.5057 (4.67); 5.8076 (1.23); 5.7950 (1.39); 5.7886 (1.33); 5.7758 (1.19); 5.3525 (1.24); 5.3241 (2.42); 5.2606 (2.46); 5.2323 (1.29); 4.3835 (0.74); 4.3617 (0.79); 4.0454 (0.50); 4.0336 (1.54); 4.0217 (1.56); 4.0099 (0.53); 3.9806 (0.75); 3.9577 (0.78); 3.9189 (1.07); 3.8997 (1.30); 3.8902 (1.38); 3.8711 (1.15); 3.4091 (0.41); 3.4028 (0.65); 3.3964 (0.51); 3.3898 (0.91); 3.3834 (1.46); 3.3753 (1.49); 3.3504 (474.54); 3.3268 (8.13); 3.3000 (1.54); 3.2873 (1.89); 3.2713 (1.56); 3.2588 (1.98); 3.2438 (0.60); 3.2396 (0.53); 2.8542 (0.55); 2.8505 (0.58); 2.8296 (1.00); 2.8122 (0.55); 2.8083 (0.49); 2.6209 (0.40); 2.6179 (0.83); 2.6150 (1.11); 2.6120 (0.79); 2.6090 (0.36); 2.5427 (0.68); 2.5242 (2.84); 2.5212 (3.66); 2.5180 (4.29); 2.5092 (64.10); 2.5062 (130.48); 2.5032 (174.29); 2.5002 (125.86); 2.4973 (57.51); 2.4799 (0.46); 2.4766 (0.46); 2.4738 (0.44); 2.3933 (0.43); 2.3904 (0.86); 2.3874 (1.12); 2.3844 (0.81); 2.3814 (0.39); 2.2201 (0.42); 2.2057 (16.00); 2.1901 (0.44); 2.1246 (0.69); 2.0965 (1.20); 2.0782 (2.29); 2.0713 (0.77); 1.9905 (6.90); 1.8821 (0.38); 1.8742 (0.72); 1.8695 (0.72); 1.8640 (0.95); 1.8612 (1.12); 1.8566 (0.62); 1.8537 (0.68); 1.8502 (0.75); 1.8406 (0.54); 1.8104 (0.60); 1.8039 (0.62); 1.7922 (0.72); 1.7867 (0.78); 1.7849 (0.70); 1.7792 (0.77); 1.7736 (0.34); 1.7716 (0.49); 1.7660 (0.46); 1.7354 (1.68); 1.5793 (0.35); 1.5662 (0.72); 1.5589 (0.80); 1.5456 (0.75); 1.5391 (0.65); 1.3969 (0.44); 1.2338 (0.92); 1.1860 (1.90); 1.1741 (3.78); 1.1623 (1.83); 1.0889 (0.49); 1.0841 (0.52); 1.0818 (1.11); 1.0767 (1.64); 1.0716 (0.99); 1.0687 (1.20); 1.0636 (1.59); 1.0587 (1.07); 1.0513 (0.99); 1.0481 (0.88); 1.0438 (1.23); 1.0381 (1.09); 1.0305 (0.91); 1.0247 (0.56); 1.0183 (0.45); 1.0128 (1.07); 1.0103 (0.64); 1.0053 (2.12); 1.0003 (1.42); 0.9975 (0.90); 0.9918 (2.06); 0.9789 (1.26); 0.9693 (0.47); 0.9641 (0.41); 0.0052 (1.72); -0.0002 (43.02); -0.0057 (1.34);
**Bsp. 55*, Lösungsmittel: DMSO, Spektrometer: 399.95 MHz**
   7.9900 (5.11); 7.9528 (0.50); 7.3211 (0.55); 7.3176 (0.67); 7.2998 (3.31); 7.2812 (2.93); 7.0705 (1.58); 7.0499 (1.35); 6.9729 (0.99); 6.9546 (1.73); 6.9355 (0.83); 6.4972 (3.18); 5.8503 (0.83); 5.8310 (0.97); 5.8223 (0.94); 5.8027 (0.85); 5.7551 (4.32); 5.3500 (0.66); 5.3070 (1.88); 5.2535 (1.78); 5.2112 (0.63); 4.3837 (0.53); 4.3514 (0.57); 4.1549 (1.74); 4.1435 (2.88); 4.1318 (1.83); 3.9877 (0.51); 3.9499 (0.57); 3.8507 (0.85); 3.8225 (0.88); 3.8079 (1.06); 3.7800 (0.93); 3.6311 (1.80); 3.6195 (2.15); 3.6085 (1.44); 3.4026 (0.53); 3.3942 (0.34); 3.3750 (0.87); 3.3652 (0.66); 3.3274 (220.17); 3.2923 (1.45); 3.2735 (1.17); 3.2628 (0.82); 3.2288 (16.00); 2.8907 (2.87); 2.8654 (0.38); 2.8617 (0.39); 2.8323 (0.69); 2.8040 (0.40); 2.7315 (2.65); 2.6709 (0.42); 2.5018 (70.79); 2.3284 (0.42); 2.2064 (11.12); 2.1227 (0.49); 2.0958 (1.01); 2.0736 (0.61); 2.0630 (0.58); 1.9887 (0.52); 1.8156 (0.47); 1.8063 (0.47); 1.7840 (0.43); 1.7776 (0.42); 1.5733 (0.45); 1.5657 (0.46); 1.5433 (0.45); 1.5368 (0.43); 1.2357 (0.62); -0.0002 (10.11); -0.0082 (0.34);
**Bsp. 56*, Lösungsmittel: DMSO, Spektrometer: 399.95 MHz** 8.7868 (0.75); 8.0463 (0.81); 8.0388 (8.80); 7.4462 (1.28); 7.4419 (1.52); 7.4270 (1.61); 7.4229 (2.06); 7.4147 (0.94); 7.4104 (0.83); 7.3957 (1.69); 7.3911 (1.41); 7.3761 (1.48); 7.3717 (1.20); 7.3397 (0.34); 7.3110 (1.23); 7.3082 (1.42); 7.2922 (1.83); 7.2893 (1.97); 7.2736 (0.78); 7.2706 (0.76); 7.1763 (2.17); 7.1735 (2.19); 7.1562 (1.87); 7.1534 (1.78); 6.4972 (4.64); 5.7554 (11.20); 5.7402 (1.29); 5.7311 (1.31); 5.7115 (1.10); 5.3515 (0.79); 5.3091 (2.37); 5.2551 (2.53); 5.2130 (0.86); 4.3857 (0.69); 4.3536 (0.72); 4.0558 (0.33); 4.0379 (1.00); 4.0201 (1.05); 4.0023 (0.53); 3.9867 (0.68); 3.9522 (0.71); 3.8958 (1.11); 3.8672 (1.30); 3.8526 (1.44); 3.8241 (1.19); 3.4178 (0.34); 3.4083 (0.53); 3.3985 (0.40); 3.3893 (0.64); 3.3796 (1.07); 3.3705 (0.66); 3.3608 (0.54); 3.3515 (0.79); 3.3267 (95.06); 3.3230 (198.62); 3.2903 (1.88); 3.2705 (1.95); 3.2471 (1.43); 3.2359 (0.69); 3.2276 (1.64); 2.8628 (0.55); 2.8356 (0.90); 2.8060 (0.51); 2.6750 (0.40); 2.6705 (0.54); 2.6658 (0.41); 2.5406 (0.34); 2.5238 (0.95); 2.5191 (1.43); 2.5098 (30.88); 2.5057 (60.00); 2.5013 (83.80); 2.4971 (58.45); 2.4928 (29.21); 2.3329 (0.41); 2.3281 (0.52); 2.3236 (0.39); 2.2066 (16.00); 2.1936 (0.85); 2.1291 (0.65); 2.0990 (1.32); 2.0734 (1.01); 1.9884 (4.67); 1.8725 (0.41); 1.8566 (0.73); 1.8468 (0.87); 1.8412 (1.36); 1.8356 (0.87); 1.8301 (0.85); 1.8223 (0.97); 1.8105 (0.93); 1.7848 (0.54); 1.5759 (0.57); 1.5668 (0.59); 1.5442 (0.58); 1.5355 (0.54); 1.2344 (0.35); 1.1923 (1.29); 1.1745 (2.61); 1.1567 (1.28); 1.0728 (0.54); 1.0647 (0.67); 1.0578 (0.73); 1.0521 (1.19); 1.0467 (1.39); 1.0304 (2.30); 1.0194 (1.51); 1.0140 (2.46); 1.0076 (1.75); 1.0021 (1.05); 0.9962 (0.99); 0.9871 (1.16); 0.9824 (0.83); 0.9742 (0.89); 0.9631 (1.22); 0.9488 (0.63); 0.9430 (0.61); -0.0002 (3.06);
   *:nicht erfindungsgemäße Verbindungen (Vergleichsbeispiele)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

### Verwendungsbeispiele

### Beispiel A

### Phytophthora-Test (Tomate) / protektiv

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von ***Phytophthora infestans*** inokuliert und stehen dann 24h bei 100% rel. Feuchte und 22°C. Anschließend werden die Pflanzen in einer Klimazelle bei ca. 96% relativer Luftfeuchtigkeit und einer Temperatur von ca. 20°C aufgestellt. 7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr.

**Tabelle 2:**

| **Ex_no.** | **Eff.%** |
|---|---|
| 1 | 100 |
| 2 | 100 |
| 3 | 100 |
| 4 | 95 |
| 5 | 95 |
| 6 | 95 |
| 7 | 100 |
| 8* | 100 |
| 9* | 94 |
| 11* | 85 |
| 12* | 75 |
| 13* | 89 |
| 18* | 100 |
| 19* | 98 |
| 25* | 100 |
| 26* | 100 |
| 29* | 100 |
| 30* | 97 |
| 34* | 100 |
| 35* | 100 |
| 36* | 100 |
| 37* | 94 |
| 38* | 94 |
| 39* | 94 |
| 40* | 100 |
| 41* | 98 |
| 41* | 97 |
| 42* | 94 |
| 43* | 97 |
| 44* | 94 |
| 45* | 100 |
| 46* | 100 |
| 47* | 100 |
| 48* | 70 |
| 49* | 90 |
| 50* | 70 |
| 51* | 85 |
| 52* | 97 |
| 53 | 98 |
| 54* | 94 |
| 55* | 92 |
| 56* | 89 |

| | |
|---|---|
| *:nicht erfindungsgemäße Verbindungen (Vergleichsbeispiele) | |

### Beispiel B

### Plasmopara-Test (Rebe) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Plasmopara viticola*** inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 10ppm einen Wirkungsgrad von 70% oder mehr.

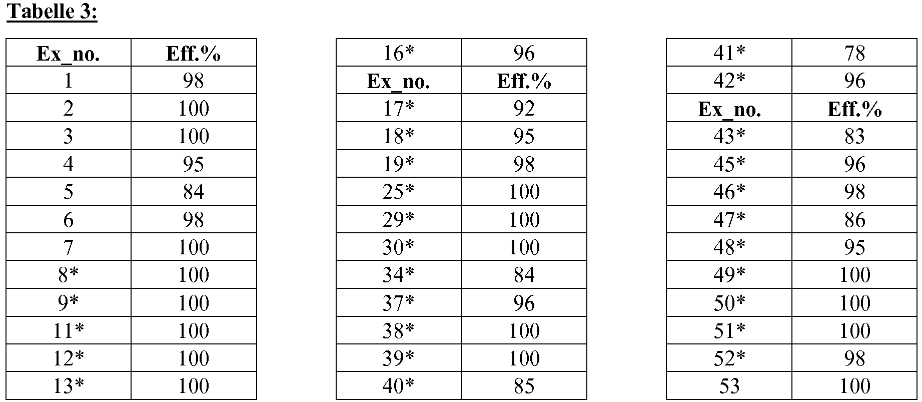

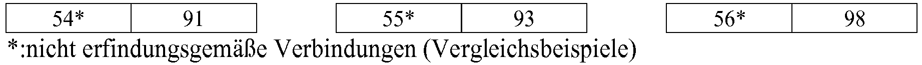

## Patentansprüche

1. Verbindungen der Formel **(I),** 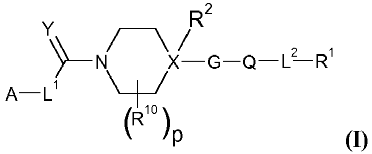 in welcher die Restedefinitionen folgende Bedeutungen haben:
A steht für Phenyl, das bis zu zwei Substituenten enthalten kann, wobei die Substituenten unabhängig voneinander ausgewählt sind aus folgender Liste: Fluor, Brom, Iod, Chlor, Cyano, Nitro, Methyl, Ethyl, *n*-Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, *n*-Propoxy, Methoxy, Trifluormethoxy, Difluormethoxy, 1-Methylethylthio, Methylthio, Ethylthio, *n*-Propylthio, Difluormethylthio oder Trifluormethylthio, oder
A steht für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten gleich oder verschieden und unabhängig voneinander ausgewählt sind aus folgender Liste:
Substituenten am Kohlenstoff:
Fluor, Chlor, Brom, Iod, Cyano, Nitro, Methyl, Ethyl, *n*-Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, *n*-Propoxy, Methoxy, Trifluormethoxy, Difluormethoxy, 1-Methylethylthio, Methylthio, Ethylthio, *n*-Propylthio, Difluormethylthio, Trifluormethylthio oder Phenyl,
Substituenten am Stickstoff:
Methyl, Ethyl, *n*-Propyl, 1-Methylethyl, Methylsulfonyl, Trifluormethylsulfonyl, Methylcarbonyl, Trifluormethylcarbonyl, Chlormethylcarbonyl, 2,2-Trifluorethyl, 2,2-Difluorethyl, 2,2-Dichlor-2-fluorethyl, 2-Chlor-2-difluorethyl oder 2-Chlor-2-fluorethyl
L¹ steht für CHR^{L11} oder NR^{L12},
R^{L11} steht für Wasserstoff oder Methyl,
R^{L12} steht für Wasserstoff oder Methyl,
Y steht für Sauerstoff,
X steht für Kohlenstoff,
R² steht für Wasserstoff, Fluor, Chlor, Brom oder Hydroxy,
R¹⁰ steht für Fluor, Chlor, Brom oder Hydroxy,
p steht für 0,
G steht für 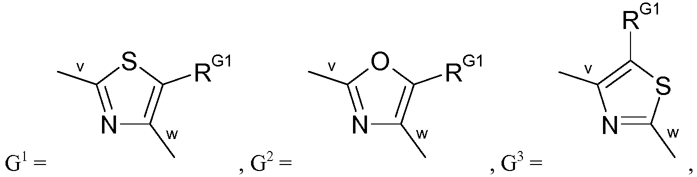
wobei die Bindung, die mit "v" identifiziert ist, direkt an X gebunden ist, und wobei die Bindung, die mit "w" identifiziert ist, direkt an Q gebunden ist,
Q steht für 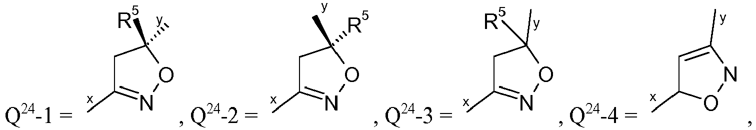 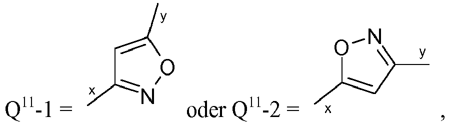
wobei die Bindung, die mit "x" identifiziert ist, direkt an G gebunden ist und die Bindung, die mit "y" identifiziert ist, direkt an L² gebunden ist,
R⁵ steht für Wasserstoff, Cyano, Methyl, Trifluomethyl, Difluormethyl oder Methoxymethyl,
R^{G1} steht für Wasserstoff,
L² steht für eine direkte Bindung, -C(=O)-, -CHR²⁰- oder -NR²¹-,
R²⁰ steht für Wasserstoff, Methyl, Ethyl, Trifluormethyl,
R²¹ steht für Wasserstoff oder Methyl,
R¹ steht für Phenyl, das 1, 2 oder 3 Substituenten enthalten kann, wobei ein Substituent ausgewählt ist aus Z⁴ und gegebenenfalls weitere Substituenten ausgewählt sind aus folgender Liste: Fluor, Chlor, Brom, Iod, Cyano, Nitro, Hydroxy, Amino, -SH, -C(=O)H, Methyl, Ethyl, *n*-Propyl, 1-Methylethyl, *n*-Butyl, 1,1-Dimethylethyl, 1,2-Dimethylethyl, Ethenyl, Ethinyl, Trifluormethyl, Difluormethyl, Trichlormethyl, Dichlormethyl, Cyclopropyl, Methoxy, Ethoxy, *n*-Propoxy, 1-Methylethoxy, 1,1-Dimethylethoxy, Methylcarbonyl, Ethylcarbonyl, Trifluormethylcarbonyl, Methoxycarbonyl, Ethoxycarbonyl, *n*Propoxycarbonyl, 1-Methylethoxycarbonyl, 1,1-Dimethylethoxycarbonyl, 1-Ethenyloxy, 2-Propenyloxy, 2-Propinyloxy, Methylcarbonyloxy, Trifluoralkylcarbonyloxy, Chlormethylcarbonyloxy, Methylthio, Ethylthio, Methylsulfonyl, Methylsulfonylamino, Trifluormethylsulfonylamino oder -L³R³,
L³ steht für eine direkte Bindung, R³ steht für Wasserstoff, Methyl, Ethyl, *n*-Propyl, 1-Methylethyl, *n*-Butyl oder 1,1-Dimethylethyl,
Z⁴ steht für C(=O)H, Alkylsulfonylamino oder Halogenalkylsulfonylamino,
sowie Salze, Metallkomplexe und N-Oxide der Verbindungen der Formel **(I),** mit der Maßgabe, dass folgende Verbindungen ausgenommen sind: 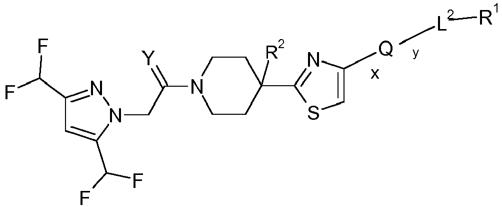 in welcher die Symbole folgende Bedeutungen haben:
Y steht für Sauerstoff oder Schwefel,
R² steht für Wassertoff oder Halogen
Q steht für 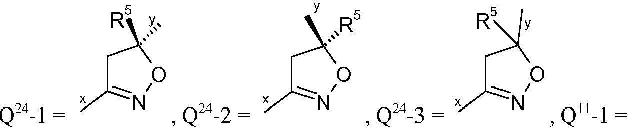 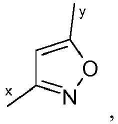
R⁵ steht für Wasserstoff, Cyano, C₁-C₃-Alkyl oder C₁-C₃-Halogenalkyl,
L² steht für eine direkte Bindung und
R¹ hat die oben genannte Bedeutung, wobei wenn R1 für jeweils substituiertes Phenyl, Naphthyl, Dihydronaphthalenyl, Tetrahydronaphthalenyl, Hexahydronaphthalenyl, Octahydronaphthalenyl, Indenyl oder für eine gegebenenfalls benzokondensiertes, 5- oder 6-gliedriges Heteroaryl steht, höchstens zwei Substituenten an den genannten Carbocyclen bzw. Heterocyclen vorliegen.

2. Verbindungen der Formel (I) nach Anspruch 1, in welcher die Restedefinitionen folgende Bedeutungen haben:
A steht für Phenyl, der bis zu zwei Substituenten enthalten kann, wobei die Substituenten unabhängig voneinander ausgewählt sind aus folgender Liste: Methyl, Ethyl, Iod, Chlor, Brom, Fluor, Methoxy, Ethoxy, Difluormethyl oder Trifluormethyl oder
A steht für Pyrazol-1-yl, der bis zu zwei Substituenten enthalten kann, wobei die Substituenten unabhängig voneinander ausgewählt sind aus folgender Liste: Methyl, Ethyl, Chlor, Brom, Fluor oder Trifluormethyl.

3. Verbindungen der Formel (I) nach einem der vorherigen Ansprüche, in welcher die Restedefinitionen folgende Bedeutungen haben:
G steht für G¹, G² oder G³ und insbesondere für G¹,
R^{G1} steht für Wasserstoff.

4. Verbindungen der Formel (I) nach einem der vorherigen Ansprüche, in welcher die Restedefinitionen folgende Bedeutungen haben:
A steht für 5-Methyl-3-(trifluormethyl)-1H-pyrazol-1-yl, 5-Chlor-2-methylphenyl oder für 2,5-Bis(difluoromethyl)phenyl,
L¹ steht für -CH₂- oder -NH-,
L² steht für eine direkte Bindung,
Y steht für Sauerstoff,
X steht für Kohlenstoff,
p steht für 0,
G steht für G¹,
R^{G1} steht für Wasserstoff,
Q steht für Q²⁴-3,
R² steht für Wasserstoff,
R⁵ steht für Wasserstoff,
R¹ steht für 3-Formylphenyl, 2-Formylphenyl oder 2-[(Methylsulfonyl)amino]phenyl.

5. Verbindungen der Formel **(VII),** 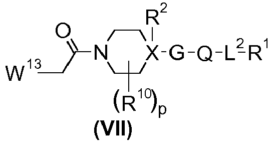 sowie Salze, Metallkomplexe und N-Oxide davon, in denen die Symbole R², X, G, Q, L², R¹, R¹⁰ und p die in einem der Ansprüche 1 bis 4 angegebene Bedeutung haben und W¹³ für eine Abgangsgruppe steht.

6. Verbindungen der Formel **(XVIId),** 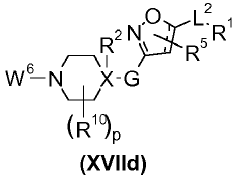 sowie Salze Metallkomplexe und N-Oxide davon, in denen die Symbole W⁶ für Acetyl, C₁-C₄ Alkoxycarbonyl, Benzyl oder Benzyloxycarbonyl steht und die Symbole X, L², p, R¹, R², R⁵ und R¹⁰ die in einem der Ansprüche 1 bis 4 angegebene Bedeutung haben.

7. Verbindungen der Formel **(XIIIa),** 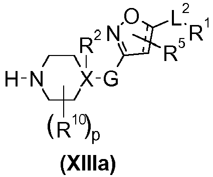 sowie Salze, Metallkomplexe und N-Oxide davon, in denen die Symbole X, L², p, G, R¹, R², R⁵ und R¹⁰ die in einem der Ansprüche 1 bis 4 angegebene Bedeutung haben.

8. Verfahren zur Bekämpfung von pflanzenpathogenen Schadpilzen, **dadurch gekennzeichnet, dass** man Verbindungen der Formel (I) gemäß einem der Ansprüche 1 bis 4 auf die pflanzenpathogenen Schadpilze und/oder deren Lebensraum ausbringt.

9. Mittel zur Bekämpfung von pflanzenpathogenen Schadpilzen, **gekennzeichnet durch** einen Gehalt an mindestens einer Verbindung der Formel (I) gemäß einem der Ansprüche 1 bis 4, neben Streckmitteln und/oder oberflächenaktiven Stoffen.

10. Verwendung von Verbindungen der Formel (I), gemäß einem der Ansprüche 1 bis 4 zur Bekämpfung von pflanzenpathogenen Schadpilzen.

11. Verfahren zur Herstellung von Mitteln zur Bekämpfung von pflanzenpathogenen Schadpilzen, **dadurch gekennzeichnet, dass** man Verbindungen der Formel (I) gemäß einem der Ansprüche 1 bis 4 mit Streckmitteln und/oder oberflächenaktiven Stoffen vermischt.

12. Verwendung von Verbindungen der Formel (I) gemäß einem der Ansprüche 1 bis 4 zur Behandlung von transgenen Pflanzen.

## Claims

1. Compounds of the formula **(I)** 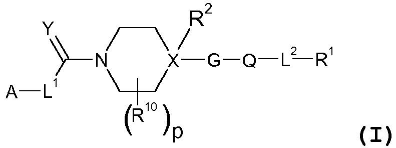 in which the radicals are each defined as follows:
A is phenyl which may contain up to two substituents, where the substituents are each independently selected from the following list: fluorine, bromine, iodine, chlorine, cyano, nitro, methyl, ethyl, *n*-propyl, 1-methylethyl, 1,1-dimethylethyl, chlorofluoromethyl, dichloromethyl, dichlorofluoromethyl, difluoromethyl, trichloromethyl, trifluoromethyl, cyclopropyl, ethoxy, 1-methylethoxy, *n*-propoxy, methoxy, trifluoromethoxy, difluoromethoxy, 1-methylethylthio, methylthio, ethylthio, *n*-propylthio, difluoromethylthio or trifluoromethylthio, or
A is a heteroaromatic radical selected from the following group: furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,3-triazol-1-yl, 1,2,4-triazol-1-yl, pyridin-2-yl, pyridin-3-yl, pyridin-4-yl, pyridazin-3-yl, pyridazin-4-yl, pyrimidin-2-yl, pyrimidin-4-yl or pyrimidin-5-yl which may contain up to two substituents, where the substituents are indentical or different and are each independently selected from the following list: substituents on carbon: fluorine, chlorine, bromine, iodine, cyano, nitro, methyl, ethyl, n-propyl, 1-methylethyl, 1,1-dimethylethyl, chlorofluoromethyl, dichloromethyl, dichlorofluoromethyl, difluoromethyl, trichloromethyl, trifluoromethyl, cyclopropyl, ethoxy, 1-methylethoxy, n-propoxy, methoxy, tri-fluoromethoxy, difluoromethoxy, 1-methylethylthio, methylthio, ethylthio, n-propylthio, difluoromethylthio, trifluoromethylthio or phenyl, substituents on nitrogen: methyl, ethyl, n-propyl, 1-methylethyl, methylsulphonyl, trifluoromethylsulphonyl, methylcarbonyl, trifluoromethylcarbonyl, chloromethylcarbonyl, 2,2-trifluoroethyl, 2,2-difluoroethyl, 2,2-dichloro-2-fluoroethyl, 2-chloro-2-difluoroethyl or 2-chloro-2-fluoroethyl,
L¹ is CHR^{L11} or NR^{L12},
R^{L11} is hydrogen or methyl,
R^{L12} is hydrogen or methyl,
Y is oxygen,
X is carbon,
R² is hydrogen, fluorine, chlorine, bromine or hydroxyl,
R¹⁰ is fluorine, chlorine, bromine or hydroxyl,
p is 0,
G is 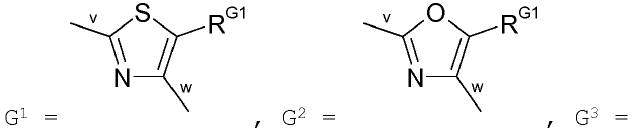 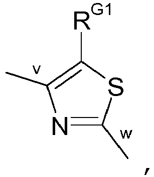
where the bond identified by "v" is bonded directly to X and where the bond identified by "w" is bonded directly to Q,
Q is 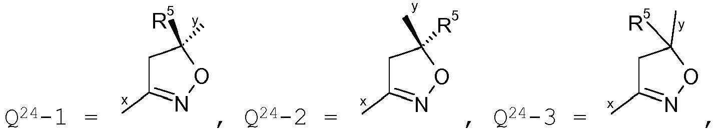 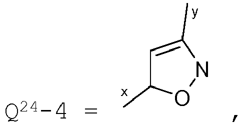 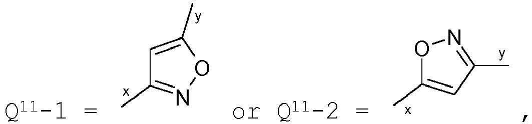
where the bond identified by "x" is bonded directly to G, and the bond identified by "y" is bonded directly to L²,
R⁵ is hydrogen, cyano, methyl, trifluoromethyl, difluoromethyl or methoxymethyl,
R^{G1} is hydrogen,
L² is a direct bond, -C(=O)-, -CHR²⁰- or -NR²¹-,
R²⁰ is hydrogen, methyl, ethyl, trifluoromethyl,
R²¹ is hydrogen or methyl,
R¹ is phenyl which may contain 1, 2 or 3 substituents, where one substituent is selected from Z⁴ and any further substituents are selected from the following list: fluorine, chlorine, bromine, iodine, cyano, nitro, hydroxyl, amino, -SH, -C(=O)H, methyl, ethyl, *n*-propyl, 1-methylethyl, *n*-butyl, 1,1-dimethylethyl, 1,2-dimethylethyl, ethenyl, ethynyl, trifluoromethyl, difluoromethyl, trichloromethyl, di-chloromethyl, cyclopropyl, methoxy, ethoxy, *n-*propoxy, 1-methylethoxy, 1,1-dimethylethoxy, methylcarbonyl, ethylcarbonyl, trifluoromethylcarbonyl, methoxycarbonyl, ethoxycarbonyl, *n-*propoxycarbonyl, 1-methylethoxycarbonyl, 1,1-dimethylethoxycarbonyl, 1-ethenyloxy, 2-propenyloxy, 2-propynyloxy, methylcarbonyloxy, trifluoroalkylcarbonyloxy, chloromethylcarbonyloxy, methylthio, ethylthio, methylsulphonyl, methylsulphonylamino, trifluoromethylsulphonylamino or -L³R³,
L³ is a direct bond
R³ is hydrogen, methyl, ethyl, *n*-propyl, 1-methylethyl, *n*-butyl or 1,1-dimethylethyl,
Z⁴ is C(=O)H, alkylsulphonylamino or haloalkylsulphonylamino,
and salts, metal complexes and N-oxides of the compounds of the formula **(I),**
with the proviso that the following compounds are excluded: 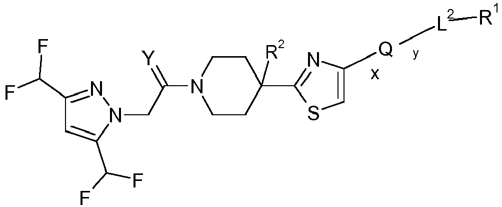 in which the symbols are each defined as follows:
Y is oxygen or sulphur,
R² is hydrogen or halogen,
Q is 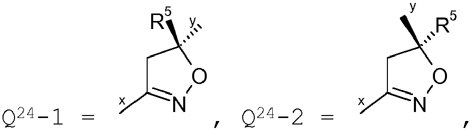 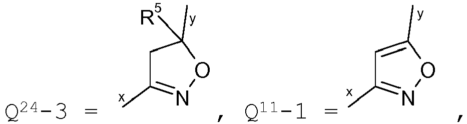
R⁵ is is hydrogen, cyano, C₁-C₃-alkyl or C₁-C₃-haloalkyl,
L² is a direct bond and
R¹ is as defined above, where, when R1 is sub-stituted phenyl, naphthyl, dihydronaphthalenyl, tetrahydronaphthalenyl, hexahydronaphthalenyl, octahydronaphthalenyl or indenyl or an optionally benzofused, 5- or 6-membered hetero-aryl, not more than two substituents are present on the carbocycles or heterocycles mentioned.

2. Compounds of the formula (I) according to Claim 1, in which the radical definitions are each as follows:
A is phenyl which may contain up to two substituents, where the substituents are each independently selected from the following list: methyl, ethyl, iodine, chlorine, bromine, fluorine, methoxy, ethoxy, difluoromethyl or trifluoromethyl or
A is pyrazol-1-yl which may contain up to two substituents, where the substituents are each independently selected from the following list: methyl, ethyl, chlorine, bromine, fluorine or trifluoromethyl.

3. Compounds of the formula (I) according to either of the preceding claims, in which the radical definitions are each as follows:
G is G¹, G² or G³ and especially G¹,
R^{G1} is hydrogen.

4. Compounds of the formula (I) according to any of the preceding claims, in which the radical definitions are each as follows:
A is 5-methyl-3-(trifluoromethyl)-1H-pyrazol-1-yl, 5-chloro-2-methylphenyl or 2,5-bis(difluoromethyl)phenyl,
L¹ is -CH₂- or -NH-,
L² is a direct bond,
Y is oxygen,
X is carbon,
p is 0,
G is G¹,
R^{G1} is hydrogen,
Q is Q²⁴-3,
R² is hydrogen,
R⁵ is hydrogen,
R¹ is 3-formylphenyl, 2-formylphenyl or 2-[(methylsulphonyl)amino]phenyl.

5. Compounds of the formula **(VII)** 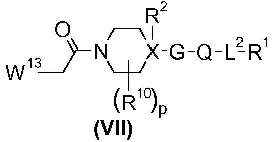 and salts, metal complexes and N-oxides thereof, in which the symbols R², X, G, Q, L², R¹, R¹⁰ and p are each as defined in any of Claims 1 to 4 and W¹³ is a leaving group.

6. Compounds of the formula **(XVIId)** 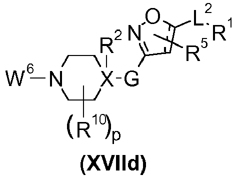 and salts, metal complexes and N-oxides thereof, in which the symbol W⁶ is acetyl, C₁-C₄ alkoxycarbonyl, benzyl or benzyloxycarbonyl and the symbols X, L², p, R¹, R², R⁵ and R¹⁰ are each as defined in any of Claims 1 to 4.

7. Compounds of the formula **(XIIIa)** 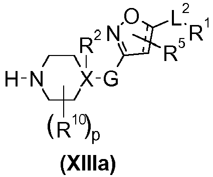 and salts, metal complexes and N-oxides thereof, in which the symbols X, L², p, G, R¹, R², R⁵ and R¹⁰ are each as defined in any of Claims 1 to 4.

8. Method for controlling phytopathogenic harmful fungi, **characterized in that** compounds of the formula (I) according to any of Claims 1 to 4 are applied to the phytopathogenic harmful fungi and/or their habitat.

9. Composition for controlling phytopathogenic harmful fungi, **characterized by** a content of at least one compound of the formula (I) according to any of Claims 1 to 4, in addition to extenders and/or surfactants.

10. Use of compounds of the formula (I) according to any of Claims 1 to 4 for controlling phytopathogenic harmful fungi.

11. Process for producing compositions for controlling phytopathogenic harmful fungi, **characterized in that** compounds of the formula (I) according to any of Claims 1 to 4 are mixed with extenders and/or surfactants.

12. Use of compounds of the formula (I) according to any of Claims 1 to 4 for treatment of transgenic plants.

## Revendications

1. Composés de formule (I) 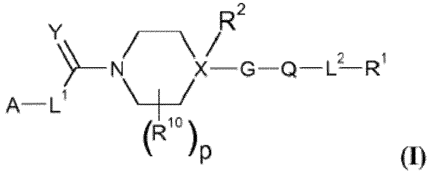 dans laquelle les radicaux ont les significations suivantes :
A représente phényle, qui peut contenir jusqu'à deux substituants, les substituants étant choisis indépendamment les uns des autres dans la liste suivante : fluor, brome, iode, chlore, cyano, nitro, méthyle, éthyle, *n*-propyle, 1-méthyléthyle, 1,1-diméthyléthyle, chlorofluorométhyle, dichlorométhyle, dichlorofluorométhyle, difluorométhyle, trichlorométhyle, trifluorométhyle, cyclopropyle, éthoxy, 1-méthyléthoxy, *n*-propoxy, méthoxy, trifluorométhoxy, difluorométhoxy, 1-méthyléthylthio, méthylthio, éthylthio, *n*-propylthio, difluorométhylthio ou trifluorométhylthio, ou
A représente un radical hétéroaromatique choisi dans le groupe suivant : furan-2-yle, furan-3-yle, thiophén-2-yle, thiophén-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,3-triazol-1-yle, 1,2,4-triazol-1-yle, pyridin-2-yle, pyridin-3-yle, pyridin-4-yle, pyridazin-3-yle, pyridazin-4-yle, pyrimidin-2-yle, pyrimidin-4-yle ou pyrimidin-5-yle, qui peut contenir jusqu'à deux substituants, les substituants étant identiques ou différents, et étant choisis indépendamment les uns des autres dans la liste suivante : substituants sur le carbone : fluor, chlore, brome, iode, cyano, nitro, méthyle, éthyle, *n*-propyle, 1-méthyléthyle, 1,1-diméthyléthyle, chlorofluorométhyle, dichlorométhyle, dichlorofluorométhyle, difluorométhyle, trichlorométhyle, trifluorométhyle, cyclopropyle, éthoxy, 1-méthyléthoxy, *n*-propoxy, méthoxy, trifluorométhoxy, difluorométhoxy, 1-méthyléthylthio, méthylthio, éthylthio, *n*-propylthio, difluorométhylthio, trifluorométhylthio ou phényle, substituants sur l'azote: méthyle, éthyle, *n*-propyle, 1-méthyléthyle, méthylsulfonyle, trifluorométhylsulfonyle, méthylcarbonyle, trifluorométhylcarbonyle, chlorométhylcarbonyle, 2,2-trifluoroéthyle, 2,2-difluoroéthyle, 2,2-dichloro-2-fluoroéthyle, 2-chloro-2-difluoroéthyle ou 2-chloro-2-fluoroéthyle,
L¹ représente CHR^{L11} ou NR^{L12},
R^{L11} représente hydrogène ou méthyle,
R^{L12} représente hydrogène ou méthyle,
Y représente oxygène,
X représente carbone,
R² représente hydrogène, fluor, chlore, brome ou hydroxy,
R¹⁰ représente fluor, chlore, brome ou hydroxy,
p représente 0,
G représente 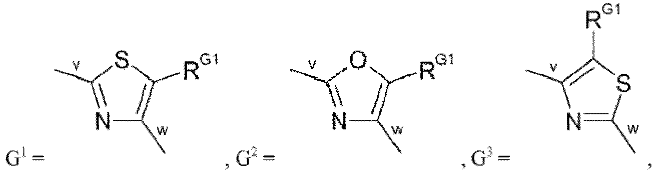
la liaison qui est identifiée par « v » étant directement reliée à X et la liaison qui est identifiée par « w » étant directement reliée à Q,
Q représente 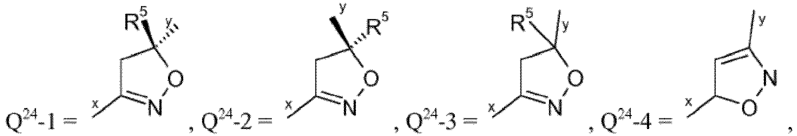 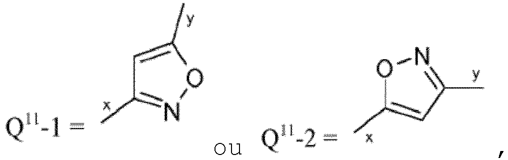
la liaison qui est identifiée par « x » étant directement reliée à G et la liaison qui est identifiée par « y » étant directement reliée à L²,
R⁵ représente hydrogène, cyano, méthyle, trifluorométhyle, difluorométhyle ou méthoxyméthyle,
R^{G1} représente hydrogène,
L² représente une liaison directe, -C(=O)-, -CHR²⁰- ou -NR²¹-,
R²⁰ représente hydrogène, méthyle, éthyle, trifluorométhyle,
R²¹ représente hydrogène ou méthyle,
R¹ représente phényle, qui peut contenir 1, 2 ou 3 substituants, un substituant étant choisi parmi Z⁴ et d'autres substituants étant éventuellement choisis dans la liste suivante: fluor, chlore, brome, iode, cyano, nitro, hydroxy, amino, -SH,-C(=O)H, méthyle, éthyle, n-propyle, 1-méthyléthyle, *n*-butyle, 1,1-diméthyléthyle, 1,2-diméthyléthyle, éthényle, éthinyle, trifluorométhyle, difluorométhyle, trichlorométhyle, dichlorométhyle, cyclopropyle, méthoxy, éthoxy, *n*-propoxy, 1-méthyléthoxy, 1,1-diméthyléthoxy, méthylcarbonyle, éthylcarbonyle, trifluorométhylcarbonyle, méthoxycarbonyle, éthoxycarbonyle, *n*-propoxycarbonyle, 1-méthyléthoxycarbonyle, 1,1-diméthyléthoxycarbonyle, 1-éthényloxy, 2-propényloxy, 2-propinyloxy, méthylcarbonyloxy, trifluoroalkylcarbonyloxy, chlorométhylcarbonyloxy, méthylthio, éthylthio, méthylsulfonyle, méthylsulfonylamino, trifluorométhylsulfonylamino ou -L³R³,
L³ représente une liaison directe,
R³ représente hydrogène, méthyle, éthyle, *n*-propyle, 1-méthyléthyle, *n*-butyle ou 1,1-diméthyléthyle,
Z⁴ représente C(=O)H, alkylsulfonylamino ou halogénoalkylsulfonylamino,
ainsi que les sels, complexes métalliques et N-oxydes des composés de formule (I),
à condition que les composés suivants soient exclus : 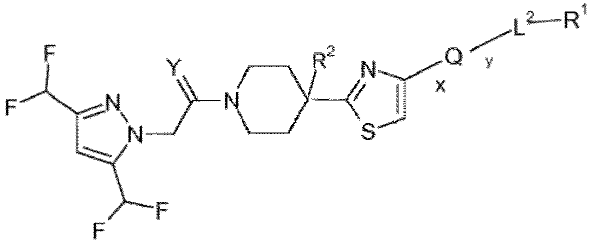 dans laquelle les symboles ont les significations suivantes :
Y représente oxygène ou soufre,
R² représente hydrogène ou halogène,
Q représente 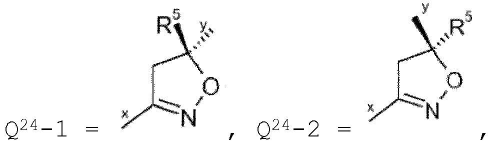 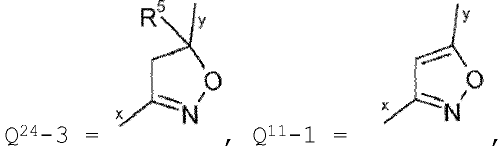
R⁵ représente hydrogène, cyano, alkyle en C₁-C₃ ou halogénoalkyle en C₁-C₃,
L² représente une liaison directe, et
R¹ a la signification indiquée précédemment, lorsque R¹ représente un phényle, un naphtyle, un di-hydronaphtalényle, un tétrahydronaphtalényle, un hexahydronaphtalényle, un octahydronaphtalényle, un indényle ou un hétéroaryle à 5 ou 6 chaînons, éventuellement benzocondensé, à chaque fois substitué, au plus deux substituants étant présents sur les carbocycles ou hétérocycles mentionnés.

2. Composés de formule (I) selon la revendication 1, dans lesquels les radicaux ont les significations suivantes :
A représente phényle, qui peut contenir jusqu'à deux substituants, les substituants étant choisis indépendamment les uns des autres dans la liste suivante : méthyle, éthyle, iode, chlore, brome, fluor, méthoxy, éthoxy, difluorométhyle ou trifluorométhyle,
ou
A représente pyrazol-1-yl, qui peut contenir jusqu'à deux substituants, les substituants étant choisis indépendamment les uns des autres dans la liste suivante : méthyle, éthyle, chlore, brome, fluor ou tri-fluorométhyle.

3. Composés de formule (I) selon l'une quelconque des revendications précédentes, dans lesquels les radicaux ont les significations suivantes :
G représente G¹, G² ou G³, et notamment G¹,
R^{G1} représente hydrogène.

4. Composés de formule (I) selon l'une quelconque des revendications précédentes, dans lesquels les radicaux ont les significations suivantes :
A représente 5-méthyl-3-(trifluorométhyl)-1H-pyrazol-1-yle, 5-chloro-2-méthylphényle ou 2,5-bis(difluorométhyl)phényle,
L¹ représente -CH₂- ou -NH-,
L² représente une liaison directe,
Y représente oxygène,
X représente carbone,
p représente 0,
G représente G¹,
R^{G1} représente hydrogène,
Q représente Q²⁴-3,
R² représente hydrogène,
R⁵ représente hydrogène,
R¹ représente 3-formylphényle, 2-formylphényle ou 2-[(méthylsulfonyl)amino]phényle.

5. Composés de formule (VII) 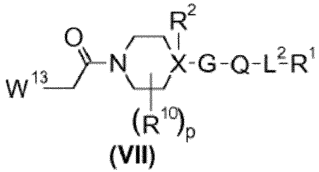 ainsi que leurs sels, complexes métalliques et N-oxydes, dans lesquels les symboles R², X, G, Q, L², R¹, R¹⁰ et p ont la signification indiquée dans l'une quelconque des revendications 1 à 4, et W¹³ représente un groupe partant.

6. Composés de formule (XVIId) 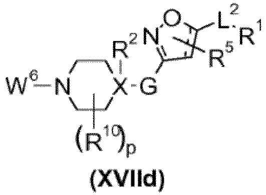 ainsi que leurs sels, complexes métalliques et N-oxydes, dans lesquels le symbole W⁶ représente acétyle, alcoxycarbonyle en C₁-C₄, benzyle ou benzyloxycarbonyle, et les symboles X, L², p, R¹, R², R⁵ et R¹⁰ ont la signification indiquée dans l'une quelconque des revendications 1 à 4.

7. Composés de formule (XIIIa) 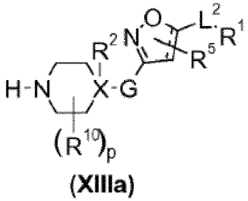 ainsi que leurs sels, complexes métalliques et N-oxydes, dans lesquels les symboles X, L², p, G, R¹, R², R⁵ et R¹⁰ ont la signification indiquée dans l'une quelconque des revendications 1 à 4.

8. Procédé de lutte contre des champignons nocifs phytopathogènes, **caractérisé en ce que** des composés de formule (I) selon l'une quelconque des revendications 1 à 4 sont déployés sur les champignons nocifs phytopathogènes et/ou leur habitat.

9. Agent de lutte contre des champignons nocifs phytopathogènes, **caractérisé par** une teneur en au moins un composé de formule (I) selon l'une quelconque des revendications 1 à 4, en plus d'extendeurs et/ou de substances tensioactives.

10. Utilisation de composés de formule (I) selon l'une quelconque des revendications 1 à 4 pour lutter contre des champignons nocifs phytopathogènes.

11. Procédé de fabrication d'agents de lutte contre des champignons nocifs phytopathogènes, **caractérisé en ce que** des composés de formule (I) selon l'une quelconque des revendications 1 à 4 sont mélangés avec des extendeurs et/ou des substances tensioactives.

12. Utilisation de composés de formule (I) selon l'une quelconque des revendications 1 à 4 pour le traitement de plantes transgéniques.
